# EUROPEAN PATENT SPECIFICATION

(11) **EP 2 389 582 B1**
(45) Date of publication and mention of the grant of the patent: **06.04.2016**
(21) Application number: 10701563.8
(22) Date of filing: 20.01.2010
(51) Int. Cl.: G01N 33/50

(54) **METHODS FOR PREDICTING AUTOIMMUNE DISEASE RISK**
VERFAHREN ZUR VORHERSAGE EINES AUTOIMMUNKRANKHEITSRISIKOS
PROCÉDÉS DE PRÉDICTION D'UN RISQUE DE MALADIE AUTO-IMMUNE

(30) Priority: 20.01.2009 US 145824 P; 01.09.2009 US 238752 P
(43) Date of publication of application: 30.11.2011
(73) Proprietor: Cambridge Enterprise Limited, Trinity Lane Cambridge Cambridgeshire CB2 1TN (GB)
(72) Inventor: SMITH, Ken, Cambridge CB2 OXY (GB); LYONS, Paul, Cambridge CB2 OXY (GB); MCKINNEY, Eoin, Cambridge CB2 OXY (GB)
(74) Representative: Keirstead, Tanis Evelyne
(86) International application number: PCT/GB2010/000085
(87) International publication number: WO 2010/084312

(56) References cited:
- DE-A1- 10 349 162
- MURALI-KRISHNA K ET AL: "Cutting edge: Naive T cells masquerading as memory cells" JOURNAL OF IMMUNOLOGY 20000815 US, vol. 165, no. 4, 15 August 2000 (2000-08-15), pages 1733-1737, XP002577336 ISSN: 0022-1767
- YAMASHITA ET AL: "Severe chronic graft-versus-host disease is characterized by a preponderance of CD4 effector memory cells relative to central memory cells" BLOOD, AMERICAN SOCIETY OF HEMATOLOGY, US, vol. 103, no. 10, 1 January 2004 (2004-01-01), pages 3986-1988, XP009131696 ISSN: 0006-4971
- FILIPPI C M ET AL: "Transforming growth factor-[beta] suppresses the activation of cd8 <+> t-cells when naive but promotes their survival and function once antigen experienced: a two-faced impact on autoimmunity" DIABETES, AMERICAN DIABETES ASSOCIATION, US, vol. 57, no. 10, 1 January 2008 (2008-01-01), pages 2684-2692, XP009131766 ISSN: 0012-1797
- MCKINNEY EOIN ET AL: "A CD8 T-cell memory transcription signature predicts prognosis in autoimmune disease" APMIS, WILEY INTERSCIENCE, DK, vol. 117, no. Suppl.127, 1 June 2009 (2009-06-01), page 127, XP009131751 ISSN: 0903-4641

## Description

### Field of the Invention

The present invention relates to means and methods for determining whether a subject is at high or low risk of autoimmune disease progression by determining the CD8 or CD4 cell subtype of the subject. Autoimmune diseases of particular interest include vasculitis, systemic lupus erythematosus (SLE), rheumatoid arthritis, multiple sclerosis, and inflammatory bowel disease.

### Background to the Invention

### Autoimmune Disease

Autoimmune disease is common, affecting about 10% of the population, and includes diseases such as vasculitis, systemic lupus erythematosus (SLE), rheumatoid arthritis, multiple sclerosis, and inflammatory bowel disease.

Management of autoimmune diseases usually involves immunosuppressive therapy. However, although immunosuppressive therapy can be effective at managing these diseases, infection as a result of therapeutic suppression of the immune system is a significant cause of the substantial morbidity and mortality associated with these diseases.

Many autoimmune diseases present with an initial acute phase followed by sporadic relapses rather than a continuous disease progression. Treatment usually involves an initial period of intensive treatment, referred to as induction therapy, during the first presentation of the disease followed by maintenance therapy, which is aimed at preventing relapses. However, disease progression varies widely between individuals, ranging from those that have frequent relapses after the initial acute phase to those which have no relapses at all.

Given the substantive morbidity and mortality associated with immunosuppressive therapy, it would be advantageous if patients unlikely to have relapses of the disease in question could be identified. Identification of these patients would allow clinicians to reduce the immunosuppressive maintenance therapy for these individuals, or even stop it completely, with a corresponding decrease in the morbidity and mortality associated with this form of treatment. In addition, individuals likely to have frequent relapses may benefit from a more intensive form of maintenance therapy, which would not be justified if given to all patients indiscriminately due to the severity of the likely side effects.

Although many autoimmune disorders present with heterogeneous clinical features in the clinic, it is not possible, on the basis of these clinical features, to determine what the likely pattern of disease progression for a given patient will be, and a number of tests have been developed with a view to addressing this problem. For example, in the case of the autoimmune disease anti-neutrophil cytoplasmic antibody (ANCA)-associated vasculitis, two autoimmune antibodies, one directed against proteinase-3 (PR-3), the other against myeloperoxidase (MPO), have been identified. However, although statistically the presence of anti-proteinase 3 antibodies is associated with disease progression, the association is not sufficiently strong to allow treatment decisions to be made based on the detection of these antibodies. In the case of SLE, the titre of anti-double stranded DNA antibodies has been used to predict disease progression. However, again the association of these antibodies with disease progression is not sufficiently strong to determine therapy.

Thus, there remains a need in the art for accurate methods of predicting disease progression in autoimmune disorders in order to avoid excess morbidity and mortality as a result of unnecessary immunosuppressive therapy.

### Microarray analysis

Gene chip arrays have been widely used to analyze gene transcription profiles of healthy and diseased tissues, with the aim of identifying genes which are differentially expressed (Kinter *et al*., 2008). The differentially expressed genes provide specific gene expression profiles (signatures), which allow diseased tissues to be classified. Such expression profiles may find application in, for example, diagnosis of the disease in question.

Similarly, gene chip arrays have also been used to analyze the gene transcription profiles of tissues from patients with different types of the same disease, such as more and less intensive forms of a particular type of cancer, in order to identify differentially expressed genes (Rhee *et al.,* 2008; Bao and Davidson, 2008). An attempt is then usually made to correlate the identified gene expression signature with disease progression. However, one of the problems frequently associated with this type of gene profiling is that it is often not clear whether the identified expression signatures are truly prognostic when applied to other patients.

Gene chips typically analyze the expression levels of many thousands of genes simultaneously and thus generate vast amounts of raw data. To analyze this data, a number of statistical analysis tools have been developed. These statistical techniques help determine, for example, which genes are consistently differentially expressed between different tissue types and also whether the level of differential expression detected is statistically significant, thus allowing the specific gene expression profile of a particular tissue type to be identified.

### Disclosure of the Invention

The autoimmune disease ANCA-associated vasculitis (AAV) presents with heterogeneous clinical features in the clinic, and severity as well as disease progression between different patients varies widely, with some patients having frequent relapses, or flares, of the disease, while others have infrequent, or no, relapses after initial presentation.

At present, ANCA-associated vasculitis patients are treated with induction therapy during the initial presentation of the disease followed by maintenance therapy. This is suboptimal given the difference with which the disease progresses in different patients, and leads to some patients receiving maintenance therapy despite the fact that they are unlikely to experience relapses, while maintenance therapy alone is insufficient to prevent relapses in other patients, who thus would potentially benefit from more intensive forms of treatment.

In an attempt to identify gene expression profiles associated with the different presentations of ANCA-associated vasculitis, the present inventors isolated peripheral blood mononuclear cells (PBMCs) from patients with this disease and used gene chip profiling to identify any differentially expressed genes. However, analysis of these PBMC gene expression profiles showed no meaningful substructure. In other words, it was not possible to separate patients into clinically useful subgroups based on their PBMC gene expression profiles.

Surprisingly, when the present inventors compared the gene expression profiles of CD8 cells obtained from different patients with ANCA-associated vasculitis, patients fell into one of two groups. In other words, patients could be divided into two distinct groups based on the gene expression profiles of their CD8 T cells. Two distinct groups of patients were also observed when the gene expression profiles of CD4 T cells of ANCA-associated vasculitis patients were compared. It has been shown that cancer patients can be divided into distinct groups based on their tumour gene expression profiles but such a division has not previously been observed for patients with autoimmune disease. Depending on the cells used for gene expression profiling, the two groups are referred to herein as CD8.1 (8.1) and CD8.2 (8.2), or CD4.1 (4.1) and CD4.2 (4.2). The patient groups identified on the basis of the CD8 and CD4 gene expression profiles differed slightly in size, but all patients that fell within group 8.1 also fell within group 4.1.

When the patients in the two groups were followed, patients in group 8.1 had significantly more relapses (flares) of the disease after initial presentation than patients in group 8.2. The same was also true of patients in group 4.1 when compared with patients in group 4.2. Thus, the CD8 and CD4 T cell gene expression signatures identified in ANCA-associated vasculitis patients are predictive of disease progression.

The present inventors then tested whether progression of other autoimmune disease could be predicted on the basis of the patient's CD8 gene expression profile. Surprisingly, when the CD8 T cell gene expression signatures of systemic lupus erythematosus (SLE) patients were compared, two distinct groups of patients, 8.1 and 8.2, could again be identified. The SLE patients in the two groups were followed and, as already observed in the case of ANCA-associated vasculitis patients, patients in group 8.1 also had significantly more flares of the disease after initial presentation than individuals in group 8.2. Thus, the CD8 T cell gene expression signatures observed in SLE patients are also predictive of disease progression.

Similarly, when the CD8 T cell gene expression signatures of inflammatory bowel disease (IBD) were compared, two distinct groups of patients, 8.1 and 8.2, could again be identified. The IBD patients in the two groups were followed and, in line with the observation made on the AAV and SLE patients, patients in group 8.1 were significantly more likely to experience disease progression than patients in patients in group 8.2. In the case of IBD, disease progression was defined as an event requiring increased therapy in the form of either increased immunosuppression or surgery. Such events include flares (or relapses) of the disease, as well as cases where the disease did not enter remission in response to initial therapy. Thus, the CD8 T cell gene expression signatures observed in IBD patients are also predictive of disease progression.

The above findings have important implications for the optimal management of autoimmune disease for patients in group 8.1 compared with those in group 8.2. For the patients in group 8.2, any benefits of immunosuppressive maintenance therapy may not outweigh the associated increase in morbidity and mortality. In contrast, patients in group 8.1 are likely to benefit substantially from immunosuppressive maintenance therapy, and the benefits are likely to outweigh the risks. In addition, patients in group 8.1 may benefit from more intensive treatment than is usual during the maintenance phase but which would not be justified if given to all patients indiscriminately due to the severity of the likely side effects of such treatment. The same is also true of patients in groups 4.1 compared with those in group 4.2, although to a lesser extent.

Given the surprising finding that individuals with autoimmune diseases can be divided into two distinct subgroups on the basis of their T cell gene expression profiles, the present inventors tested whether these distinct subgroups also exist in normal healthy individuals, i.e. individuals not suffering from any autoimmune disorders. Surprisingly, individuals could again be divided into two distinct groups, 8.1 and 8.2, on the basis of their CD8 gene expression profiles.

Given that the CD8 subtypes, 8.1 and 8.2, are predictive of disease progression in individuals with autoimmune disorders, it is possible that these subtypes also affect immune responses in normal healthy individuals, e.g. in response to infections or vaccinations. Similarly, these subtypes may also affect immune responses in transplant patients. The same also applies to individuals with CD4 subtypes, 4.1 and 4.2.

To confirm this, the inventors compared the CD8 T cell gene expression signatures of renal transplant patients. Again two distinct groups of patients, 8.1 and 8.2, could be identified. The transplant patients in the two groups were followed and patients in group 8.1 had a significantly higher likelihood of acute transplant rejection than individuals in group 8.2. Thus, the CD8 T cell gene expression signatures observed in transplant patients are also predictive of acute transplant rejection
The invention is as set out in the claims.

In one aspect the invention provides a method of assessing whether a subject is at high or low risk of autoimmune disease progression, which method comprises establishing, by determining the expression level of one or more genes in a CD8 cell from said subject, whether said subject has a high risk (CD8.1) or low risk (CD8.2) CD8 cell subtype,
wherein a CD8.1 subtype is characterised by upregulated expression of genes 1 to 11 listed in Table 1 (i.e. genes TXNDC9, POLR2H, MRPL14, GZMH, ATP8B1, LOC150759, IL7R, CD69, MCM6, and CD8B1) relative to the level of expression of the same genes in subtype CD8.2, as set out in claim 1.

In another embodiment, a CD8.1 subtype may be characterised by differential expression of genes 1 to 13 listed in Table 1 (i.e. genes TXNDC9, POLR2H, MRPL14, GZMH, ATP8B1, LOC150759, IL7R, CD69, MCM6, CD8B1, ITGA2, and PTPN22) relative to the level of expression of the same genes in subtype CD8.2. In a further embodiment, a CD8.1 subtype may be characterised by differential expression of the genes listed in Table 1 relative to the level of expression of the same genes in subtype CD8.2. This applies both to this and any other aspect of the present invention as described herein.

In another aspect, the invention provides a method of assessing whether a subject is at high or low risk of autoimmune disease progression, which method comprises establishing, by determining the expression level of one or more genes in a CD4 cell from said subject, whether said subject has a high risk (CD4.1) or low risk (CD4.2) CD4 cell subtype,
wherein a CD4.1 subtype is characterised by differential expression of genes 1 to 10 listed in Table 2 (i.e. genes SLAMF1, TNFSF5, DGKA, GPR171, IFI44, P2RY5, LEF1, CD69, IL7R, and RP42) relative to the level of expression of the same genes in subtype CD4.2 , as set out in claim 3.
In another embodiment, a CD4.1 subtype may be characterised by differential expression of genes 1 to 12 listed in Table 2 (i.e. genes SLAMF1, TNFSF5, DGKA, GPR171, IFI44, P2RY5, LEF1, CD69, IL7R, RP42, ITGA2 and PTPN22) relative to the level of expression of the same genes in subtype CD4.2. In a further embodiment, a CD4.1 subtype may be characterised by differential expression of the genes listed in Table 2 relative to the level of expression of the same genes in subtype CD4.2. This applies both to this and any other aspect of the present invention as described herein.

Also disclosed herein is a method of assessing whether a subject has a CD8.1 or CD8.2 CD8 cell subtype, which method comprises determining the expression level of one or more genes in a CD8 cell from said subject,
wherein a CD8.1 subtype is characterised by upregulated expression of genes 1 to 11 listed in Table 1 relative to the level of expression of the same genes in subtype CD8.2.

Also disclosed herein is a method of assessing whether a subject has a CD4.1 or CD4.2 CD4 cell subtype, which method comprises determining the expression level of one or more genes in a CD4 cell from said subject,
wherein a CD4.1 subtype is characterised by differential expression of genes 1 to 10 listed in Table 2 relative to the level of expression of the same genes in subtype CD4.2.

In another aspect, the invention provides a method of identifying genes differentially expressed in subjects with a high risk and subjects with a low risk of autoimmune disease progression, comprising:
(i) determining the level of CD8 cell gene expression in subjects with autoimmune disease using microarray analysis,
(ii) dividing the subjects into two groups based on their CD8 cell gene expression levels using principal components analysis and/or hierarchical clustering,
(iii) identifying the group with the higher level of autoimmune disease progression, and
(iv) identifying the genes differentially expressed in subjects with a high risk (CD8.1) CD8 cell subtype and subjects with a low risk (CD8.2) CD8 cell subtype, as set out in claim 12.

In another aspect, the invention provides a method of identifying genes differentially expressed in subjects with a high risk and subjects with a low risk of autoimmune disease progression, comprising:
(i) determining the level of CD4 cell gene expression in subjects with autoimmune disease using microarray analysis,
(ii) dividing the subjects into two groups based on their CD4 cell gene expression levels using principal components analysis and/or hierarchical clustering,
(iii) identifying the group with the higher level of autoimmune disease progression, and
(iv) identifying the genes differentially expressed in subjects with a high risk (CD4.1) CD4 cell subtype and subjects with a low risk (CD4.2) CD4 cell subtype, as set out in claim 14.

Also disclosed herein is a method of identifying genes differentially expressed in subjects with a CD8.1 or CD8.2 CD8 cell subtype, comprising:
(i) determining the level of CD8 cell gene expression in subjects using microarray analysis,
(ii) dividing the subjects into two groups based on their CD8 cell gene expression levels using principal components analysis and/or hierarchical clustering,
(iii) identifying the genes differentially expressed in subjects with a CD8.1 subtype and subjects with a CD8.2 subtype.

Also disclosed herein is a method of identifying genes differentially expressed in subjects with a CD4.1 or CD4.2 CD4 cell subtype, comprising:
(i) determining the level of CD4 cell gene expression in subjects using microarray analysis,
(ii) dividing the subjects into two groups based on their CD4 cell gene expression levels using principal components analysis and/or hierarchical clustering,
(iii) identifying the genes differentially expressed in subjects with a CD4.1 subtype and subjects with a CD4.2 subtype.

Also disclosed herein is a method of assessing whether a subject is at high or low risk of autoimmune disease progression, which method comprises determining the number of memory CD8 cells in a sample obtained from said subject and comparing said number to a reference number,
wherein a subject at high risk of autoimmune disease progression is characterised by an increased number of memory CD8 cells relative to the number of memory CD8 cells in a subject at low risk of autoimmune disease progression.

Also disclosed herein is a method of assessing whether a subject has a CD8.1 or CD8.2 CD8 cell subtype, which method comprises determining the number of memory CD8 cells in a sample obtained from said subject and comparing said number to a reference number,
wherein a subject with a CD8.1 subtype is characterised by an increased number of memory CD8 cells relative to the number of memory CD8 cells in a subject with a CD8.2 subtype.

Also disclosed herein is a method of assessing whether a subject is at high or low risk of autoimmune disease progression, which method comprises determining the number of memory CD4 cells in a sample obtained from said subject and comparing said number to a reference number,
wherein a subject at high risk of autoimmune disease progression is characterised by an increased number of memory CD4 cells relative to the number of memory CD4 cells in a subject at low risk of autoimmune disease progression.

Also disclosed herein is a method of assessing whether a subject has a CD4.1 or CD4.2 CD4 cell subtype, which method comprises determining the number of memory CD4 cells in a sample obtained from said subject and comparing said number to a reference number,
wherein a subject with a CD4.1 subtype is characterised by an increased number of memory CD4 cells relative to the number of memory CD4 cells in a subject with a CD4.2 subtype.

Also disclosed herein is a method of assessing whether a subject is at high or low risk of autoimmune disease progression, which method comprises establishing, by determining the level of expression of one or more proteins on the surface of a CD8 cell from said subject, whether said subject has a high risk (CD8.1) or low risk (CD8.2) CD8 cell subtype,
wherein a CD8.1 subtype is characterised by differential expression of the protein relative to the level of expression of the protein in subtype CD8.2.

Also disclosed herein is a method of assessing whether a subject has a CD8.1 or CD8.2 CD8 cell subtype, which method comprises determining the level of expression of one or more proteins on the surface of a CD8 cell from said subject,
wherein a CD8.1 subtype is characterised by differential expression of the protein relative to the level of expression of the protein in subtype CD8.2.

Also disclosed herein is a method of assessing whether a subject is at high or low risk of autoimmune disease progression, which method comprises establishing, by determining the level of expression of one or more proteins on the surface of a CD4 cell from said subject, whether said subject has a high risk (CD4.1) or low risk (CD4.2) CD4 cell subtype,
wherein a CD4.1 subtype is characterised by differential expression of the protein relative to the level of expression of the protein in subtype CD4.2.

Also disclosed herein is a method of assessing whether a subject has a CD4.1 or CD4.2 CD4 cell subtype, which method comprises determining the level of expression of one or more proteins on the surface of a CD4 cell from said subject,
wherein a CD4.1 subtype is characterised by differential expression of the protein relative to the level of expression of the protein in subtype CD4.2.

Also disclosed herein is a method of assessing whether a subject is at high or low risk of autoimmune disease progression, which method comprises establishing, by determining level of expression of one or more proteins in a sample obtained from said subject, whether said subject has a high risk (CD8.1) or low risk (CD8.2) CD8 cell subtype,
wherein a CD8.1 subtype is characterised by differential expression of the protein relative to the level of expression of the protein in subtype CD8.2.

Also disclosed herein is a method of assessing whether a subject has a CD8.1 or CD8.2 CD8 cell subtype, which method comprises determining the level of expression of one or more proteins in a sample obtained from said subject,
wherein a CD8.1 subtype is characterised by differential expression of the protein relative to the level of expression of the protein in subtype CD8.2.

Also disclosed herein is a method of assessing whether a subject is at high or low risk of autoimmune disease progression, which method comprises establishing, by determining the level of expression of one or more proteins in a sample obtained from said subject, whether said subject has a high risk (CD4.1) or low risk (CD4.2) CD4 cell subtype,
wherein a CD4.1 subtype is characterised by differential expression of the protein relative to the level of expression of the protein in subtype CD4.2.

Also disclosed herein is a method of assessing whether a subject has a CD4.1 or CD4.2 CD4 cell subtype, which method comprises determining the level of expression of one or more proteins In a sample obtained from said subject,
wherein a CD4.1 subtype is characterised by differential expression of the protein relative to the level of expression of the protein in subtype CD4.2.

Also disclosed herein is a kit for assessing whether a subject is at high or low risk of autoimmune disease progression, wherein said kit comprises reagents for establishing the expression level of one or more genes in a CD8 cell from said subject, for determining whether said subject has a high risk (CD8.1) or low risk (CD8.2) CD8 cell subtype,
wherein a CD8.1 subtype is characterised by upregulated expression of genes 1 to 11 listed in Table 1 relative to the level of expression of the same genes in subtype CD8.2.

In another aspect, the invention provides the use of a kit according for assessing whether a subject is at high or low risk of autoimmune disease progression by determining whether said subject has a high risk (CD8.1) or low risk (CD8.2) CD8 cell subtype, as set out in claim 10.

Also disclosed herein is a kit for assessing whether a subject is at high or low risk of autoimmune disease progression, wherein said kit comprises reagents for establishing the expression level of one or more genes in a CD4 cell from said subject, for determining whether said subject has a high risk (CD4.1) or low risk (CD4.2) CD4 cell subtype,
wherein a CD4.1 subtype is characterised by differential expression of genes 1 to 10 listed in Table 2 relative to the level of expression of the same genes in subtype CD4.2.

In another aspect, the invention provides use of a kit for assessing whether a subject is at high or low risk of autoimmune disease progression by determining whether said subject has a high risk (CD4.1) or low risk (CD4.2) CD4 cell subtype, as set out in claim 11.

Also disclosed herein is a kit for assessing whether a subject has a CD8.1 or CD8.2 CD8 cell subtype, wherein said kit comprises reagents for establishing the expression level of one or more genes in a CD8 cell from said subject,
wherein a CD8.1 subtype is characterised by upregulated expression of genes 1 to 11 listed in Table 1 relative to the level of expression of the same genes in subtype CD8.2.

Also disclosed herein is a kit for assessing whether a subject has a CD4.1 or CD4.2 CD4 cell subtype, wherein said kit comprises reagents for establishing the expression level of one or more genes in a CD4 cell from said subject,
wherein a CD4.1 subtype is characterised by differential expression of genes 1 to 10 listed in Table 2 relative to the level of expression of the same genes in subtype CD4.2.

These and further aspects are set out in more detail below.

### Description of the Drawings and Tables

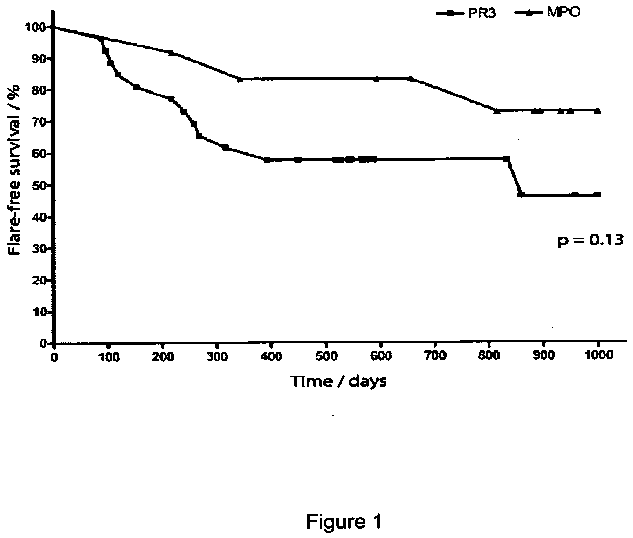

**Fig. 1****: ANCA status does not predict time to first flare.** Flare-free survival shown as a Kaplan-Meier plot with significance measured using the log-rank test.

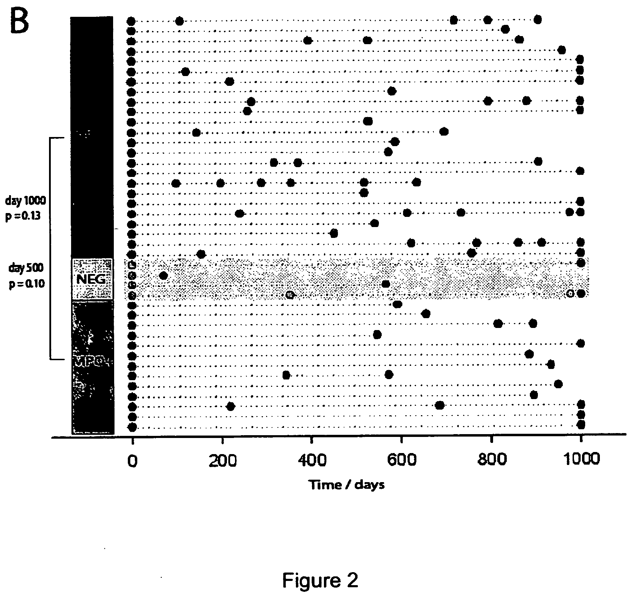

**Fig. 2****: ANCA status does not predict clinical outcome following induction therapy.** PR3-ANCA and MPO-ANCA positive patients showed similar numbers of flares when followed to 1000 days post-treatment.

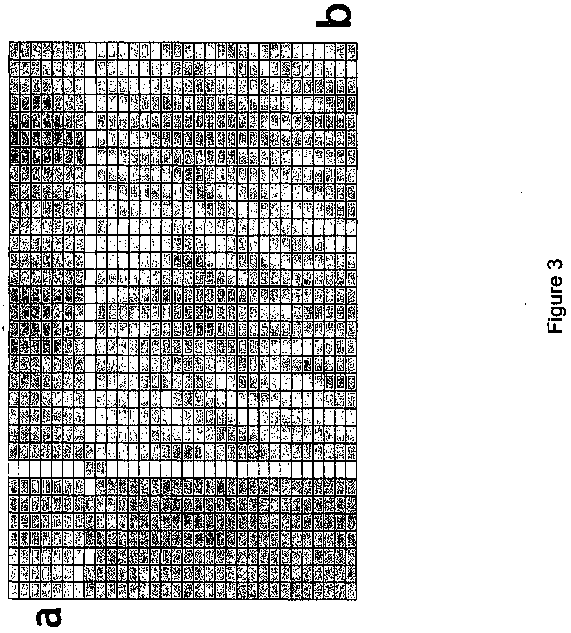

**Fig. 3****: Microarray gene expression data from CD8 or CD4 T cells divides AAV patients into two distinct groups.** Schematic diagram of the **CD8** and CD4 T cell microarray gene expression data obtained. Each row represents one gene, while each column represents one individual. Light coloured areas represent genes which are down-regulated in one T cell subtype (e.g. CD8.2) compared the level of expression seen in the other T cell subtype (e.g. CD8.1). Dark coloured areas represent genes which are up-regulated in one T cell subtype (e.g. CD8.1) compared the level of expression seen in the other T cell subtype (e.g. CD8.2).

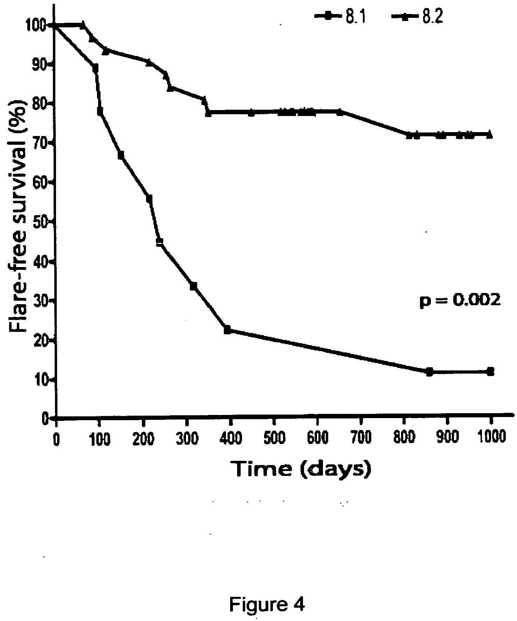

**Fig. 4****: CD8 T cell subtypes identified on the basis of gene expression predict clinical outcome following treatment.** Following induction therapy, patients in subgroup 8.1 showed a shorter time to first flare than patients in subgroup 8.2. Kaplan-Meier plot showing flare-free survival, significance measured using the log-rank test.

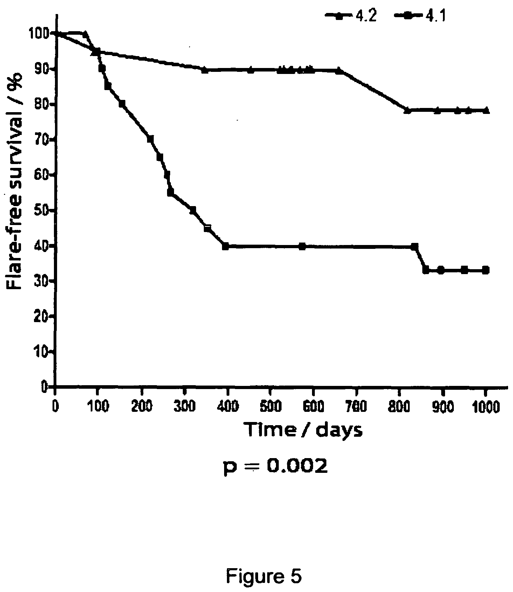

**Fig. 5****: CD4 T cell subtypes Identified on the basis of gene expression predict clinical outcome following treatment.** Following induction therapy, patients in subgroup 4.1 showed a shorter time to first flare than patients in subgroup 4.2. Kaplan-Meier plot showing flare-free survival, significance measured using the log-rank test.

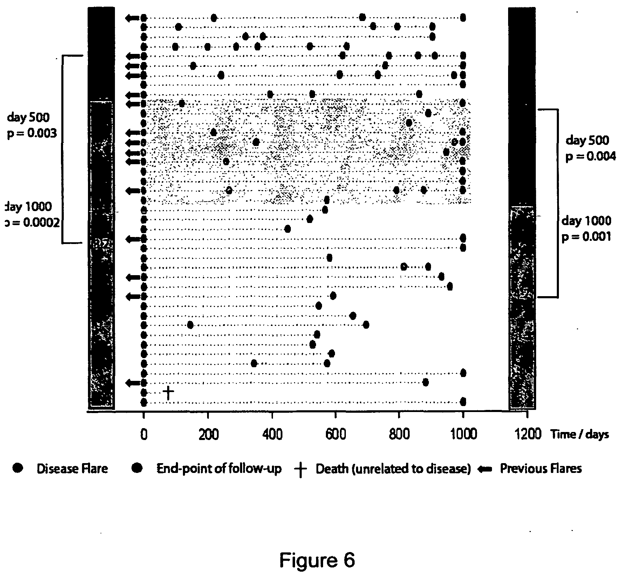

**Fig. 6****: T cell subtypes identified on the basis of gene expression predict clinical outcome following treatment.** In addition to a shorter time to first flare, patients in subgroups 8.1 and 4.1 exhibited significantly more flares than patients in subgroups 8.2 or 4.2, respectively, when followed out to 1,000 days post induction therapy. Flare rate normalised to duration of follow-up with Mann-Whitney U test of significance performed at day 500 and 1000.

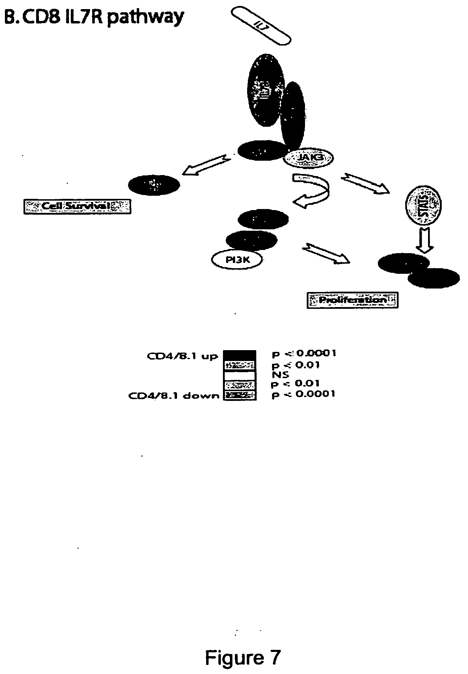

**Fig. 7****: Poor prognosis correlates with differential expression of mRNAs encoding proteins associated with the IL7 receptor pathway.** Analysis of the mRNAs differentially expressed between the 8.1 and 8.2 subtypes revealed upregulation of those involved in IL7 but not IL2 or IL4 signalling.

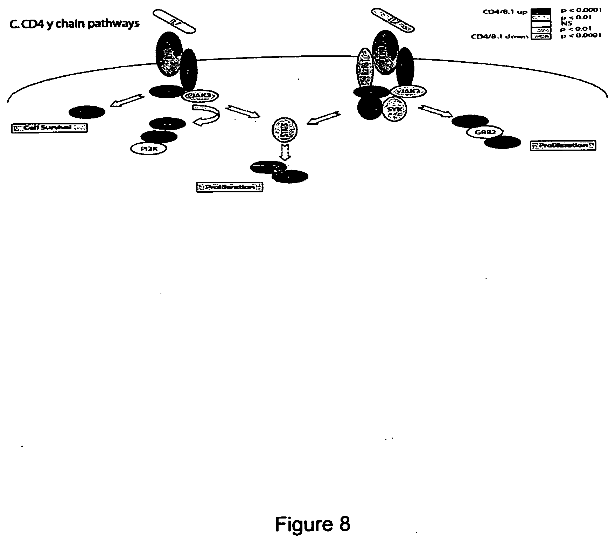

**Fig. 8****: Poor prognosis correlates with differential expression of mRNAs encoding proteins associated with T cell memory.** Analysis of the gene signature defining the 4.1 subtype showed upregulation of mRNAs encoding proteins associated with both IL7 and IL2 but not IL4 signalling.

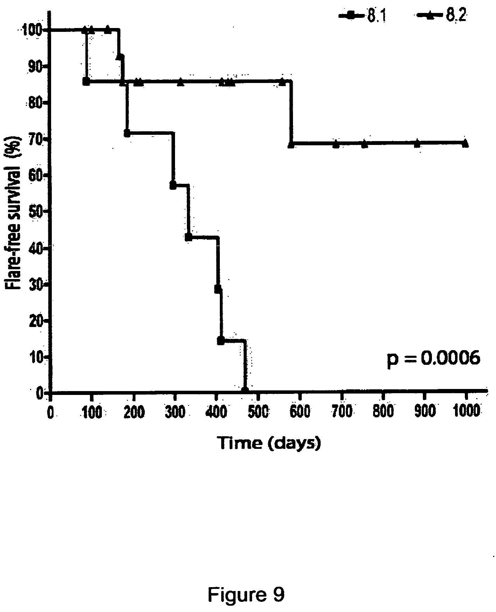

**Fig. 9****: Stable differences in memory T cells that determine prognosis in AAV define similar subtypes with adverse prognosis in SLE.** Kaplan-Meier plot showing flare-free survival, significance measured using the log-rank test.

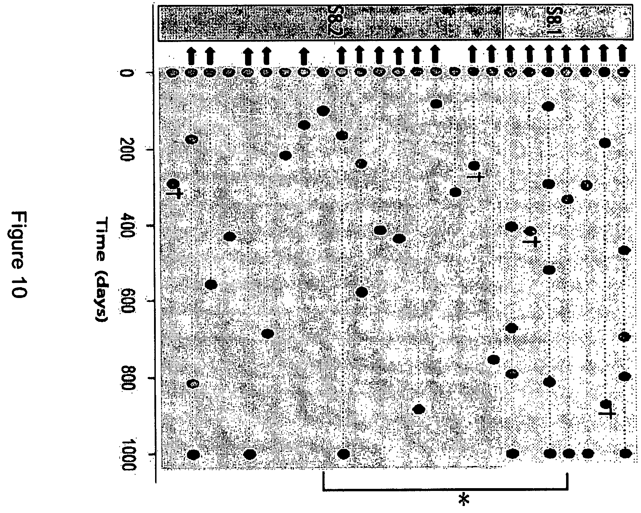

**Fig. 10****: Stable differences in memory T cells that determine prognosis in AAV define similar subtypes with adverse prognosis in SLE.** Patients in subgroup s8.1 (SLE subtype 8.1) also experienced significantly more flares than patients in subgroup s8.2 (SLE subtype 8.2) when followed out to 1,000 days post induction therapy. Flare-rate normalised to duration of follow-up with Mann-U Whitney test of significance performed at day 500 (flare-rate 0.86/year vs 0.08/year, p = 0.0006) and at day 1,000 (flare-rate 0.81/year vs 0.09/year, p = 0.001).

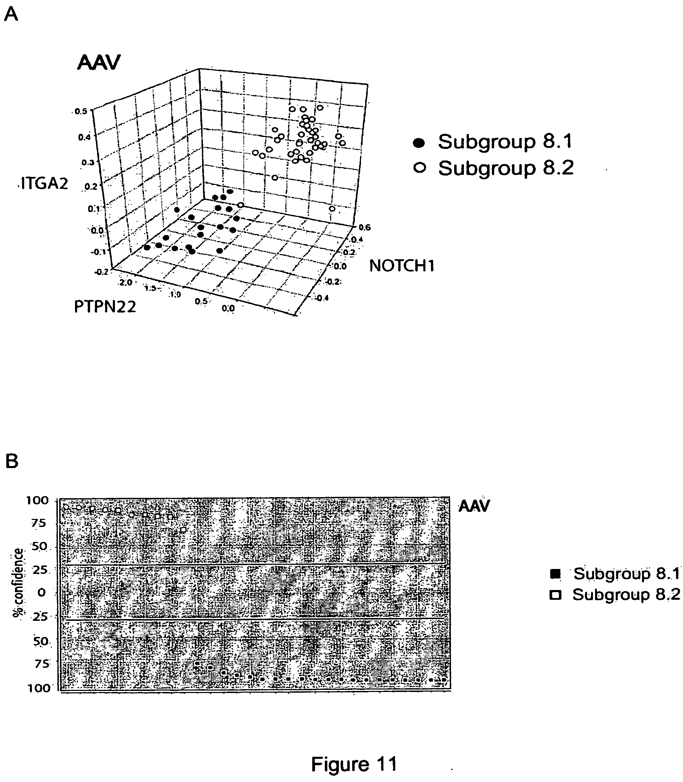

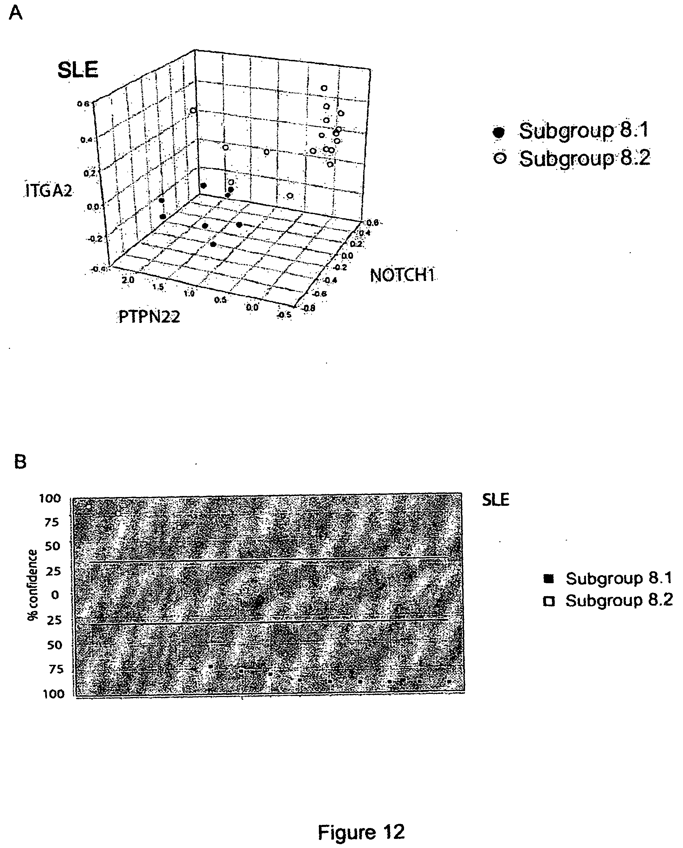

**Figs. 11** **and** **12****: A predictive model based on the expression of 3 genes, ITGA2, PTPN22 and NOTCH1, robustly identifies prognostic groups 8.1 and 8.2 in both AAV and SLE.** Figures 11A and 12A show 3D scatterplots illustrating the distribution of AAV (n=59) and SLE (n=26) patients by expression of three CD8 T cell memory-related genes (ITGA2, PTPN22 and NOTCH1), respectively. Axes x, y and z in Figures 11A and 12A show mRNA expression of ITGA2, PTPN22 and NOTCH1 as log₂ ratios. Figures 11B and 12B show that subgroups 8.1 and 8.2 could be confidently and accurately predicted based on the expression of these three genes both in AAV and SLE (positive predictive value [PPV] 100%, negative predictive value [NPV] 100%). The y axis in Figures 11B and 12B shows the confidence of prediction (%). The genes ITGA2, PTPN22 and NOTCH1 therefore comprise an optimized predictive model.

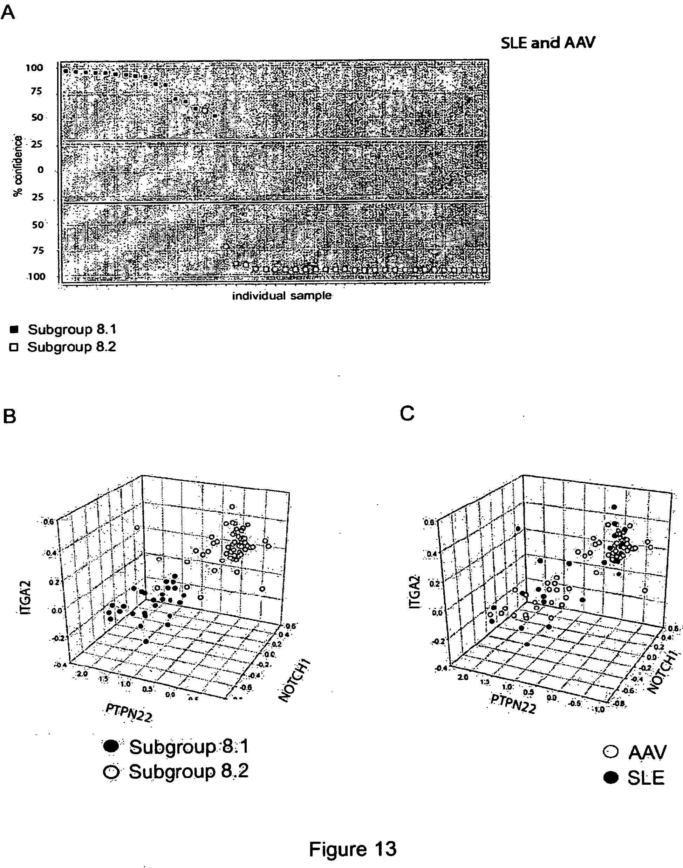

**Fig. 13****: The optimized predictive model based on the expression of the genes ITGA2, PTPN22 and NOTCH1 developed on the AAV dataset could also robustly determine prognostic groups 8.1 and 8.2 when applied to the CD8 expression dataset as a whole (incorporating both SLE and AAV).** Figure 13A shows that subgroups 8.1 and 8.2 could be accurately predicted based on the expression of genes ITGA2, PTPN22 and NOTCH1 (PPV= 94%, NPV=100%). The single patient inaccurately classified was the only "borderline" case, originally classed as 8.1 by one clustering technique and as 8.2 by another. Reclassification of this individual as 8.1 would improve rather than weaken the association with poor outcome. The y axis in Figure 13A shows the confidence of prediction (%). Figure 13B and C show 3D scatterplots illustrating the distribution of patients by expression of the genes ITGA2, PTPN22 and NOTCH1. Patients were differentiated by prognostic group (Figure 13B) but not by disease type (Figure 13C). Axes x, y and z in Figures 13B and C show mRNA expression of ITGA2, PTPN22 and NOTCH1 as log₂ ratios.

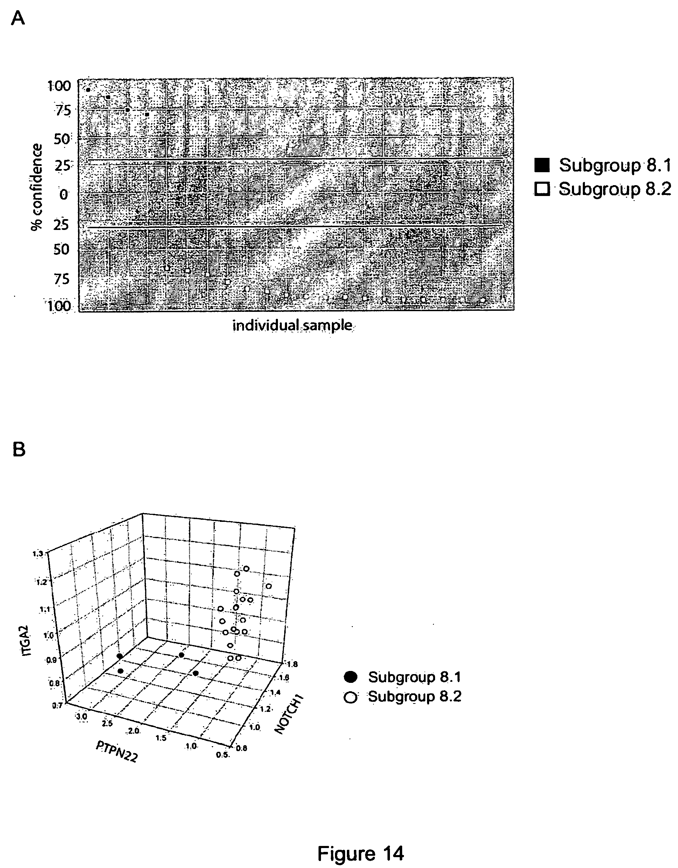

**Fig. 14****: The optimized predictive model based on the expression of the genes ITGA2, PTPN22 and NOTCH1 derived from the AAV dataset could also robustly predict subgroups 8.1 and 8.2 identity in a control dataset from healthy individuals.** Figure 14B shows a 3D scatterplot illustrating the distribution of healthy individuals by expression of the genes ITGA2, PTPN22 and NOTCH1. Axes x, y and z in Fig 14B show mRNA expression of ITGA2, PTPN22 and NOTCH1 as log₂ ratio. Figure 14A shows that subgroups 8.1 and 8.2 could be accurately predicted based on the expression of genes ITGA2, PTPN22 and NOTCH1 (PPV100%, NPV 100%). The y axis in Figure 14A and shows the confidence of prediction (%).

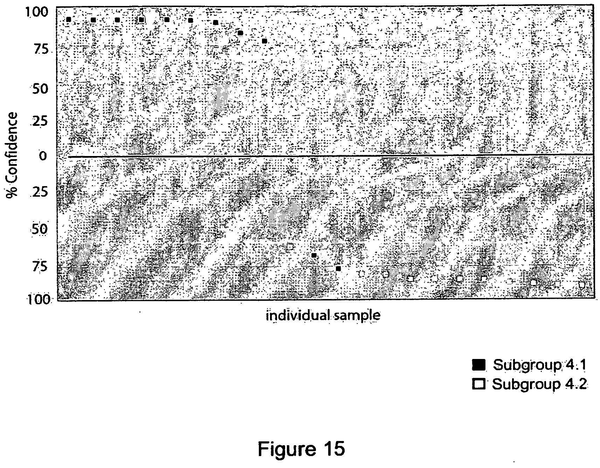

**Fig. 15****:** The predictive model based on the expression of the genes ITGA2, PTPN22 and NOTCH1 could also predict subgroups 4.1 and 4.2 in the AAV CD4 dataset with good accuracy (PPV 100%, NPV 85%). The y axis in Figure 15 shows the confidence of prediction (%).

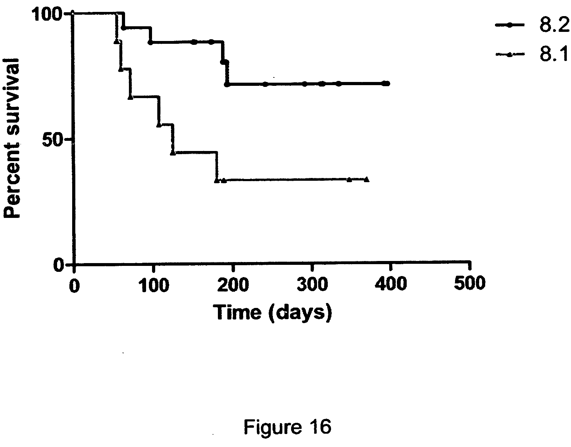

**Fig. 16****: Subgroup 8.1 correlates with poor clinical outcome in inflammatory bowel disease (IBD) patients.** Fig. 16 shows a Kaplan-Meier plot showing event-free survival over time. An "event" in this case was classified as a requirement for increased therapy in the form of either increased immunosuppression or need for surgery. Event-free survival differed significantly between subgroups 8.1 and 8.2 (p=0.019). Statistical significance was assessed using the log-rank test.

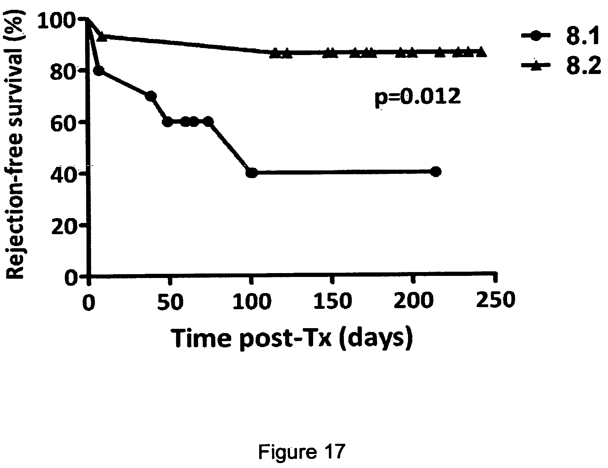

**Fig. 17****: Subgroup 8.1 correlates with increased likelihood of acute transplant rejection in transplant patients.** Following transplantation, renal transplant patients were followed for up to 250 days. Fig. 17 shows a Kaplan-Meier plot showing acute rejection-free survival over time. Rejection-free survival differed significantly between subgroups 8.1 and 8.2 (p=0.012). Statistical significance was assessed using the log-rank test.

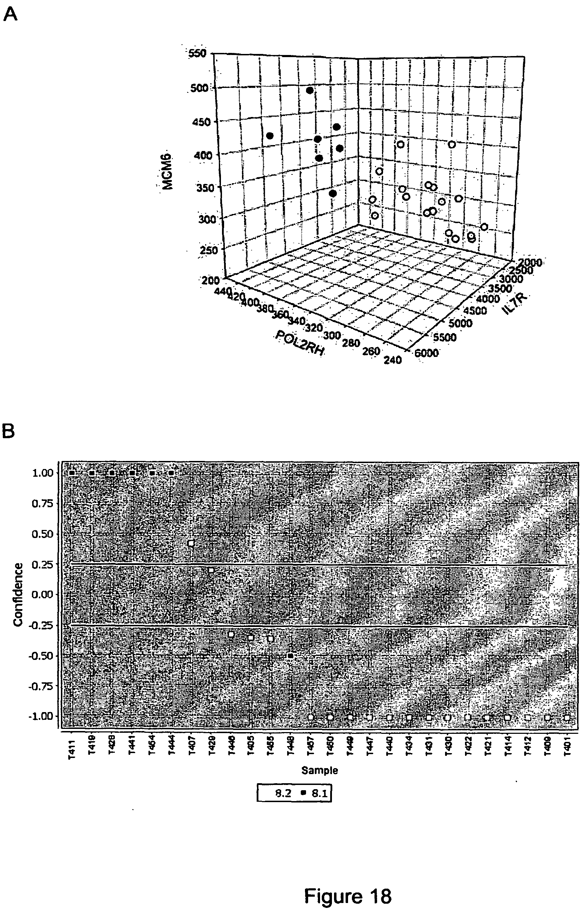

**Fig. 18****: Inflammatory bowel disease (IBD) patients can be classified as subgroup 8.1 or 8.2 based on the expression of 3 genes, MCM6, POLR2H and IL7R.** Figure 18A shows a 3C scatterplot illustrating the distribution of IBD (Crohn's disease) patients by expression of three genes (MCM6, POLR2H and IL7R). Axes x, y and z in Figure 18A show mRNA expression of MCM6, POLR2H and IL7R as log₂ ratios. Figure 18B shows that IBD patients could be classified as subgroup 8.1 or 8.2 based on the expression of these three genes (positive predictive value [PPV] 86%, negative predictive value [NPV] 94%). The confidence threshold applied in this case was 25% (indicated by solid black lines) and one patient sample remained unclassified. The y axis in Figure 18B shows the confidence of prediction (%). Classification was performed using a weighted voting algorithm with leave-one-out cross-validation.

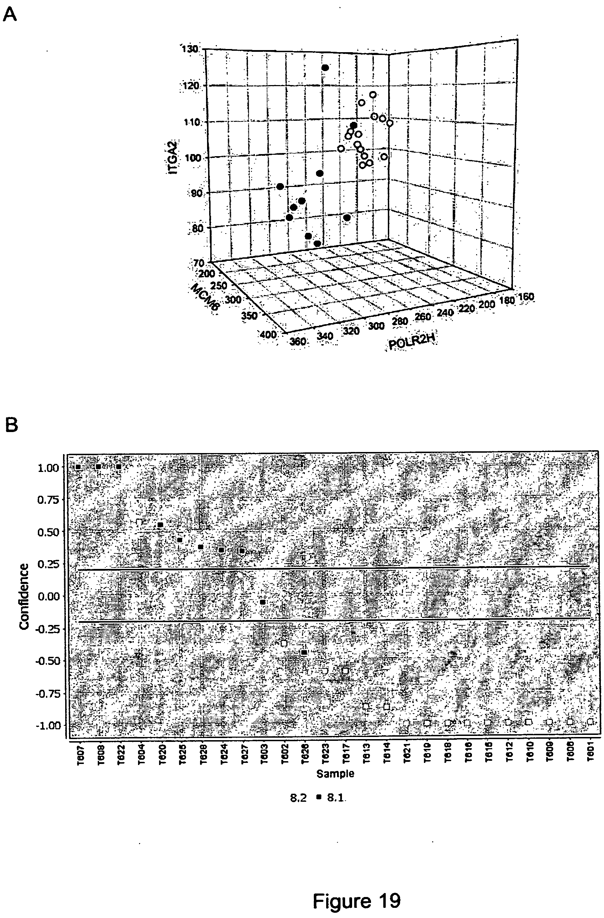

**Fig. 19****: Transplant patients can be classified as subgroup 8.1 or 8.2 based on the expression of 3 genes, ITGA2, MCM6 and POLR2H.** Figure 19A shows a 3D scatterplot illustrating the distribution of renal transplant recipients by expression of three genes (ITGA2, MCM6 and POLR2H). Axes x, y and z in Figure 19A show mRNA expression of ITGA2 MCM6 and POLR2H as log₂ ratios. Figure 19B shows that renal transplant recipients could be classified as subgroup 8.1 or 8.2 based on the expression of these three genes (positive predictive value [PPV] 89%, negative predictive value [NPV] 94%). The confidence threshold applied in this case was 20% (indicated by solid black lines) and one patient sample remained unclassified. The y axis in Figure 19B shows the confidence of prediction (%). Classification was performed using a weighted voting algorithm with leave-one-out cross-validation.

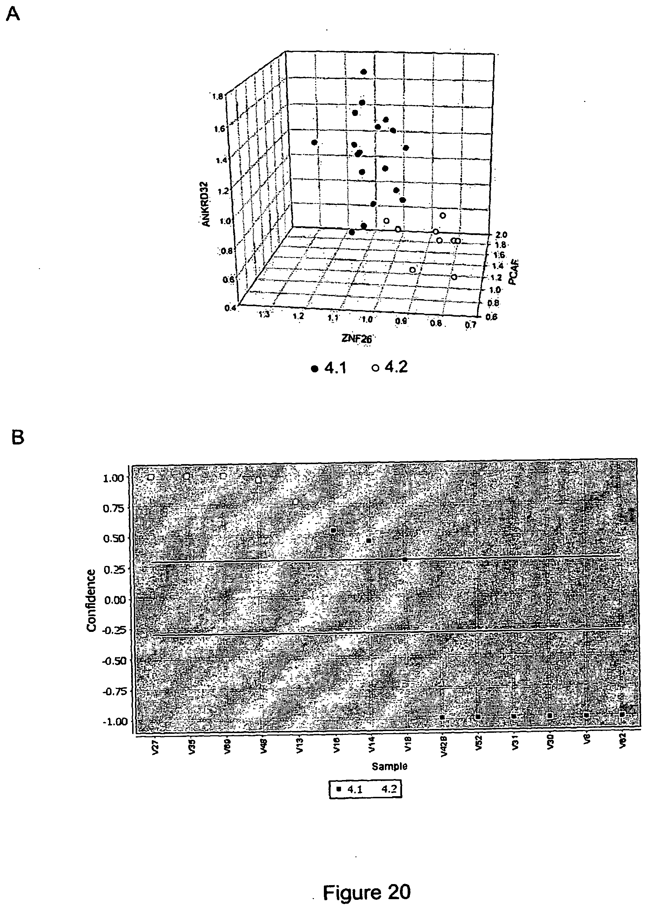

**Fig. 20****: AAV patients can be classified as subgroup 4.1 or 4.2 based on the expression of 3 genes, PCAF, ANKRD32 and ZNF26, in unseparated PBMCs.** Figure 20A shows a 3D scatterplot illustrating the distribution of 27 AAV patients by expression of three genes (PCAF, ANKRD32 and ZNF26) in unseparated PBMCs. Axes x, y and z in Figure 20A show mRNA expression of PCAF, ANKRD32 and ZNF26 as log₂ ratios. Figure 20B shows that AAV patients could be classified as subgroup 4.1 or 4.2 based on the expression of these three genes (positive predictive value [PPV] 100%, negative predictive value [NPV] 71%) in unseparated PBMC samples. The confidence threshold applied in this case was 25% (indicated by solid black lines) and one patient sample remained unclassified. The y axis in Figure 20B shows the confidence of prediction (%). Classification was performed using a weighted voting algorithm with leave-one-out cross-validation.

**Table 1: preferred genes for differentiating between subtypes 8.1 and 8.2.** For each gene Table 1 lists the gene symbol allocated to the gene in the HGNC (HUGO Gene Nomenclature Committee) database and the GenBank Accession Number, along with a brief description. Most of the genes listed in Table 1 are upregulated in subtype 8.1 compared to subtype 8.2, as indicated. The p-value is the feature specific p-value based on permutation testing. The FDR level is an estimate of the false-discovery rate using the Benjamini and Hochberg procedure (Hochberg and Benjamini, 1990) and shows the expected proportion of erroneous rejections among all rejections. The fold-change indicates the fold-change in gene expression observed between subtypes 8.1 and 8.2. The p-values, FDR levels and fold-change values listed in Table 1 were determined based on the AAV cohort. Also provided in Table 1 are the sequences of exemplary primer pairs which can be used to determine the level of expression of the genes listed in this table.
**Table 2: preferred genes for differentiating between subtypes 4.1 and 4.2.** Table 2 lists the gene symbol allocated to the gene in the HGNC (HUGO Gene Nomenclature Committee) database and the GenBank Accession Number, along with a brief description. Most of the genes listed in Table 2 are upregulated in subtype 4.1 compared to subtype 4.2, as indicated. The p-value is the feature specific p-value based on permutation testing. The FDR leve! is an estimate of the false-discovery rate using the Benjamini and Hochberg procedure (Hochberg and Benjamini, 1990) and shows the expected proportion of erroneous rejections among all rejections. The fold-change indicates the fold-change in gene expression observed between subtypes 4.1 and 4.2. The p-values, FDR levels and fold-change values listed in Table 2 were determined based on the AAV cohort. Also provided in Table 2 are the sequences of exemplary primer pairs which can be used to determine the level of expression of the genes listed in this table.

### Detailed Description of the Invention

By "autoimmune disease" or "autoimmune disorder" it is meant any condition which involves an overactive immune response of the body against substances and tissues normally present in the body. Specific autoimmune diseases include, vasculitis (including e.g. Henoch-Schonlein purpura; Takayasu's arteritis; cryoglobulinemic vasculitis; polyarteritis nodosa (PAN); giant cell arteritis; and ANCA-associated vasculitis, which includes Wegener's granulomatosis, microscopic polyangitis (MPA) and Churg-Strauss syndrome), systemic lupus erythematosus (SLE), rheumatoid arthritis (RA), diabetes mellitus type 1 (type 1 diabetes; juvenile diabetes), multiple sclerosis (MS), autoimmune hematological disorders (including e.g. hemolytic anemia, aplastic anemia, pure red cell anemia and idiopathic thrombocytopenia), polychondritis, sclerodoma, dermatomyositis, chronic active hepatitis, myasthenia gravis, psoriasis, Steven-Johnson syndrome, idiopathic sprue, (autoimmune) inflammatory bowel disease (IBD) (including e.g. ulcerative colitis, Crohn's disease, collagenous colitis, lymphocytic colitis, ischaemic colitis, diversion colitis, Behçet's syndrome, infective colitis, and indeterminate colitis), Sjogren's syndrome, endocrine ophthalmopathy, Graves' disease, sarcoidosis, primary biliary cirrhosis, uveitis (anterior and posterior), keratoconjunctivitis sicca and vernal keratoconjunctivitis, interstitial lung fibrosis, chronic obstructive pulmonary disease (COPD), psoriatic arthritis, glomerulonephritis (including membranous nephropathy, IgA nephropathy, and cryoglobulinaemic nephropathy), and juvenile dermatomyositis.

Autoimmune diseases of particular interest include vasculitis, systemic lupus erythematosus (SLE), rheumatoid arthritis, multiple sclerosis, and inflammatory bowel disease (IBD).

In a preferred embodiment, the autoimmune disease is selected from the group of: vasculitis, rheumatoid arthritis, multiple sclerosis, and inflammatory bowel disease (IBD).

In a preferred embodiment, the autoimmune disease is selected from the group of: vasculitis, systemic lupus erythematosus (SLE), and inflammatory bowel disease (IBD).

In a preferred embodiment, the autoimmune disease is selected from the group of: vasculitis, and systemic lupus erythematosus (SLE).

In a preferred embodiment, the autoimmune disease is systemic lupus erythematosus (SLE).

In a preferred embodiment, the autoimmune disease is inflammatory bowel disease (IBD).

In a preferred embodiment, the autoimmune disease is vasculitis.

In a preferred embodiment, the autoimmune disease is anti-neutrophil cytoplasmic antibody (ANCA)-associated vasculitis (AAV).

By "treating", it is meant both therapeutic treatment of ongoing disease intended to manage the disease (e.g. to prevent relapses or flares of the disease), to cure the disease, or to provide relief from the symptoms of the disease, as well as prophylactic treatment to prevent disease in a subject at risk of developing an autoimmune disease. In the case of treatment of an autoimmune disease, the subject may be a subject having had an initial presentation of an autoimmune disease, a subject who has had an initial presentation of the disease followed by one or more relapses (flares) of the disease, or a subject who has one or more genetic risk factors for the disease. The invention may find particular application in determining the optimal induction or maintenance treatment (therapy) for a subject with autoimmune disease.

By "a subject" it is intended a mammalian subject including, but not limited to, a human subject.

By "autoimmune disease progression" it is meant the progression of the autoimmune disease after initial presentation of the disease in a subject. For example, autoimmune disease progression may refer to relapses, or flares, of the disease experienced by the subject after initial presentation. A relapse or flare may be an event that requires increased therapy, e.g. increased immunosuppressive therapy or surgery. A high risk of autoimmune disease progression may accordingly refer to a high risk that the subject will experience (frequent) flares of the disease after initial presentation, while a low risk of autoimmune disease progression may refer to a low risk that the subject will experience (frequent) flares of the disease after initial presentation. In other words, subjects with a low risk of disease progression may be likely to experience few, or even no, flares after initial presentation of the disease. Autoimmune disease progression may also refer to an ongoing worsening of clinical features, which can occur in the absence of discrete flares. This type of disease progression is known to occur, for example, in multiple sclerosis. A high risk of autoimmune disease progression may accordingly refer to a high risk that the subject will experience an ongoing worsening of clinical features after initial presentation, while a low risk of autoimmune disease progression may refer to a low risk that the subject will experience an ongoing worsening of clinical features after initial presentation. Thus, subjects with a low risk of disease progression may be unlikely to experience an ongoing worsening of clinical features after initial presentation. Autoimmune disease progression, in particular in the case of IBD, may also refer to an event requiring increased therapy in the form of either increased immunosuppression or surgery. Such events included relapses, or flares, of the disease after a period of remission, as well as instances where the disease does not enter remission in response to initial therapy and increased immunosuppression or surgery is required as a result. A high risk of autoimmune disease progression may accordingly refer to a high risk that the subject will experience (frequent) events requiring increased therapy after initial presentation of the disease, while a low risk of autoimmune disease progression may refer to a low risk that the subject will experience (frequent) events requiring increased therapy after initial presentation of the disease. In other words, subjects with a low risk of disease progression may be likely to experience few, or even no, events requiring increased therapy after initial presentation of the disease.

By "expression level" in relation to a gene it is meant the level of gene expression observed relative to a reference level. For example, the level of expression of a given gene in subtype CD8.1 may be expressed as being upregulated or downregulated relative to the level of expression of the same gene observed in subtype CD8.2, and vice versa. Similarly, the level of expression of a given gene in subtype CD4.1 may be expressed as being upregulated or downregulated relative to the level of expression of the same gene observed in subtype CD4.2, and vice versa. Methods for determining the expression level of a gene of interest are described elsewhere herein.

By "gene expression profile" or "gene expression signature" it is meant the specific gene expression pattern observed in a sample compared to a reference sample. The gene expression profile of a sample may, for example, be determined using microarray analysis.

By "CD8.1 subtype" (CD8.1 subgroup) or "8.1 subtype" (8.1 subgroup) it is meant CD8 T cells characterised by a specific gene expression profile (signature) which differentiates these CD8 T cells from the CD8 T cells of other subjects. CD8 T cells of subtype CD8.1 may be characterised by upregulated expression of genes 1 to 11 listed in Table 1. In another embodiment, CD8 T cells of subtype CD8.1 may be characterised by differential (upregulated or downregulated) expression of genes 1 to 13 listed in Table 1, as indicated. In a further embodiment, CD8 T cells of subtype CD8.1 may be characterised by differential (upregulated or downregulated) expression of the genes listed in Table 1, as indicated. The level of expression of these genes in subtype CD8.1 is may be differentially expressed relative to the level of expression of the same genes in CD8 T cells of subtype CD8.2. The level of expression of the genes listed in Table 1 may be determinable using the primer pairs listed in Table 1. The presence of CD8 T cells of subtype CD8.1 in a subject with autoimmune disease, such as ANCA-associated vasculitis, SLE or IBD, indicates that the subject is at high risk of autoimmune disease progression.

By "CD8.2 subtype" (CD8.2 subgroup) or "8.2 subtype" (8.2 subgroup) it is meant CD8 T cells characterised by a specific gene expression profile (signature) which differentiates these CD8 T cells from the CD8 T cells of other subjects. CD8 T cells of subtype CD8.2 may be characterised by downregulated expression of genes 1 to 11 listed in Table 1. In another embodiment, CD8 T cells of subtype CD8.2 may be characterised by differential (upregulated or downregulated) expression of genes 1 to 13 listed in Table 1, as indicated. In a further embodiment, CD8 T cells of subtype CD8.2 may be characterised by differential (upregulated or downregulated) expression of the genes listed in Table 1, as indicated. The level of expression of these genes in subtype CD8.2 is differentially expressed relative to the level of expression of the same genes in CD8 T cells of subtype CD8.1. The level of expression of the genes listed in Table 1 may be determinable using the primer pairs listed in Table 1. The presence of CD8 T cells of subtype CD8.2 in a subject with autoimmune disease, such as ANCA-associated vasculitis, SLE or IBD, indicates that the subject is at low risk of autoimmune disease progression.

By "CD4.1 subtype" (CD4.1 subgroup) or "4.1 subtype" (4.1 subgroup) it is meant CD4 T cells characterised by a specific gene expression profile (signature) which differentiates these CD4 T cells from the CD4 T cells of other subjects. CD4 T cells of subtype CD4.1 may be characterised by differential (upregulated or downregulated) expression of genes 1 to 10 listed in Table 2, as indicated. In another embodiment, CD4 T cells of subtype CD4.1 may be characterised by differential (upregulated or downregulated) expression of genes 1 to 12 listed in Table 2, as indicated. In a further embodiment, CD4 T cells of subtype CD4.1 may be characterised by differential (upregulated or downregulated) expression of the genes listed in Table 2, as indicated. The level of expression of these genes in subtype CD4.1 is upregulated or downregulated relative to the level of expression of the same genes in CD4 T cells of subtype CD4.2. The level of expression of the genes listed in Table 2 may be determinable using the primer pairs listed in Table 2. The presence of CD4 T cells of subtype CD4.1 in a subject with autoimmune disease, such as ANCA-associated vasculitis, indicates that the subject is at high risk of autoimmune disease progression.

By "CD4.2 subtype" (CD4.2 subgroup) or "4.2 subtype" (4.2 subgroup) it is meant CD4 T cells characterised by a specific gene expression profile (signature) which differentiates these CD4 T cells from the CD4 T cells of other subjects. CD4 T cells of subtype CD4.2 may be characterised by differential (upregulated or downregulated) expression of genes 1 to 10 listed in Table 2, as indicated. In another embodiment, CD4 T cells of subtype CD4.2 may be characterised by differential (upregulated or downregulated) expression of genes 1 to 12 listed in Table 2, as indicated. In a further embodiment, CD4 T cells of subtype CD4.2 may be characterised by differential (upregulated or downregulated) expression of the genes listed in Table 2, as indicated. The level of expression of these genes in subtype CD4.2 is upregulated or downregulated relative to the level of expression of the same genes in CD4 T cells of subtype CD4.1. The level of expression of the genes listed in Table 2 may be determinable using the primer pairs listed in Table 2. The presence of CD4 T cells of subtype CD4.2 in a subject with autoimmune disease, such as ANCA-associated vasculitis, indicates that the subject is at low risk of autoimmune disease progression.

The present inventors have surprisingly found that subjects with autoimmune disorders, such as ANCA-associated vasculitis, SLE or IBD, can be divided into two distinct groups, CD8.1 and CD8.2, on the basis of the gene expression profiles of their CD8 T cells. These subtypes are predictive of disease progression, with subjects with CD8 T cells of subtype CD8.1 being at high risk of disease progression, while subjects with CD8 T cells of subtype CD8.2 are at low risk of disease progression.

Thus, in one aspect there is provided a method of assessing whether a subject is at high or low risk of autoimmune disease progression, which method comprises establishing, by determining the expression level of one or more genes in a CD8 cell from said subject, whether said subject has a high risk (CD8.1) or low risk (CD8.2) CD8 cell subtype,
wherein a CD8.1 subtype is characterised by upregulated expression of genes 1 to 11 listed in Table 1 relative to the level of expression of the same genes in subtype CD8.2 and,
wherein a CD8.2 subtype is characterised by downregulated expression of genes 1 to 11 listed in Table 1 relative to the level of expression of the same genes in subtype CD8.1.

In another embodiment, a CD8.1 subtype may be characterised by differential expression of genes 1 to 13 listed in Table 1 relative to the level of expression of the same genes in subtype CD8.2 and, a CD8.2 subtype may be characterised by differential expression of genes 1 to 13 listed in Table 1 relative to the level of expression of the same genes in subtype CD8.1. In a further embodiment, a CD8.1 subtype may be characterised by differential expression of the genes listed in Table 1 relative to the level of expression of the same genes in subtype CD8.2 and, a CD8.2 subtype may be characterised by differential expression of the genes listed in Table 1 relative to the level of expression of the same genes in subtype CD8.1.

Gene expression levels can be determined in many different ways both directly and indirectly. For example, gene expression levels can be detected directly by detecting the mRNA transcripts of the gene in questions. Alternatively, gene expression levels can also be detected by reverse transcribing the mRNA transcripts into DNA using reverse transcriptase first before detecting the DNA transcripts thus generated. In addition, the protein(s) encoded by a particular gene can also be detected and used as an indirect measure for determining the expression level of the gene in question. The protein may be a cell surface protein (e.g. a protein expressed on the cell surface of CD4 and/or CD8 cells) or may be a secreted protein. Suitable methods for detecting the presence of mRNA, DNA and proteins are well known in the art and include: microarray analysis, polymerase chain reactions (e.g. quantitative PCR), enzyme-linked immunosorbent assays (ELISA), protein chips, flow cytometry, mass spectrometry, Western blotting, and northern blotting.

A method as described herein may comprise providing a sample obtained from the subject. The sample may for example be a whole blood sample, a peripheral blood mononuclear cell (PBMC) sample, or sample comprising CD8 and/or CD4 T cells. A sample comprising CD8 and/or CD4 T cells may be purified or unpurified. In one example, the sample is a purified sample of CD8 and/or CD4 T cells. Although analysis of PBMC gene expression profiles showed no meaningful substructure when de novo analysis was performed, the CD8 or CD4 cell subtype of a subject may be determined using e.g. a PBMC sample, as the gene signatures defining these subtypes have now been identified. Thus, in another example, the sample is a PBMC sample, e.g. a sample of unseparated PBMCs.

The method may further comprise bringing sample into contact with a reagent suitable for determining the expression level of said one or more genes, e.g. a reagent suitable for determining the expression level of said one or more genes using microarray analysis, polymerase chain reaction (e.g. quantitative PCR), enzyme-linked immunosorbent assay (ELISA), protein chips, flow cytometry, mass spectrometry, or Western blotting. For example, the reagent may be a nucleic acid primer, or pair of nucleic acid primers, suitable for determining the expression level of said one or more genes using quantitative PCR (qPCR). Exemplary primers pairs suitable for determining the expression level of the genes listed in Tables 1 and 2 are provided in these tables. Alternatively, the reagent may be an antibody suitable for determining the expression level of said one or more genes using ELISA or Western blotting.

Microarrays (gene chips) allow gene expression in two samples to be compared. Total RNA is first isolated from tissues or cells using, for example, Trizol or an RNeasy mini kit (Qiagen). Total RNA from T cells (CD8 or CD4) can be obtained, for example, by taking a blood sample from a subject and isolating the T cells using centrifugation over ficoll, followed by positive selection using magnetic beads, prior to isolating the RNA. The isolated total RNA is then reverse transcribed into double-stranded cDNA using reverse transcriptase and polyT primers and labelled using e.g. Cy3- or Cy5-dCTP. Appropriate Cy3- and Cy5-labelled samples are then pooled and hybridised to custom spotted oligonucleotide microarrays comprised of probes representing suitable genes and control features, such as the microarray described in Willcocks *et al*., 2008). Samples may be hybridised in duplicate, using a dye-swap strategy, against a common reference RNA derived from pooled PBMC samples. Following hybridisation, arrays are washed and scanned on e.g. an Agilent G2565B scanner. Suitable alternatives to the steps described above are well known in the art and would be apparent to the skilled person. The raw microarray data obtained can then be analyzed using suitable methods to determine the relative expression of relevant genes.

Quantitative real time (RT) PCR allows amplification and simultaneous quantification of a target DNA molecule. To analyze gene expression levels using quantitative PCR, the total mRNA of a cell is first isolated and reverse transcribed into DNA using reverse transcriptase. For example, mRNA levels can be determined using e.g. Taqman Gene Expression Assays (Applied Biosystems) on an ABI PRISM 7900HT instrument according to the manufacturer's instructions. Transcript abundance can then be calculated by comparison to a standard curve.

Enzyme-linked immunosorbent assay (ELISA) allows the relative amounts of proteins present in a sample to be detected. The sample is first immobilized on a solid support, such as a polystyrene microtiter plate, either directly or via an antibody specific for the protein of interest. After immobilization, the antigen is detected using an antibody specific for the target protein. Either the primary antibody used to detect the target protein may be labelled to allow detection, or the primary antibody can be detected using a suitably labelled secondary antibody. For example, the antibody may be labelled by conjugating the antibody to a reporter enzyme. In this case, the plate developed by adding a suitable enzymatic substrate to produce a visible signal. The intensity of the signal is dependent on the amount of target protein present in the sample.

Protein chips, also referred to as protein arrays or protein microarrays, allow the relative amounts of proteins present in a sample to be detected. Different capture molecules may be affixed to the chip. Examples include antibodies, antigens, enzymatic substrates, nucleotides and other proteins. Protein chips can also contain molecules that bind to a range of proteins. Protein chips are well known in the art and many different protein chips are commercially available.

Western blotting also allows the relative amounts of proteins present in a sample to be detected. The proteins present in a sample are first separated using gel electrophoresis. The proteins are then transferred to a membrane, e.g. a nitrocellulose or PVDF membrane, and detected using monoclonal or polyclonal antibodies specific to the target protein. Many different antibodies are commercially available and methods for making antibodies to a given target protein are also well established in the art. To allow detection, the antibodies specific for the protein(s) of interest, or suitable secondary antibodies, may, for example, be linked to a reporter enzyme, which drives a colourimetric reaction and produces a colour when exposed to an appropriate substrate. Other reporter enzymes include horseradish peroxidase, which produces chemiluminescence when provided with an appropriate substrate. Antibodies may also be labelled with suitable radioactive or fluorescent labels. Depending on the label used, protein levels may be determined using densitometry, spectrophotometry, photographic film, X-ray film, or a photosensor.

Flow cytometry allows the relative amounts of proteins present in e.g. a serum sample obtained from a subject to be determined. In addition, flow cytometry methods (e.g. fluorescence-activated cell sorting [FACS]) allow the number cells of a particular type present in a sample to be determined. For example, flow cytometry can be used to determined the number of T cells, e.g. memory CD8 cells, present in a sample obtained from a subject. Flow cytometry can also be used to detect or measure the level of expression of a protein of interest on the surface of cells. Detection of proteins and cells using flow cytometry normally involves first attaching a fluorescent label to the protein or cell of interest. The fluorescent label may for example be a fluorescently-labelled antibody specific for the protein or cell of interest. Many different antibodies are commercially available and methods for making antibodies specific for a protein of interest are also well established in the art.

Mass spectrometry, e.g. matrix-assisted laser desorption/ionization (MALDI) mass spectrometry, allows the identification of proteins present in a sample obtained from a subject using e.g. peptide mass finger printing. Prior to mass spectrometry the proteins present in the sample may be isolated using gel electrophoresis, e.g. SDS-PAGE, size exclusion chromatography, or two-dimensional gel electrophoresis.

Methods of assessing whether a subject is at high or low risk of autoimmune disease progression described herein may further comprise treating a subject identified as having a high risk CD8.1 subtype with a more frequent, more intense, or novel disease treatment regimen. An example of a more intense disease treatment regimen is intermittent rituximab treatment. Alternatively, or additionally, the method may also comprise treating a subject identified as having a low risk CD8.2 CD8 cell subtype with a less frequent, less intense, or novel disease treatment regimen. A more frequent or more intense disease treatment regimen may refer to a disease treatment regimen that is more frequent or more intense than the treatment normally administered during the maintenance phase of the autoimmune disease. A less frequent or less intense disease treatment regimen may refer to a disease treatment regimen that is less frequent or less intense than the treatment normally administered during the maintenance phase of the autoimmune disease. For example, "treatment" with a less frequent or less intense disease treatment regimen may comprise stopping maintenance therapy for a subject identified as having a low risk CD8.2 CD8 cell subtype. A novel disease treatment regimen may comprise treatment with a pharmaceutical agent not previously administered to the patient to treat the disease. A novel disease treatment regimen may comprise treatment with a pharmaceutical agent not normally administered during the maintenance phase of the disease. For example, the novel disease treatment regimen may be a disease treatment regimen demonstrated to be particularly effective in the relevant subtype of patients. A novel disease treatment regimen for a subject identified as having a high risk CD8.1 CD8 cell subtype may for example comprise administering a T cell depleting agent to the subject in question. A novel disease treatment regimen may comprise administering an agent which targets a gene overexpressed in the subtype in question, either by targeting (e.g. inhibiting) overexpression of the gene directly, or by targeting (e.g. inhibiting) a protein expressed by said gene. For example, a novel disease treatment regimen for a subject identified as having a high risk CD8.1 CD8 cell subtype may for example comprise administering an IL7 receptor inhibitor or IL7 receptor antagonist to the subject in question.

The present inventors have also surprisingly found that subjects with autoimmune disorders, such as ANCA-associated vasculitis, can be divided into two distinct groups, CD4.1 and CD4.2, on the basis of the gene expression profiles of their CD4 T cells. These subtypes are predictive of disease progression, with subjects with CD4 T cells of subtype 4.1 being at high risk of disease progression, while subjects with CD4 T cells of subtype 4.2 are at low risk of disease progression. Again, risk of disease progression may refer, for example, to the risk of the subject experiencing (frequent) flares of the disease after initial presentation.

Thus, in another aspect there is provided a method of assessing whether a subject is at high or low risk of autoimmune disease progression, which method comprises establishing, by determining the expression level of one or more genes in a CD4 cell from said subject, whether said subject has a high risk (CD4.1) or low risk (CD4.2) CD4 cell subtype,
wherein a CD4.1 subtype is characterised by differential expression of genes 1 to 10 listed in Table 2 relative to the level of expression of the same genes in subtype CD4.2 and,
wherein a CD4.2 subtype is characterised by differential expression of genes 1 to 10 listed in Table 2 relative to the level of expression of the same genes in subtype CD4.1.

In another embodiment, a CD4.1 subtype may be characterised by differential expression of genes 1 to 12 listed in Table 2 relative to the level of expression of the same genes in subtype CD4.2 and, a CD4.2 subtype may be characterised by differential expression of genes 1 to 12 listed in Table 2 relative to the level of expression of the same genes in subtype CD4.1. In a further embodiment, a CD4.1 subtype may be characterised by differential expression of the genes listed in Table 2 relative to the level of expression of the same genes in subtype CD4.2 and, a CD4.2 subtype may be characterised by differential expression of the genes listed in Table 2 relative to the level of expression of the same genes in subtype CD4.1.

Methods of assessing whether a subject is at high or low risk of autoimmune disease progression described herein may further comprise treating a subject identified as having a high risk CD4.1 subtype with a more frequent, more intense, or novel disease treatment regimen. An example of a more intense disease treatment regimen is intermittent rituximab treatment. Alternatively, or additionally, the method may also comprise treating a subject identified as having a low risk CD4.2 CD4 cell subtype with a less frequent, less intense, or novel disease treatment regimen. A more frequent or more intense disease treatment regimen may refer to a disease treatment regimen that is more frequent or more intense than the treatment normally administered during the maintenance phase of the autoimmune disease. A less frequent or less intense disease treatment regimen may refer to a disease treatment regimen that is less frequent or less intense than the treatment normally administered during the maintenance phase of the autoimmune disease. For example, "treatment" with a less frequent or less intense disease treatment regimen may comprise stopping maintenance therapy for a subject identified as having a low risk CD4.2 CD4 cell subtype. A novel disease treatment regimen may comprise treatment with a pharmaceutical agent not previously administered to the patient to treat the disease. A novel disease treatment regimen may comprise treatment with a pharmaceutical agent not normally administered during the maintenance phase of the disease. The novel disease treatment regimen may be a disease treatment regimen demonstrated to be particularly effective in the relevant subtype of patients. A novel disease treatment regimen for a subject identified as having a high risk CD4.1 CD4 cell subtype may for example comprise administering a T cell depleting agent to the subject in question. A novel disease treatment regimen may comprise administering an agent which targets a gene overexpressed in the subtype in question, either by targeting (e.g. inhibiting) overexpression of the gene directly, or by targeting (e.g. inhibiting) a protein expressed by said gene. For example, a novel disease treatment regimen for a subject identified as having a high risk CD4.1 CD4 cell subtype may for example comprise administering an IL7 receptor inhibitor or IL7 receptor antagonist to the subject in question.

The CD8 T cells of subjects in the normal population can also be divided into two subtypes, CD8.1 and CD8.2, on the basis of their gene expression profiles. The subjects do not have autoimmune disorders, so these differences are not unique to subjects with autoimmune disease but present in the population generally. Given the differences in autoimmune disease progression observed in subjects with CD8 T cells of subtypes 8.1 and 8.2, these subtypes may also affect immune responses in normal healthy subjects, e.g. in response to infection and/or vaccination. The same also applies to individuals with CD4 T cell subtypes 4.1 and 4.2.

Specifically, CD8 and CD4 T cells are known to play a central role in immune responses and thus patients with CD8.1 or CD4.1 cell subtypes are likely to respond better to infections and/or vaccinations than patients with CD8.2 or CD4.2 cell subtypes. Specifically, patients with subtypes CD8.1 or CD4.1 are likely to clear infections more quickly and/or are likely to mount a more effective immune response to vaccines than individuals with subtypes CD8.2 or CD4.2, respectively. In particular, patients with subtype CD4.1 are likely to respond better to vaccines designed to elicit antibody responses than individuals with subtype CD4.2, and vice versa. Most vaccines are designed to elicit antibody responses and such vaccines include e.g. MMR (measles mumps rubella) and flu vaccines. Patients with subtype CD8.1 may respond better to vaccines designed to elicit CD8 responses than patients with subtype CD8.2. Such vaccines include, for example, malaria vaccines currently being tested in trials.

Determining a subject's CD8 subtype may therefore allow a subject's likely response to infections and/or vaccinations to be predicted, and allow treatments, such as antiviral therapies or vaccines, to be tailored to a particular subjects needs. For example, subjects with a CD8.2 subtype may respond less well to infections and/or vaccinations than subjects with CD8.1 subtype. The subject may, e.g. be a subject infected with human immunodeficiency virus (HIV). In this case, determining the subject's CD8 subtype may allow disease progression and/or outcome to be predicted and thereby also allow the optimal antiretroviral therapy for the patient to be determined. The same is also likely to be true of subjects with CD4 T cells of subtypes CD4.1 and CD4.2, respectively.

In addition, both CD8 and CD4 T cells are known to be involved in the acute rejection of transplants. Thus, CD8 subtypes 8.1 and 8.2 are also likely to be predictive of transplant rejection in transplant patients, in particular acute transplant rejection. The transplant patients may, for example, be renal transplant patients. Specifically, patients with a CD8.1 CD8 cell subtype are likely to be at greater risk of transplant rejection, e.g. acute transplant rejection, than patients with a CD8.2 CD8 cell subtype. This is shown in Example 6. Similarly, patients with a CD4.1 CD4 cell subtype are likely to be at greater risk of transplant rejection, e.g. acute transplant rejection, than patients with a CD4.2 CD4 cell subtype. Thus, determining a subject's CD8 or CD4 T cell subtype may allow immunosuppressive therapy designed to prevent transplant rejection to be tailored to a particular subjects needs. In particular, subjects with a CD8.1 or CD4.1 T cell subtype may benefit from more intense immunosuppressive therapy than patients with a CD8.2 or CD4.2 T cell subtype, respectively.

Also disclosed herein is a method of assessing whether a subject has a CD8.1 or CD8.2 CD8 cell subtype, which method comprises determining the expression level of one or more genes in a CD8 cell from said subject,
wherein a CD8.1 subtype is characterised by upregulated expression of genes 1 to 11 listed in Table 1 relative to the level of expression of the same genes in subtype CD8.2 and,
wherein a CD8.2 subtype is characterised by downregulated expression of genes 1 to 11 listed in Table 1 relative to the level of expression of the same genes in subtype CD8.1.

A CD8.1 subtype may be characterised by differential expression of genes 1 to 13 listed in Table 1 relative to the level of expression of the same genes in subtype CD8.2 and, a CD8.2 subtype may be characterised by differential expression of genes 1 to 13 listed in Table 1 relative to the level of expression of the same genes in subtype CD8.1.
A CD8.1 subtype may be characterised by differential expression of the genes listed in Table 1 relative to the level of expression of the same genes in subtype CD8.2 and, a CD8.2 subtype may be characterised by differential expression of the genes listed in Table 1 relative to the level of expression of the same genes in subtype CD8.1.

Also disclosed herein is a method of assessing whether a subject has a CD4.1 or CD4.2 CD4 cell subtype, which method comprises determining the expression level of one or more genes in a CD4 cell from said subject,
wherein a CD4.1 subtype is characterised by differential expression of genes 1 to 10 listed in Table 2 relative to the level of expression of the same genes in subtype CD4.2 and,
wherein a CD4.2 subtype is characterised by differential expression of genes 1 to 10 listed in Table 2 relative to the level of expression of the same genes in subtype CD4.1.

A CD4.1 subtype may be characterised by differential expression of genes 1 to 12 listed in Table 2 relative to the level of expression of the same genes in subtype CD4.2 and, a CD4.2 subtype may be characterised by differential expression of genes 1 to 12 listed in Table 2 relative to the level of expression of the same genes in subtype CD4.1.
A CD4.1 subtype may be characterised by differential expression of the genes listed in Table 2 relative to the level of expression of the same genes in subtype CD4.2 and, a CD4.2 subtype may be characterised by differential expression of the genes listed in Table 2 relative to the level of expression of the same genes in subtype CD4.1.

As discussed above, methods for assessing whether a subject has a CD8.1 or CD8.2 CD8 cell subtype, as described herein, may be used for assessing whether a subject is more likely or less likely to mount an effective immune response to vaccinations and/or infections, by determining whether said individual has a CD8.1 or CD8.2 CD8 cell subtype. An individual with a CD8.1 CD8 cell subtype is more likely to mount and effective immune response to vaccinations and/or infections. An individual with a CD8.2 CD8 cell subtype is less likely to mount and effective immune response to vaccinations and/or infections. Similarly, methods for assessing whether a subject has a CD4.1 or CD4.2 CD4 cell subtype, as described herein, may be used for assessing whether a subject is more likely or less likely to mount an effective immune response to vaccinations and/or infections, by determining whether said individual has a CD4.1 or CD4.2 CD4 cell subtype. An individual with a CD4.1 CD4 cell subtype is more likely to mount and effective immune response to vaccinations and/or infections. An individual with a CD4.2 CD4 cell subtype is less likely to mount and effective immune response to vaccinations and/or infections.

Methods for assessing whether a subject has a CD8.1 or CD8.2 CD8 cell subtype, as described herein, may be used for assessing whether a subject is at high risk or low risk of transplant rejection, e.g. acute transplant rejection, by determining whether said individual has a high risk (CD8.1) or low risk (CD8.2) CD8 cell subtype. Similarly, methods for assessing whether a subject has a CD4.1 or CD4.2 CD4 cell subtype, as described herein, may be used for assessing whether a subject is at high risk or low risk of transplant rejection, e.g. acute transplant rejection, by determining whether said individual has a high risk (CD4.1) or low risk (CD4.2) CD8 cell subtype. In this case, the individual may be a transplant patient, e.g. a renal transplant patient.

Subjects can be divided into (or classified as) subtypes CD8.1 and CD8.2 and CD4.1 and CD4.2 based on the specific gene expression patterns of their T cells. The relative expression of genes 1 to 11 listed in Table 1 may, for example, be used to determine whether a subject has CD8 T cells of subtype 8.1 or 8.2 by comparing the expression levels of these genes in different subjects. Alternatively, the relative expression of genes 1 to 13 listed in Table 1 may be used to determine whether a subject has CD8 T cells of subtype 8.1 or 8.2 by comparing the expression levels of these genes in different subjects. As a further alternative, the relative expression of the genes listed in Table 1 may be used to determine whether a subject has CD8 T cells of subtype 8.1 or 8.2 by comparing the expression levels of these genes in different subjects. Similarly, the relative expression of genes 1 to 10 listed in Table 2 may, for example, be used to determine whether a subject has CD4 T cells of subtype 4.1 or 4.2 by comparing the expression levels of these genes in different subjects. Alternatively, the relative expression of genes 1 to 12 listed in Table 2 may be used to determine whether a subject has CD4 T cells of subtype 4.1 or 4.2 by comparing the expression levels of these genes in different subjects. As a further alternative, the relative expression of the genes listed in Table 2 may be used to determine whether a subject has CD4 T cells of subtype 4.1 or 4.2 by comparing the expression levels of these genes in different subjects.

While it is possible to determine the expression levels of all of the genes listed in the relevant Tables to determine whether a subject has a CD8.1 or CD8.2 CD8 cell subtype, or a CD4.1 or CD4.2 CD4 cell subtype, respectively, this is not necessary. Determining the expression level of as few as one of the genes listed in the relevant tables may be sufficient to determine to which subtype a subjects T cells belong.

Examples 4, 5, 6, 7 and 8 show that combinations of, for example, two or three genes can be used to accurately predict the two subgroups. Accordingly, a method of assessing whether a subject has a CD8.1 or CD8.2 CD8 cell subtype may comprise determining the expression level of one or more, two or more, three or more, four or more, five or more, six or more, seven or more, eight or more, nine or more, ten or more, eleven or more, twelve or more thirteen or more or all fourteen of the genes listed in Table 1. In a preferred embodiment, a method of assessing whether a subject has a CD8.1 or CD8.2 CD8 cell subtype may comprise determining the expression level of one, two, or three of the genes selected from the group of: ITGA2 (SEQ ID NO:34), PTPN22 (SEQ ID NO:37) and NOTCH1 (SEQ ID NO:40). Thus, for example, a method of assessing whether a subject has a CD8.1 or CD8.2 CD8 cell subtype may comprise determining the expression level of: ITGA2; PTPN22; NOTCH1; ITGA2 and PTPN22; ITGA2 and NOTCH1; PTPN22 and NOTCH1; or ITGA2, PTPN22 and NOTCH1.

In another example, a method of assessing whether a subject has a CD8.1 or CD8.2 CD8 cell subtype may comprise determining the expression level of one, two, or three of the genes selected from the group of: MCM6 (SEQ ID NO:28), POLR2H (SEQ ID NO:4) and IL7R (SEQ ID NO:22). Thus, a method of assessing whether a subject has a CD8.1 or CD8.2 CD8 cell subtype may comprise determining the expression level of: MCM6; POLR2H; IL7R; MCM6 and POLR2H; MCM6 and IL7R; POLR2H and IL7R; or MCM6, POLR2H and IL7R.

In a further example, a method of assessing whether a subject has a CD8.1 or CD8.2 CD8 cell subtype may comprise determining the expression level of one, two, or three of the genes selected from the group of: MCM6 (SEQ ID NO:28), POLR2H (SEQ ID NO:4) and ITGA2 (SEQ ID NO:34). Thus, a method of assessing whether a subject has a CD8.1 or CD8.2 CD8 cell subtype may comprise determining the expression level of: MCM6; POLR2H; ITGA2; MCM6 and POLR2H; MCM6 and ITGA2; POLR2H and ITGA2; or MCM6, POLR2H and ITGA2.

In a further example, a method of assessing whether a subject has a CD8.1 or CD8.2 CD8 cell subtype may comprise determining the expression level of one or two genes selected from the group of: Jak1 (SEQ ID NO:67) and GZMK (SEQ ID NO:68). Thus, a method of assessing whether a subject has a CD8.1 or CD8.2 CD8 cell subtype may comprise determining the expression level of: Jak1; GZMK; or Jak1 and GZMK.

A method of assessing whether a subject has a CD4.1 or CD4.2 CD4 cell subtype may comprise determining the expression level of one or more, two or more, three or more, four or more, five or more, six or more, seven or more, eight or more, nine or more, ten or more, eleven or more, twelve or more, or all thirteen of the genes listed in Table 2. In a preferred embodiment, a method of assessing whether a subject has a CD4.1 or CD4.2 CD4 cell subtype may comprise determining the expression level of one, two, or three of the genes selected from the group of: ITGA2 (SEQ ID NO:34), PTPN22 (SEQ ID NO:37) and NOTCH1 (SEQ ID NO:40). Thus, for example, a method of assessing whether a subject has a CD4.1 or CD4.2 CD4 cell subtype may comprise determining the expression level of: ITGA2; PTPN22; NOTCH1; ITGA2 and PTPN22; ITGA2 and NOTCH1; PTPN22 and NOTCH1; or ITGA2, PTPN22 and NOTCH1.

In a further example, a method of assessing whether a subject has a CD4.1 or CD4.2 CD4 cell subtype may comprise determining the expression level of one or two genes selected from the group of: PCAF (NM_003884; GI 8850), ANKRD32 (NM_032290; GI 84250), ZNF26 (NM_019591; GI 7574). Thus, a method of assessing whether a subject has a CD4.1 or CD4.2 CD4 cell subtype may comprise determining the expression level of: PCAF; ANKRD32; ZNF26; PCAF and ANKRD32; PCAF and ZNF26; ANKRD32 and ZNF26; or PCAF, ANKRD32 and ZNF26.

The genes listed in Tables 1 are not exhaustive and there are additional genes which are also differentially expressed in CD8 cells of subtypes CD8.1 and CD8.2. Thus, in another aspect there is provided a method of identifying genes differentially expressed in subjects with a high risk and subjects with a low risk of autoimmune disease progression. The method may comprise the steps of:
(i) determining the level of CD8 cell gene expression in subjects with autoimmune disease using microarray analysis,
(ii) dividing the subjects into two groups based on their CD8 cell gene expression levels using a clustering method (e.g. principal components analysis, hierarchical clustering, self-organising maps, and/or a k-means algorithm),
(iii) identifying the group with the higher level of autoimmune disease progression, and
(iv) identifying the genes differentially expressed in subjects with a high risk (CD8.1) CD8 cell subtype and subjects with a low risk (CD8.2) CD8 cell subtype, as set out in claim 12.

Genes which are differentially expressed in CD8 cells of subtypes CD8.1 and CD8.2 in normal healthy subjects can be identified in a similar manner. Thus, also disclosed herein is a method of identifying genes differentially expressed in subjects with a CD8.1 or CD8.2 CD8 cell subtype, comprising:
(i) determining the level of CD8 cell gene expression in subjects using microarray analysis,
(ii) dividing the subjects into two groups based on their CD8 cell gene expression levels using a clustering method (e.g. principal components analysis, hierarchical clustering, self-organising maps, and/or a k-means algorithm),
(iii) identifying the genes differentially expressed in subjects with a CD8.1 subtype and subjects with a CD8.2 subtype. Preferably, the subjects do not have autoimmune disease.

Genes identified using the above methods may be used to establish whether a subject has a CD8.1 or CD8.2 CD8 cell subtype by determining the expression level of one or more of said genes.

Similarly, the genes listed in Tables 2 are also not exhaustive and there are additional genes which are also differentially expressed in CD4 cells of subtypes CD4.1 and CD4.2. Thus, in a further aspect there is provided a method of identifying genes differentially expressed in subjects with a high risk and subjects with a low risk of autoimmune disease progression. The method may comprise the steps of:
(i) determining the level of CD4 cell gene expression in subjects with autoimmune disease using microarray analysis,
(ii) dividing the subjects into two groups based on their CD4 cell gene expression levels using a clustering method (e.g. principal components analysis, hierarchical clustering, self-organising maps, and/or a k-means algorithm),
(iii) identifying the group with the higher level of autoimmune disease progression, and
(iv) identifying the genes differentially expressed in subjects with a high risk (CD4.1) CD4 cell subtype and subjects with a low risk (CD4.2) CD4 cell subtype, as set out in claim 14.

Genes which are differentially expressed in CD4 cells of subtypes CD4.1 and CD4.2 in normal healthy subjects can also be identified in a similar manner. Thus, also disclosed herein is a method of identifying genes differentially expressed in subjects with a CD4.1 or CD4.2 CD4 cell subtype, comprising:
(i) determining the level of CD4 cell gene expression in subjects using microarray analysis,
(ii) dividing the subjects into two groups based on their CD4 cell gene expression levels using a clustering method (e.g. principal components analysis, hierarchical clustering, self-organising maps, and/or a k-means algorithm),
(iii) identifying the genes differentially expressed in subjects with a CD4.1 subtype and subjects with a CD4.2 subtype. Preferably, the subjects do not have autoimmune disease.

Genes identified using the above methods may be used to establish whether a subject has a CD4.1 or CD4.2 CD4 cell subtype by determining the expression level of one or more of said genes.

Subjects can also be divided in two distinct groups based on the number of memory CD8 cells that can be detected in a sample obtained from the subject. Specifically, subjects with a high risk (CD8.1) CD8 cell subtype have higher numbers of memory CD8 cells than in subjects with a low risk (CD8.2) CD8 cell subtype. Thus, also disclosed herein is a method of assessing whether a subject is at high or low risk of autoimmune disease progression, which method comprises determining the number of memory CD8 cells (e.g. effector memory T cells, central memory T cells, and/or CD3⁺CD8⁺CD45RA⁻ T cells) in a sample obtained from said subject and comparing said number to a reference number,
wherein a subject at high risk of autoimmune disease progression is characterised by an increased number of memory CD8 cells relative to the number of memory CD8 cells in a subject at low risk of autoimmune disease progression, and
wherein a subject at low risk of autoimmune disease progression is characterised by a decreased number of memory CD8 cells relative to the number of memory CD8 cells in a subject at high risk of autoimmune disease progression.

For example, the reference number may be the number of memory CD8 cells characteristic of a subject with a high risk of autoimmune disease progression or the number of memory CD8 cells characteristic of a subject with a low risk of autoimmune disease progression.
Also disclosed herein is a method of assessing whether a subject has a CD8.1 or CD8.2 CD8 cell subtype, which method comprises determining the number of memory CD8 cells (e.g. effector memory T cells, central memory T cells, and/or CD3*CD8*CD45RA⁻ T cells) in a sample obtained from said subject and comparing said number to a reference number,
wherein a subject with a CD8.1 subtype is characterised by an increased number of memory CD8 cells relative to the number of memory CD8 cells in a subject with a CD8.2 subtype, and
wherein a subject with a CD8.2 subtype is characterised by a decreased number of memory CD8 cells relative to the number of memory CD8 cells in a subject with a CD8.1 subtype. The subject may be a subject which does not have autoimmune disease. The reference number may, for example, be the number of memory CD8 cells characteristic of a subject with a CD8.1 subtype or the number of memory CD8 cells characteristic of a subject CD8.2 subtype.

The number of memory CD8 cells in a sample obtained from a subject can be detected using e.g. flow cytometry.

Subjects may also be divided in two distinct groups based on the number of memory CD4 cells that can be detected in a sample obtained from the subject. Specifically, subjects with a high risk (CD4.1) CD4 cell subtype have higher numbers of memory CD4 cells than in subjects with a low risk (CD4.2) CD8 cell subtype. Thus, also disclosed herein is a method of assessing whether a subject is at high or low risk of autoimmune disease progression, which method comprises determining the number of memory CD4 cells (e.g. effector memory T cells, or central memory T cells, and/or CD3⁺CD4⁺CD45RA⁻ T cells) in a sample obtained from said subject and comparing said number to a reference number,
wherein a subject at high risk of autoimmune disease progression is characterised by an increased number of memory CD4 cells relative to the number of memory CD4 cells in a subject at low risk of autoimmune disease progression.

For example, the reference number may be the number of memory CD4 cells characteristic of a subject with a high risk of autoimmune disease progression or the number of memory CD4 cells characteristic of a subject with a low risk of autoimmune disease progression.

Further disclosed herein is a method of assessing whether a subject has a CD4.1 or CD4.2 CD4 cell subtype, which method comprises determining the number of memory CD4 cells (e.g. effector memory T cells, or central memory T cells, and/or CD3⁺CD4⁺CD45RA⁻ T cells) in a sample obtained from said subject and comparing said number to a reference number,
wherein a subject with a CD4.1 subtype is characterised by an increased number of memory CD4 cells relative to the number of memory CD4 cells in a subject with a CD4.2 subtype. The subject may be a subject which does not have autoimmune disease. The reference number may, for example, be the number of memory CD4 cells characteristic of a subject with a CD4.1 subtype or the number of memory CD4 cells characteristic of a subject CD4.2 subtype.

The number of memory CD4 cells in a sample obtained from a subject can be detected using e.g. flow cytometry.

Several of the genes disclosed herein as being differentially expressed in CD8 cells of subtypes CD8.1 and CD8.2 are expressed on the cell surface. In one example, the CD8 cell subtype of a subject may therefore be determined by determining the relative amount of one or more of these proteins expressed on the cell surface of CD8 cell obtained form a subject. Thus, also disclosed herein is a method of assessing whether a subject is at high or low risk of autoimmune disease progression which method comprises establishing, by determining the level of expression of one or more proteins on the surface of a CD8 cell from said subject, whether said subject has a high risk (CD8.1) or low risk (CD8.2) CD8 cell subtype,
wherein a CD8.1 subtype is characterised by differential expression of the protein relative to the level of expression of the protein in subtype CD8.2 and,
wherein a CD8.2 subtype is characterised by differential expression of the genes listed relative to the level of expression of the protein in subtype CD8.1.

Also disclosed herein is a method of assessing whether a subject has a CD8.1 or CD8.2 CD8 cell subtype, which method comprises determining the level of expression of one or more proteins on the surface of a CD8 cell from said subject,
wherein a CD8.1 subtype is characterised by differential expression of the protein relative to the level of expression of the protein in subtype CD8.2 and,
wherein a CD8.2 subtype is characterised by differential expression of the genes listed relative to the level of expression of the protein in subtype CD8.1.

The protein may be a cell surface protein listed in Table 1. For example, the protein may be the IL7 receptor. In this case, a CD8.1 subtype may be characterised by upregulated expression of the IL7 receptor relative to the level of IL7 receptor expression in subtype CD8.2 and, a CD8.2 subtype may be characterised by downregulated expression of the IL7 receptor relative to the level of IL7 receptor expression in subtype CD8.1.

Other proteins expressed on the cell surface include the proteins expressed by the genes ITGA2 and NOTCH1. Thus, a CD8.1 subtype may be characterised by downregulated expression of the protein expressed by the gene ITGA2 and/or the protein expressed by the gene NOTCH1 relative to the level of expression of the protein(s) in subtype CD8.2 and, a CD8.2 subtype may be characterised by upregulated expression of the protein expressed by the gene ITGA2 and/or the protein expressed by the gene NOTCH1 relative to the level of expression of the same protein(s) in subtype CD8.1.

Similarly, several of the genes disclosed herein as being differentially expressed in CD4 cells of subtypes CD4.1 and CD4.2 are also expressed on the cell surface. In one example, the CD4 cell subtype of a subject may therefore be determined by determining the relative amount of one or more of these proteins expressed on the cell surface of CD4 cell obtained from a subject. Thus, also disclosed herein is a method of assessing whether a subject is at high or low risk of autoimmune disease progression, which method comprises establishing, by determining the level of expression of one or more proteins on the surface of a CD4 cell from said subject, whether said subject has a high risk (CD4.1) or low risk (CD4.2) CD4 cell subtype,
wherein a CD4.1 subtype is characterised by differential expression of the protein relative to the level of expression of the protein in subtype CD4.2 and,
wherein a CD4.2 subtype is characterised by differential expression of the genes listed relative to the level of expression of the protein in subtype CD4.1.

Also disclosed herein is a method of assessing whether a subject has a CD4.1 or CD4.2 CD4 cell subtype, which method comprises determining the level of expression of one or more proteins on the surface of a CD4 cell from said subject, ,
wherein a CD4.1 subtype is characterised by differential expression of the protein relative to the level of expression of the protein in subtype CD4.2 and,
wherein a CD4.2 subtype is characterised by differential expression of the genes listed relative to the level of expression of the protein in subtype CD4.1.

The protein may be a cell surface protein listed in Table 2. For example, the protein may be the IL7 receptor. In this case, a CD4.1 subtype may be characterised by upregulated expression of the IL7 receptor relative to the level of IL7 receptor expression in subtype CD4.2 and, a CD4.2 subtype may be characterised by downregulated expression of the IL7 receptor relative to the level of IL7 receptor expression in subtype CD4.1. Alternatively, the protein may be IL2 receptor. In this case, a CD4.1 subtype may be characterised by upregulated expression of the IL2 receptor relative to the level of IL2 receptor expression in subtype CD4.2 and, a CD4.2 subtype may be characterised by downregulated expression of the IL2 receptor relative to the level of IL2 receptor expression in subtype CD4.1.

Other proteins expressed on the cell surface include the proteins expressed by the genes ITGA2 and NOTCH1. Thus, a CD4.1 subtype may be characterised by downregulated expression of the protein expressed by the gene ITGA2 and/or the protein expressed by the gene NOTCH1 relative to the level of expression of the protein(s) in subtype CD4.2 and, a CD4.2 subtype may be characterised by upregulated expression of the protein expressed by the gene ITGA2 and/or the protein expressed by the gene NOTCH1 relative to the level of expression of the same protein(s) in subtype CD4.1.

The expression level of a protein of a cell surface protein may be determined using flow cytometry.

Several of the genes disclosed herein as being differentially expressed in CD8 cells of subtypes CD8.1 and CD8.2 are secreted. In one example, the CD8 cell subtype of a subject may therefore be determined by determining the relative amount of one or more of these (secreted) proteins in a sample, e.g. a serum sample, obtained from a subject. Thus, also disclosed herein is a method of assessing whether a subject is at high or low risk of autoimmune disease progression, which method comprises establishing, by determining level of expression of one or more proteins in a sample (e.g. a serum sample) obtained from said subject, whether said subject has a high risk (CD8.1) or low risk (CD8.2) CD8 cell subtype,
wherein a CD8.1 subtype is characterised by differential expression of the protein relative to the level of expression of the protein in subtype CD8.2 and,
wherein a CD8.2 subtype is characterised by differential expression of the genes listed relative to the level of expression of the protein in subtype CD8.1.

The protein may, for example, be soluble IL7 receptor.

Also disclosed herein is a method of assessing whether a subject has a CD8.1 or CD8.2 CD8 cell subtype, which method comprises determining the level of expression of one or more proteins in a sample (e.g. a serum sample) obtained from said subject,
wherein a CD8.1 subtype is characterised by differential expression of the protein relative to the level of expression of the protein in subtype CD8.2 and,
wherein a CD8.2 subtype is characterised by differential expression of the genes listed relative to the level of expression of the protein in subtype CD8.1.

Similarly, several of the genes disclosed herein as being differentially expressed in CD4 cells of subtypes CD4.1 and CD4.2 are secreted. In one example, the CD4 cell subtype of a subject may therefore be determined by determining the relative amount of one or more of these proteins in a sample, e.g. a serum sample, obtained from a subject. Thus, also disclosed herein is method of assessing whether a subject is at high or low risk of autoimmune disease progression, which method comprises establishing, by determining the level of expression of one or more proteins in a sample (e.g. a serum sample) obtained from said subject, whether said subject has a high risk (CD4.1) or low risk (CD4.2) CD4 cell subtype,
wherein a CD4.1 subtype is characterised by differential expression of the protein relative to the level of expression of the protein in subtype CD4.2 and,
wherein a CD4.2 subtype is characterised by differential expression of the genes listed relative to the level of expression of the protein in subtype CD4.1.

Also disclosed herein is a method of assessing whether a subject has a CD4.1 or CD4.2 CD4 cell subtype, which method comprises determining the level of expression of one or more proteins in a sample (e.g. a serum sample) obtained from said subject,
wherein a CD4.1 subtype is characterised by differential expression of the protein relative to the level of expression of the protein in subtype CD4.2 and,
wherein a CD4:2 subtype is characterised by differential expression of the genes listed relative to the level of expression of the protein in subtype CD4.1.

Expression of a protein in a sample may be determined using an enzyme-linked immunosorbent assay (ELISA), western blotting, mass-spectrometry, or flow cytometry.

Also disclosed herein are kits for assessing whether a subject has a high risk (CD8.1) or low risk (CD8.2) CD8 cell subtype, or a high risk (CD4.1) or low risk (CD4.2) CD4 cell subtype.

Thus, in one example there is disclosed a kit for assessing whether a subject is at high or low risk of autoimmune disease progression, wherein said kit comprises reagents for establishing the expression level of one or more genes in a CD8 cell from said subject, for determining whether said subject has a high risk (CD8.1) or low risk (CD8.2) CD8 cell subtype,
wherein a CD8.1 subtype is characterised by upregulated expression of genes 1 to 11 listed in Table 1 relative to the level of expression of the same genes in subtype CD8.2 and,
wherein a CD8.2 subtype is characterised by downregulated expression of genes 1 to 11 listed In Table 1 relative to the level of expression of the same genes in subtype CD8.1.

A CD8.1 subtype may be characterised by differential expression of genes 1 to 13 listed in Table 1 relative to the level of expression of the same genes in subtype CD8.2 and, a CD8.2 subtype may be characterised by differential expression of genes 1 to 13 listed in Table 1 relative to the level of expression of the same genes in subtype CD8.1. In a further embodiment, a CD8.1 subtype may be characterised by differential expression of the genes listed in Table 1 relative to the level of expression of the same genes in subtype CD8.2 and, a CD8.2 subtype may be characterised by differential expression of the genes listed in Table 1 relative to the level of expression of the same genes in subtype CD8.1.
Such kits may, for example, be used to for assessing whether a subject is at high or low risk of autoimmune disease progression by determining whether said subject has a high risk (CD8.1) or low risk (CD8.2) CD8 cell subtype

Also disclosed is a kit for assessing whether a subject is at high or low risk of autoimmune disease progression, wherein said kit comprises reagents for establishing the expression level of one or more genes in a CD4 cell from said subject, for determining whether said subject has a high risk (CD4.1) or low risk (CD4.2) CD4 cell subtype,
wherein a CD4.1 subtype is characterised by differential expression of genes 1 to 10 listed in Table 2 relative to the level of expression of the same genes in subtype CD4.2 and,
wherein a CD4.2 subtype is characterised by differential expression of genes 1 to 10 listed in Table 2 relative to the level of expression of the same genes in subtype CD4.1.

A CD4.1 subtype may be characterised by differential expression of genes 1 to 12 listed in Table 2 relative to the level of expression of the same genes in subtype CD4.2 and, a CD4.2 subtype may be characterised by differential expression of genes 1 to 12 listed in Table 2 relative to the level of expression of the same genes in subtype CD4.1. In a further embodiment, a CD4.1 subtype may be characterised by differential expression of the genes listed in Table 2 relative to the level of expression of the same genes in subtype CD4.2 and, a CD4.2 subtype may be characterised by differential expression of the genes listed in Table 2 relative to the level of expression of the same genes in subtype CD4.1.
Such kits may, for example, be used to for assessing whether a subject is at high or low risk of autoimmune disease progression by determining whether said subject has a high risk (CD4.1) or low risk (CD4.2) CD4 cell subtype

Also disclosed herein are are kits for assessing whether a subject has a CD8.1 or CD8.2 CD8 cell subtype, wherein the subject may be a normal healthy subject not suffering from autoimmune disease.

Thus, also disclosed herein is a kit for assessing whether a subject has a CD8.1 or CD8.2 CD8 cell subtype, wherein said kit comprises reagents for establishing the expression level of one or more genes in a CD8 cell from said subject,
wherein a CD8.1 subtype is characterised by upregulated expression of genes 1 to 11 listed in Table 1 relative to the level of expression of the same genes in subtype CD8.2 and,
wherein a CD8.2 subtype is characterised by downregulated expression of genes 1 to 11 listed in Table 1 relative to the level of expression of the same genes in subtype CD8.1.

A CD8.1 subtype may be characterised by differential expression of genes 1 to 13 listed in Table 1 relative to the level of expression of the same genes in subtype CD8.2 and, a CD8.2 subtype may be characterised by differential expression of genes 1 to 13 listed in Table 1 relative to the level of expression of the same genes in subtype CD8.1. In a further embodiment, a CD8.1 subtype may be characterised by differential expression of the genes listed in Table 1 relative to the level of expression of the same genes in subtype CD8.2 and, a CD8.2 subtype may be characterised by differential expression of the genes listed in Table 1 relative to the level of expression of the same genes in subtype CD8.1.
The present invention also provides kits for assessing whether a subject has a CD4.1 or CD4.2 CD4 cell subtype, wherein the subject may be a normal healthy subject not suffering from autoimmune disease.

Thus, also disclosed herein is a kit for assessing whether a subject has a CD4.1 or CD4.2 CD4 cell subtype, wherein said kit comprises reagents for establishing the expression level of one or more genes in a CD4 cell from said subject,
wherein a CD4.1 subtype is characterised by differential expression of genes 1 to 10 listed in Table 2 relative to the level of expression of the same genes in subtype CD4.2 and,
wherein a CD4.2 subtype is characterised by differential expression of genes 1 to 10 listed in Table 2 relative to the level of expression of the same genes in subtype CD4.1.

A CD4.1 subtype may be characterised by differential expression of genes 1 to 12 listed in Table 2 relative to the level of expression of the same genes in subtype CD4.2 and, a CD4.2 subtype may be characterised by differential expression of genes 1 to 12 listed in Table 2 relative to the level of expression of the same genes in subtype CD4.1. In a further embodiment, a CD4.1 subtype may be characterised by differential expression of the genes listed in Table 2 relative to the level of expression of the same genes in subtype CD4.2 and, a CD4.2 subtype may be characterised by differential expression of the genes listed in Table 2 relative to the level of expression of the same genes in subtype CD4.1.

Such kits may, for example, be used for determining the likely immune response of a subject not suffering from autoimmune disease to infection and/or vaccination. Alternatively, such kits may be used for determining the likely response of a subject to transplantation. As described above, the kits of the invention may comprise reagents for establishing the expression level of one or more genes in a CD8 and/or CD4 cell from said subject. Such reagents may comprise reagents suitable for performing a method capable of determining the expression level of one or more genes of interest. For example, the reagents may comprise reagents suitable for determining the expression level of one or more genes of interest using microarray analysis, quantitative PCR, ELISAs, and/or western blotting.

For example, a kit for assessing whether a subject has a CD8.1 or CD8.2 CD8 cell subtype may comprise primers suitable for determining the level of expression of a gene which is differentially expressed in CD8 cells of subtypes CD8.1 and CD8.2, using e.g. quantitative PCR. Genes which are differentially expressed in these subtypes include the genes listed in Table 1. Thus, in one example a kit for assessing whether a subject has a CD8.1 or CD8.2 CD8 cell subtype may comprise primers suitable for determining the level of expression of one or more of the genes listed in Table 1. The sequences of exemplary primer pairs suitable for determining the expression level of these genes are detailed in Table 1. In one example, a kit may comprise primers suitable for determining the level of expression of two or more, three or more, four or more, five or more, six or more, seven or more, eight or more, nine or more, ten or more, or eleven of the genes listed in Table 1. Preferably, the kit comprises primers for determining the level of expression of two or more of the genes listed in Table 1.

A kit for assessing whether a subject has a CD8.1 or CD8.2 CD8 cell subtype may comprise primers suitable for determining the level of expression of one, two, or three of the genes selected from the group of: ITGA2 (SEQ ID NO:34), PTPN22 (SEQ ID NO:37) and NOTCH1 (SEQ ID NO:40). Thus, a kit for assessing whether a subject has a CD8.1 or CD8.2 CD8 cell subtype may comprise primers suitable for determining the level of expression of: ITGA2; PTPN22; NOTCH1; ITGA2 and PTPN22; ITGA2 and NOTCH1; PTPN22 and NOTCH1; or ITGA2, PTPN22 and NOTCH1.

A kit for assessing whether a subject has a CD8.1 or CD8.2 CD8 cell subtype may comprise primers suitable for determining the level of expression of one, two, or three of the genes selected from the group of: MCM6 (SEQ ID NO:28), POLR2H (SEQ ID NO:4) and IL7R (SEQ ID NO:22). Thus, a kit for assessing whether a subject has a CD8.1 or CD8.2 CD8 cell subtype may comprise primers suitable for determining the level of expression of: MCM6; POLR2H; IL7R; MCM6 and POLR2H; MCM6 and IL7R; POLR2H and IL7R; or MCM6, POLR2H and IL7R.

A kit for assessing whether a subject has a CD8.1 or CD8.2 CD8 cell subtype may comprise primers suitable for determining the level of expression of one, two, or three of the genes selected from the group of: MCM6 (SEQ ID NO:28), POLR2H (SEQ ID NO:4) and ITGA2 (SEQ ID NO:34). Thus, a kit for assessing whether a subject has a CD8.1 or CD8.2 CD8 cell subtype may comprise primers suitable for determining the level of expression of: MCM6; POLR2H; ITGA2; MCM6 and POLR2H; MCM6 and ITGA2; POLR2H and ITGA2; or MCM6, POLR2H and ITGA2.

A kit for assessing whether a subject has a CD8.1 or CD8.2 CD8 cell subtype may comprise primers suitable for determining the level of expression of one or two genes selected from the group of: Jak1 (SEQ ID NO:67) and GZMK (SEQ ID NO:68). Thus, a kit for assessing whether a subject has a CD8.1 or CD8.2 CD8 cell subtype may comprise primers suitable for determining the level of expression of: Jak1; GZMK; or Jak1 and GZMK.

Table 1 includes the GenBank accession number for each of the genes listed. Designing primers suitable for determining the level of expression of a gene of interest is routine in the art, and the skilled person would therefore have no difficulty in designing further primers suitable for determining the level of expression of any one of the genes listed in Table 1 based on the sequence information provided in the GenBank database.

Similarly, a kit for assessing whether a subject has a CD4.1 or CD4.2 CD4 cell subtype may comprise primers suitable for determining the level of expression of a gene which is differentially expressed in CD4 cells of subtypes CD4.1 and CD4.2, using e.g. quantitative PCR. Genes which are differentially expressed in these subtypes include the genes listed in Table 2. Thus, in one example a kit for assessing whether a subject has a CD4.1 or CD4.2 CD4 cell subtype may comprise primers suitable for determining the level of expression of at least one of the genes listed in Table 2. The sequences of exemplary primer pairs suitable for determining the expression level of these genes are detailed in Table 2. In one example, a kit may comprise primers suitable for determining the level of expression of two or more, three or more, four or more, five or more, six or more, seven or more, eight or more, nine or more, or ten or more of the genes listed in Table 2. Preferably, the kit comprises primers for determining the level of expression of two or more of the genes listed in Table 2.

A kit for assessing whether a subject has a CD4.1 or CD4.2 CD4 cell subtype may comprise primers suitable for determining the level of expression of one, two, or three of the genes selected from the group of: ITGA2 (SEQ ID NO:34), PTPN22 (SEQ ID NO:37) and NOTCH1 (SEQ ID NO:40). Thus, a kit for assessing whether a subject has a CD4.1 or CD4.2 CD4 cell subtype may comprise primers suitable for determining the level of expression of: ITGA2; PTPN22; NOTCH1; ITGA2 and PTPN22; ITGA2 and NOTCH1; PTPN22 and NOTCH1; or ITGA2, PTPN22 and NOTCH1.

A kit for assessing whether a subject has a CD4.1 or CD4.2 CD4 cell subtype may comprise primers suitable for determining the level of expression of one, or three of the genes selected from the group of: PCAF (NM_003884; GI 8850), ANKRD32 (NM_032290; GI 84250), ZNF26 (NM_019591; GI 7574). Thus, a kit for assessing whether a subject has a CD4.1 or CD4.2 CD4 cell subtype may comprise primers suitable for determining the level of expression of: PCAF; ANKRD32; ZNF26; PCAF and ANKRD32; PCAF and ZNF26; ANKRD32 and ZNF26; or PCAF, ANKRD32 and ZNF26.

Table 2 includes the GenBank accession number for each of the genes listed. Designing primers suitable for determining the level of expression of a gene of interest is routine in the art, and the skilled person would therefore have no difficulty in designing further primers suitable for determining the level of expression of any one of the genes listed in Table 2 based on the sequence information provided in the GenBank database.

Kits for assessing whether a subject has a CD8.1 or CD8.2 CD8 cell subtype may comprise antibodies suitable for determining the level of expression of a gene which is differentially expressed in CD8 cells of subtypes CD8.1 and CD8.2, using e.g. ELISAs, western blotting, and/or flow cytometry. The antibodies may be monoclonal or polyclonal. Genes which are differentially expressed in subtypes CD8.1 and CD8.2 include the genes listed in Table 1. Thus, in one example a kit for assessing whether a subject has a CD8.1 or CD8.2 CD8 cell subtype may comprise antibodies suitable for determining the level of expression of one or more of the genes listed in Table 1 by detecting a protein encoded by said gene. In one example, a kit may comprise antibodies suitable for detecting the proteins encoded by two or more, three or more, four or more, five or more, six or more, seven or more, eight or more, nine or more, ten or more, or eleven of the genes listed in Table 1. Preferably, the kit comprises antibodies for detecting the proteins encoded by two or more of the genes listed in Table 1.

A kit for assessing whether a subject has a CD8.1 or CD8.2 CD8 cell subtype may comprise antibodies suitable for determining the level of expression of one, two, or three of the genes selected from the group of: ITGA2 (SEQ ID NO:34), PTPN22 (SEQ ID NO:37) and NOTCH1 (SEQ ID NO:40). Thus, a kit for assessing whether a subject has a CD8.1 or CD8.2 CD8 cell subtype may comprise antibodies suitable for determining the level of expression of: ITGA2; PTPN22; NOTCH1; ITGA2 and PTPN22; ITGA2 and NOTCH1; PTPN22 and NOTCH1; or ITGA2, PTPN22 and NOTCH1.

A kit for assessing whether a subject has a CD8.1 or CD8.2 CD8 cell subtype may comprise antibodies suitable for determining the level of expression of one, two, or three of the genes selected from the group of: MCM6 (SEQ ID NO:28), POLR2H (SEQ ID NO:4) and IL7R (SEQ ID NO:22). Thus, a kit for assessing whether a subject has a CD8.1 or CD8.2 CD8 cell subtype may comprise antibodies suitable for determining the level of expression of: MCM6; POLR2H; IL7R; MCM6 and POLR2H; MCM6 and IL7R; POLR2H and IL7R; or MCM6, POLR2H and IL7R.

A kit for assessing whether a subject has a CD8.1 or CD8.2 CD8 cell subtype may comprise antibodies suitable for determining the level of expression of one, two, or three of the genes selected from the group of: MCM6 (SEQ ID NO:28), POLR2H (SEQ ID NO:4) and ITGA2 (SEQ ID NO:34). Thus, a kit for assessing whether a subject has a CD8.1 or CD8.2 CD8 cell subtype may comprise antibodies suitable for determining the level of expression of: MCM6; POLR2H; ITGA2; MCM6 and POLR2H; MCM6 and ITGA2; POLR2H and ITGA2; or MCM6, POLR2H and ITGA2.

A kit for assessing whether a subject has a CD8:1 or CD8.2 CD8 cell subtype may comprise antibodies suitable for determining the level of expression of one or two genes selected from the group of: Jak1 (SEQ ID NO:67) and GZMK (SEQ ID NO:68). Thus, a kit for assessing whether a subject has a CD8.1 or CD8.2 CD8 cell subtype may comprise antibodies suitable for determining the level of expression of: Jak1; GZMK; or Jak1 and GZMK.

If not already commercially available, the skilled person could isolate antibodies suitable for detecting a protein encoded by a gene listed in Table 1 using one of the well established methods for making polyclonal or monoclonal antibodies. For example, polyclonal antibodies to a protein of interest can be made by cloning the gene encoding said protein into a suitable vector, introducing the vector into a host cell, culturing the host cells under conditions suitable for expression of said protein, isolating the expressed protein, immunizing an animal (e.g. a rabbit) with the isolated protein, and isolating the antibody from said animal. The sequences of the genes listed in Table 1 are available from the GenBank database under the relevant accession number.

Similarly, kits for assessing whether a subject has a CD4.1 or CD4.2 CD4 cell subtype may comprise antibodies suitable for determining the level of expression of a gene which is differentially expressed in CD4 cells of subtypes CD4.1 and CD4.2, using e.g. ELISAs, western blotting, and/or flow cytometry. The antibodies may be monoclonal or polyclonal. Genes which are differentially expressed in subtypes CD4.1 and CD4.2 include the genes listed in Table 2. Thus, in one example a kit for assessing whether a subject has a CD4.1 or CD4.2 CD4 cell subtype may comprise antibodies suitable for determining the level of expression of one or more of the genes listed in Table 2 by detecting a protein encoded by said gene. In one example, a kit may comprise antibodies suitable for detecting the proteins encoded by two or more, three or more, four or more, five or more, six or more, seven or more, eight or more, nine or more, ten or more, or eleven of the genes listed in Table 2. Alternatively, a kit may comprise antibodies suitable for detecting the proteins encoded by at least two, at least three, at least four, at least five, at least six, at least seven, at least eight, at least nine, or at least ten of the genes listed in Table 2. Preferably, the kit comprises antibodies for detecting the proteins encoded by at least two of the genes listed in Table 2.

A kit for assessing whether a subject has a CD4.1 or CD4.2 CD4 cell subtype may comprise antibodies suitable for determining the level of expression of one, two, or three of the genes selected from the group of: ITGA2 (SEQ ID NO:34), PTPN22 (SEQ ID NO:37) and NOTCH1 (SEQ ID NO:40). Thus, a kit for assessing whether a subject has a CD4.1 or CD4.2 CD4 cell subtype may comprise antibodies suitable for determining the level of expression of: ITGA2; PTPN22; NOTCH1; ITGA2 and PTPN22; ITGA2 and NOTCH1; PTPN22 and NOTCH1; or ITGA2, PTPN22 and NOTCH1.

A kit for assessing whether a subject has a CD4.1 or CD4.2 CD4 cell subtype may comprise antibodies suitable for determining the level of expression of one, or three of the genes selected from the group of: PCAF (NM_003884; GI 8850), ANKRD32 (NM_032290; GI 84250), ZNF26 (NM_019591; GI 7574). Thus, a kit for assessing whether a subject has a CD4.1 or CD4.2 CD4 cell subtype may comprise antibodies suitable for determining the level of expression of: PCAF; ANKRD32; ZNF26; PCAF and ANKRD32; PCAF and ZNF26; ANKRD32 and ZNF26; or PCAF, ANKRD32 and ZNF26.

If not already commercially available, the skilled person could isolate antibodies suitable for detecting a protein encoded by a gene listed in Table 2 using one of the well established methods for making polyclonal or monoclonal antibodies. The sequences of the genes listed in Table 2 are available from the GenBank database under the relevant accession number.

A kit for assessing whether a subject has a CD8.1 or CD8.2 CD8 cell subtype may further comprise components suitable for isolating CD8 cells from a blood sample obtained from a subject. For example, the components may comprise components for isolating CD8 cells from a blood sample by centrifugation, e.g. over ficoll, and/or magnetic beads for positively selecting CD8 cells.

Similarly, a kit for assessing whether a subject has a CD4.1 or CD4.2 CD4 cell subtype may further comprise components suitable for isolating CD4 cells from a blood sample obtained from a subject. The components may comprise components for isolating CD4 cells from a blood sample by centrifugation, e.g. over ficoll, and/or magnetic beads for positively selecting CD4 cells.

The kits may further comprise instructions for use of the kits for determining whether a subject has a CD8.1 or CD8.2 cell subtype and/or a CD4.1 or CD4.2 cell subtype, as applicable.

Various further aspects and embodiments of the present invention will be apparent to those skilled in the art in view of the present disclosure. "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Certain aspects and embodiments of the invention will now be illustrated by way of example and with reference to the figures and tables.

Attempts to use microarrays to predict prognosis in autoimmunity have been less successful than in oncology, and none have yet proven useful for guiding therapy. Disease-related signatures have been identified, some of which have a degree of association with disease activity, such as the interferon-α signature found in peripheral blood mononuclear cells (PBMCs) in SLE (Bennett, 2003; Baechler, 2003), or that which defined the importance of IL1 in the pathogenesis of Still's disease (Allantaz, 2007). This failure of microarray analysis to predict prognosis in autoimmunity could be due to the fact that many signatures obtained through the analysis of PBMC reflect changes in the relative size of white cell subsets rather than transcriptional patterns within cells of the subsets themselves (Bennett, 2003; Batliwalla, 2005; Popper, 2007). We have found that microarray analysis of purified white blood cell subsets vastly increases the sensitivity of the technique, allowing approximately 90% more disease-associated differentially expressed genes to be identified than would be possible from PBMC alone (Lyons, 2009). We therefore used this technique in AAV to determine its potential clinical efficacy.

### Example 1 - ANCA-associated vasculitis (AAV)

AAV is a chronic and often severe autoimmune disease, which is divided into three clinical syndromes (Wegener's granulomatosis [WG], microscopic polyangitis [MPA] and Churg-Strauss syndrome) (Lane, 2005). All three are characterised by inflammation of medium and small vessels (small arteries, arterioles, capillaries and venules), anti-neutrophil cytoplasmic antibodies (ANCA) and a prominent CD8 and CD4 T cell infiltrate. ANCA are directed against neutrophil cytoplasmic antigens (anti-proteinase-3 [PR-3] is associated with WG, and anti-myeloperoxidase [MPO] with MPA), and are likely to contribute to disease pathogenesis. The syndromes have diverse and variable clinical features, including acute glomerulonephritis, granulomatous inflammation of the upper and lower respiratory tract (especially WG), neurological vasculitis and more. Mortality at five years is as high a 30%, and most of this is due to the infectious side effects of immunosuppressive therapy (Booth, 2003).

Forty-four patients with a clinical diagnosis of AAV and attending the Vasculitis and Lupus service in Cambridge were recruited. Patients were enrolled if they had active disease as defined by a Birmingham vasculitis activity score (BVAS) (Stone, 2001) and who had had no previous evidence of disease or had quiescent disease on minimal maintenance therapy. All patients had a clinical diagnosis of either WG or MPA and conformed to current classification criteria (Watts *et al.,* 2000). Patients were then treated with induction therapy, comprising high dose steroid with either cyclophosphamide or rituximab, followed by maintenance therapy with lower dose prednisolone and azathioprine or mycophenolate mofetil. Alternative therapies in refractory cases were adalimumab and DSG. Detailed clinical and laboratory information was collected prospectively on each patient. Initially 32 patients were analysed together (the "initial cohort"), and the subsequent 12 patients enrolled analysed separately as the "validation cohort".

On presentation blood was taken and CD4 and CD8 T cells, B cells, neutrophils and monocytes purified as previously described (Lyons *et al.,* 2007). RNA was extracted from both PBMC and individual cell subsets, amplified and labelled (Petalidis *et al*., 2003), and hybridised in duplicate using a dye-swap strategy against a common reference RNA to a pan-genomic, spotted oligonucleotide array (Le Brigand *et al.,* 2006).

Analysis of PBMC transcription in AAV patients showed no clear substructure, in accordance with the limitations of array studies using unpurified cells (Lyons *et al.,* 2009). We then analysed the CD4 and CD8 T cell subset.

Surprisingly, when arrays from the purified CD4 and CD8 T cells were analysed by principal component analysis, clear statistically significant "substructure" was seen, greater than would be expected in a univariate dataset (methods). Subsequent unsupervised hierarchical clustering of the transcriptome of purified CD4 cells from the initial cohort was performed using multiple probe lists (methods) and all demonstrated that patients fell into two discrete groups. To confirm separation into two robust subgroups, two independent clustering techniques (hierarchical and k-means clustering) were employed in a comparative algorithm (methods), giving a consensus subgroup for each patient. The reliability of this designation was further assessed by deriving a robustness coefficient, R (methods).

A consensus clustering algorithm (Genepattem; Reich *et al*., 2006) employed on the full CD8 transcriptome dataset determined that the existence of discrete patient subgroups explained this substructure, with subsampling across multiple runs of hierarchical clustering providing a robust measure of confidence that the optimal number of patient subgroups was 2 (Monti, 2003) (Fig. 3). A further comparative algorithm (Kapushesky *et al.,* 2004) was used to compare patient subgroups generated by 2 independent clustering techniques (k-means and hierarchical). In all cases 2 subgroups, with the same patients in each group, were found when consensus identities using multiple runs of 1 technique were compared to consensus identities derived from integration of 2 techniques. Having defined two subsets, termed v8.1 (vasculitis subtype 8.1) and v8.2 (vasculitis subtype 8.2), within the original dataset, the genes defining them were identified using ANOVA (restricting to a minimum fold-change of 2 and controlling the false discovery rate to 0.05). Hierarchical clustering using this differential list clearly demonstrates the substructure found. The same methods were also used to analyze the CD4 transcriptome dataset, again showing that the optimal number of patient subgroups was 2. Having defined two subsets, termed v4.1 (vasculitis subtype 4.1) and v4.2 (vasculitis subtype 4.2), within the original dataset, the genes defining the subsets were again identified using ANOVA.

The gene list that best differentiated the CD8 groups was then used to cluster the validation cohort, again identifying two discrete groups. The gene list in this case included 925 genes. When the validation cohort was then analysed *de novo* using unsupervised hierarchical clustering as above, an identical division of patients was observed. The gene list that best differentiated these groups in the validation cohort was then used to cluster the initial cohort, and identical patient subdivisions were observed to the *de novo* analysis. An identical analysis of patients using RNA from purified CD4 T cells also defined two subgroups of slightly different size, and confirmation of this by analysis of the validation cohort showed it to be robust. The gene list that best differentiated the CD8 groups included 384 genes. The gene lists that best differentiate the CD4 and CD8 subgroups represent genes that show >2 fold statistically significant (P<0.05, FDR,0.05) differential expression between consensus subgroups defined by unsupervised clustering.

A number of genes which differentiated the groups were validated by quantitative PCR (CD69 and IL7R in CD8 and CD4 subgroups, and CD40 and CD25 in CD4 subgroups only). Having found that a similar transcription signature defined discrete patient subgroups in independent patient cohorts, the cohorts were merged for subsequent analyses.

An attempt was made to correlate the patient groups identified by both CD8 and CD4 transcriptional analysis with disease parameters. Two striking associations were found - the first was that more patients in groups 8.1 and 4.1 relapsed after initial remission, as demonstrated by analysis of relapse-free survival (Figs. 4 and 5), and the second was that these two groups had more relapses (disease flares) per month of follow-up than 8.2 and 4.2 (Fig. 6). For example, 89% of group 8.1 had more than one relapse, compared to 6.3% of 8.2. Thus these hypothesis-free, unsupervised analyses allow prospective prediction of patients with relapsing disease, with the CD8 T cell signature being slightly superior to CD4 in this regard. The best current predictor of prognosis in AAV is the presence of anti-PR3 rather than anti-MPO antibody (Weidner *et al.,* 2004), though this is not accurate enough to be clinically useful in determining therapy (Langford, 2004). In our cohorts no significant association between ANCA and outcome was seen (p=0.13, compared to p = 0.0004 differentiating the array-based groups; Figs. 1 and 2).

There was no correlation with other parameters, notably including disease activity score at enrolment (time 0), conventional markers of inflammation such as erythrocyte sedimentation rate (ESR) or C-reactive protein (CRP), or rate of remission in response to induction therapy. There were trends for association of the 8.1 and 4.1 groups with a higher incidence of pre-existing disease, worsening serum creatinine, Wegener's granulomatosis, and a younger age-all parameters that might be expected to be worse in a subgroup with subsequent relapsing disease, but none associated strongly enough to survive correction for multiple testing. More patients in 8.1 and 4.1 were on base-line steroid treatment at enrolment, though this too did not survive Bonferroni correction. This is likely to reflect the fact that these groups have relapsing disease and thus are more likely to be enrolled at flare (when on maintenance therapy) than at diagnosis. Steroid treatment itself, however, does not explain existence of the transcriptionally-defined subsets: evidence for this includes the fact that steroid therapy was given to a number of patients in either group and thus could not account for the division, that the transcriptional changes induced in T cells by corticosteroids have been well defined and are not enriched in the subset-defining signatures when subject to gene set enrichment analysis (GSEA), and that the use of steroids would be expected to be associated with reduced, and not increased, disease.

We then sought to determine if these transcription signatures were associated with biological changes that might explain their ability to predict relapsing disease. GSEA demonstrated an enrichment of genes associated with signalling through the common γ chain in group 4.1, but not 8.1. This association in CD4 subsets was reflected in a concerted up-regulation of genes subserving signalling by IL7 and IL2, but not IL4 (Fig. 8). In the CD8 subsets consistent overexpression was only seen in the IL7 pathway, as IL2R is expressed only at low levels in this population (Fig. 7). Signalling through the IL7Rα (CD127) is vital for promoting T cell survival, likely to be mediated by bcl-2 family-mediated inhibition of the pro-apoptotic effects of Bim (Pellegrini *et al*., 2004), and is critical for the expansion of T cell subpopulations driving effector and memory populations of CD8, and to a lesser extent CD4, T cells (Li *et al.,* 2003; Kondrack *et al.,* 2003; Seddon *et al*., 2003; Kaech *et al.,* 2003;Hand *et al*., 2007). It is therefore of additional interest that bcl-2 is consistently upregulated in both group 8.1 and 4.1. IL-2 signalling also plays a role in the differentiation of effector T cells, though not necessarily in the maintenance of CD8 memory (Carr *et al*., 2006), and in particular of regulatory T cells. CD27 and CD40L were also upregulated in 4.1 and 8.1. Together these observations suggest T cells are more prone to activation and expansion in the relapsing groups, which could in part be due to expansion of a memory or pre-effector population.

AAV has been associated with expanded T_{CM} (central memory) and T_{EM} (effector memory) populations (Abdulahad *et al.,* 2006) and a skewed T_{H}17 subset (Abdulahad *et al*., 2008), but not consistently with abnormalities in peripheral T_{H}1, T_{H}2 or Treg populations (Lamprecht, 2005). GSEA analysis using publically available array data demonstrated strong enrichment of a human T_{EM} expression signature in subgroup 8.1 and a T_{CM} signature in subgroup 4.1. When patients were subjected to hierarchical clustering using the human T_{EM} expression signature groups v8.1 and v8.2 were regenerated precisely. No enrichment was seen in either CD4 subgroup of signatures generated from activated T, T_{H}1, T_{H}2, human CD4+CD25+ or mouse regulatory T cells. CD8 subgroups were not enriched with an activated CD8 cell signature, though there was a trend for association of group 8.1 with a mouse CD8 T_{EM} signature, consistent with the recent description of a T cell memory array signature common to different species (Haining *et al.,* 2008). A T_{H}17 signature curated from known differentiation markers (Ivanov *et al.,* 2007, Chen *et al.,* 2007) showed equivalent expression in both subgroups in CD4 and CD8 sets. A NK cell signature curated from an online repository showed equivalent expression in the 8.1 and 8.2 subgroups.

To determine if these array correlations suggesting increased T_{EM} were reflected in cell populations, a cross-section of 30 of the same patients on maintenance therapy between 11 and 42 months after enrolment was phenotyped. While the relationship between these results and the situation that existed at enrolment must be somewhat speculative, a clear difference in CD8 T cell populations was seen. Patients from 8.1 had an increased number of CD8 T cells in general, but of T_{M} cells in particular, defined as CD3⁺CD8⁺CD45RA⁻. Similar changes were not seen in CD4 cells at these delayed time points although they may be present at earlier time points. These cells were increased in both proportion and absolute number in group v8.1. Most of these cells were CCR7 low, consistent with a T_{EM} (or perhaps T memory precursor effector cell T_{MPEC}) phenotype. The "central memory" cells described by Lanzavecchia were not always seen as a discrete population, but if defined by gating on the CCR7* component of the T_{EM} population they were also significantly increased in both number and proportion. The gene list which defines group v8.1 could not, however, be explained solely by the increased T_{EM} population seen, as Bcl-2, for example, was up-regulated in most subpopulations, but particularly in naive T cells.

Thus the genes which define group v8.1 provide a plausible mechanism for the increase in disease flares seen in this group. They have an increase in T_{EM}-T_{MPEC} cells, which have the capacity both to persist as a memory population and, once stimulated, to rapidly expand and differentiate into effector cells driving inflammatory disease. In addition, a more global increase in genes such as bcl-2 in T cells would be expected to amplify autoreactive T cell responses in general (Strasser *et al.,* 1991). In CD4 cells an increase in T_{M} was not seen at late times (but may have been present at enrolment), but transcriptional changes suggest an increased capacity for both T expansion and T cell help. The latter could assist both CD8 T cells and B cells drive disease.

### Example 2 - Systemic Lupus Erythematosus (SLE)

To determine if the signature which correlates with prognosis in AAV is specific to that disease or is seen in other autoimmune diseases, we arrayed purified CD8 T cells from a cohort of patients with SLE, enrolled in parallel to those with AAV.

26 patients meeting the American Rheumatological Association definition of SLE were recruited. Patients had active disease on enrolment, as defined by the British Isles Lupus Activity Grade (BILAG) disease activity assessment, and they had had no previous evidence of disease or had quiescent disease on minimal maintenance therapy. All patients were then treated with high dose steroid and one of a number of induction therapies - all responding as evidenced by a fall in BILAG to 0 by 3 months. Maintenance therapy comprised lower dose prednisolone and azathioprine or mycophenolate mofetil. Samples were processed and data analysed in an identical fashion to that described for AAV above, though as a single cohort. Multidimensional scaling (BRB; hftp:/linus.nci.nih.gov/BRB-ArrayTools.html) with a global test of clustering (McShane *et al.,* 2002) showed evidence of substructure. Application of a consensus clustering algorithm and subsequent comparative algorithm again showed this substructure was best explained by the presence of 2 discrete patient subgroups (called s8.1 and s8.2). The genes defining the subgroups were identified using ANOVA (restricting to a minimum fold-change of 2 and controlling the false discovery rate to 0.05), and hierarchical clustering using this gene list clearly demonstrates the two groups. The gene list used for hierarchical clustering in this case comprised 1,913 genes. The gene list best defining the SLE subgroups was then used to cluster the AAV patients - this recreated the AAV groups v8.1 and v8.2. The converse analysis recreated the SLE groups, indicating a striking similarity in the CD8 T cell transcriptional changes defining the patient subgroups in these two distinct autoimmune diseases.

Correlation between these SLE subgroups and disease features and course was then sought. As for AAV, there was a striking correlation between SLE group s8.1 and relapsing disease. All SLE group 8.1 patients had flared by 500 days after enrolment, compared to 15% of 8.2 patients (Fig. 9). Again group s8.1 patients had more disease flares per month of follow-up than 8.2, and all patients with more than one flare were found in s8.1 (Fig. 10). Patients in subgroup S8.1 also experienced significantly more flares than patients in subgroup S8.2 when followed out to 1,000 days post induction therapy. The best described predictor of prognosis in SLE is the titre of anti-double stranded DNA antibodies (Bootsma *et al.,* 1997), though this is not adequate to determine therapy. In our cohort no significant association between anti-double stranded DNA antibodies and outcome was seen, and there was no correlation between s8.1 and s8.2 and other disease parameters

### Example 3 -Normal subjects

As a CD8 transcription signature divided two distinct autoimmune disease cohorts into 2 prognostically useful subgroups, we wondered whether such groups existed in the normal population. We therefore analysed a cohort of 22 normal controls in identical fashion to that described for the AAV and SLE patients described above. Interestingly, unsupervised analysis showed that these controls fell into two groups, c8.1 (control subtype 8.1) and c8.2 (control subtype 8.2), in broadly similar proportions to the AAV and SLE patients described above.

The gene list that defined c8.1 and c8.2 was used to cluster both SLE and AAV cohorts, and all patients were placed into the same subgroups as they had been when clustering was performed using gene lists generated from the disease groups themselves. To further confirm the similarity in transcription signatures defining the 8.1 and 8.2 subgroups, all CD8 T cell data (AAV, SLE and Control) was pooled and unsupervised hierarchical clustering performed. Two groups were again generated, divided not by diagnosis, but by the 8.1 and 8.2 subgroups described above. Thus, despite the fact that the c8.1 and c8.2 subgroups were identified in an unsupervised fashion, the gene signature defining them was strikingly similar to that which defined the subsets in AAV and SLE patients, and the gene lists identifying them could be used interchangeably to generate the same subgroups from both patients and controls. Thus, the same CD8 T cell-defined subgroup that predicts relapsing AAV and SLE is also present in the population as a whole. This raises the possibility that they may also predict prognosis in other autoimmune diseases, and may have wider implications, for example, in controlling responses to infection, vaccination and transplantation.

### Example 4 - Development of a three gene classification model

In order to facilitate the translation of these prognostic expression signatures to clinical practice a predictive model using expression data from a smaller number of genes was developed as follows. The AAV cohort was split into 2 (50:50 split preserving the relative proportions of each subgroup) to form 'training' and 'test' sets. A support vector machines algorithm (Reich *et al.,* 2006) was then used to derive a classifier on microarray data using 3 genes in the training set. The algorithm was then applied to the test set to assess performance. Three genes, *ITGA2, PTPN22* and *NOTCH1,* were selected on the basis of (i) a favourable signal-to-noise ratio of expression between subgroups 8.1 and 8.2 (ii) known immunological significance, particularly in CD8 T cell memory and (iii) reaching a significance threshold of a false discovery rate p < 0.05. No fold-change restriction was applied. The choice of 3 genes (rather than 1, 2, 4 etc) was determined empirically by increasing the number of probes fulfilling criteria (i) and (ii) until no further improvement in accuracy of classification was seen.

Measuring expression of the three genes, *ITGA2, PTPN22, NOTCH* by microarray analysis or by quantitative PCR platform allowed robust discrimination of subgroups 8.1 and 8.2 in the AAV cohort (positive predictive value (PPV) 100%, negative predictive value (NPV) 100%, Fig. 11A and B). Interestingly, the three genes in the optimal predictive model (*ITGA2, PTPN22, NOTCH1*) have all been associated with the development of T cell memory responses (Rieck *et al.,* 2007; Okamoto *et al*., 2008; and Kassiotis *et al*., 2006) and with autoimmunity (Charbonnier *et al*., 2006; Smyth *et al*., 2008; and Matesic *et al*., 2006).

As the gene expression signatures defining subgroups 8.1 and 8.2 in AAV and SLE cohorts were highly overlapping, it was tested whether the use of a single predictive model would allow prediction of prognosis in both. Use of the three gene predictive model (*ITGA2, PTPN22, NOTCH1*) developed on the AAV dataset could also robustly determine subgroup identities in SLE (PPV 100%, NPV 100%; Fig. 12A and B). Indeed, when applied to the CD8 expression dataset as a whole (incorporating both SLE and AAV) the three gene predictive model clearly separated subgroup 8.1 from 8.2 (Fig. 13A and B), but showed no discrimination between the two disease phenotypes (Fig. 13C). Furthermore, when unsupervised hierarchical clustering was performed on CD8 expression data for all genes across both diseases the data separated into the prognostically-relevant subgroups rather than by disease. In this particular case, the accuracy of prediction depended on the use of RNA from purified CD8 T cells and could not be replicated using RNA from unseparated PBMC. To further confirm the similarity in transcription signatures defining the 8.1 and 8.2 subgroups, the three gene predictive model derived from the AAV dataset was applied directly to the control dataset, again robustly predicting subgroup identity (PPV 100%, NPV 100%, Fig. 14A and B).

In addition, the three gene predictive model could also be used to determine subgroup identity when applied to the AAV CD4 dataset (for predicting 4.1: PPV 100%, NPV 85%) (Fig. 15).

### Example 5 - Inflammatory Bowel Disease (IBD)

To determine if the signature which correlates with prognosis in AAV and SLE patients is also found IBD patients, 26 patients with IBD (Crohn's disease) were recruited. CD8 T cell samples were taken from these patients and RNA extracted. The RNA was then amplified, labelled and hybridised to the Affymetrix human gene ST 1.0 microarray platform.

Unsupervised clustering of the array data showed that the patients again fell into two discrete patient groups, 8.1 and 8.2. The genes list that defined subgroups 8.1 and 8.2 in AAV patients were also found to be significantly enriched in the gene lists that define these subgroups in IBD patients, as determined using Gene Set Enrichment Analysis. Thus, the same CD8 T cell-defined subgroup that is associated with an increased likelihood of flares or relapses in AAV and SLE patients is also present in IBD patients.

Next it was tested whether there is any correlation between these subgroups and disease progression.

IBD is slightly different to AAV and SLE in that not all patients achieve clinical remission after initial therapy. As a result these patients subsequently require increased immunosuppression or surgery in order to achieve remission. This difference is due to the fact that AAV and SLE patients receive intensive immunosuppression at first presentation of the disease, which aims to ensure remission at the risk of side-effects. In contrast, IBD patients mostly receive a less intensive immunosuppressive therapy at first presentation of the disease. For most IBD patients this is sufficient to achieve remission of the disease without the need for maintenance therapy, although not for all.

For IBD, disease progression was therefore defined as an event requiring increased therapy in the form of either increased immunosuppression or surgery. As already mentioned above, such events included flares (or relapses) of the disease after a period of remission, as well as cases where the disease did not enter remission in response the initial therapy and increased immunosuppression or surgery was required as a result.

The results showed that, similarly to AAV and SLE, IBD patients in subgroup 8.1 were more likely to experience an event requiring increased therapy than patients in subgroup 8.2 (Fig. 16). Patients were followed for up 400 days and by 250 days after enrolment approximately 65% of patients in subgroup 8.1 had experienced an event requiring increased therapy while the figure for patients in subgroup 8.2 was only around 30% (Fig. 16).

That the difference in disease progression between subgroups 8.1 and 8.2 was statistically significant was confirmed using a Log-rank test (p=0.019). IBD patients in subgroup 8.1 are therefore significantly more likely to experience an event requiring increased therapy than patients in subgroup 8.2.

The inventors then tested whether IBD patients could be classified as subgroup 8.1 or 8.2 by measuring the expression of a limited number of genes. The genes selected from the genes listed in Table 1 for this purpose were MCM6, POLR2H and IL7R.

The results show that by measuring the expression of genes MCM6, POLR2H and IL7R it was possible to classify IBD patients as subgroup 8.1 or 8.2 (positive predictive value [PPV] 86%, negative predictive value [NPV] 94%) (Figure 18A and B). Gene expression was measured by analysing the microarray expression data from the Affymetrix platform. The confidence threshold applied in this case was 25% (indicated by solid black lines in Figure 18B) and one patient sample remained unclassified.

### Examale 6 - Transplant Patients

To determine whether the signature which correlates with prognosis in AAV, SLE and IBD patients is also found in transplant patients, 26 renal transplant patients were recruited and blood was taken for expression profiling immediately prior to transplantation. CD8 T cells were isolated from these blood samples and RNA extracted. The RNA was then amplified, labelled and hybridised to the Affymetrix human gene ST 1.0 microarray.

Unsupervised clustering of the array data showed that the patients again fell into two discrete patient groups, 8.1 and 8.2. The genes list that defined subgroups 8.1 and 8.2 in AAV patients were also found to be significantly enriched in the gene lists that define these subgroups in renal transplant patients, as determined using Gene Set Enrichment Analysis. Thus, the same CD8 T cell-defined subgroup that predicts relapsing AAV and SLE is also present in IBD patients.

Analysis of the array showed that the patients again fell into two discrete patient groups, 8.1 and 8.2. The genes list that defined subgroups 8.1 and 8.2 in renal transplant patients showed a high degree of overlap with the gene lists that define these subgroups in AAV, SLE, and IBD patients and healthy controls. Thus, the same CD8 T cell-defined subgroup that is associated with an increased likelihood of flares or relapses in AAV and SLE patients is also present in transplant patients.

Next it was determined whether there is any correlation between these subgroups and acute transplant rejection. In line with the results obtained for AAV, SLE and IBD, patients in subgroup 8.1 were more likely to experience acute transplant rejection than patients in subgroup 8.2 (Fig. 17). Specifically, by 200 days after transplantation, about 60% of patients in subgroup 8.1 had experienced acute rejection, while the figure for patients in subgroup 8.2 was only about 10% (Fig. 17). That the difference in transplant rejection between subgroups 8.1 and 8.2 was statistically significant was confirmed using a Log-rank test (p=0.012). Patients in subgroup 8.1 are therefore significantly more likely to experience acute transplant rejection than patients in subgroup 8.2.

As for IBD, the inventors the tested whether transplant patients could be classified as subgroup 8.1 or 8.2 by measuring the expression of a limited number of genes. The genes selected from the genes listed in Table 1 for this purpose were MCM6, POLR2H and ITGA2.

The results show that by measuring the expression of genes MCM6, POLR2H and ITGA2 it was possible to classify IBD patients as subgroup 8.1 or 8.2 (positive predictive value [PPV] 89%, negative predictive value [NPV] 94%) (Figure 19A and B.). Gene expression was measured using the Affymetrix human gene ST 1.0 microarray platform. The confidence threshold applied in this case was 20% (indicated by solid black lines in Figure 19B) and one patient sample remained unclassified.

### Example 7 - Subgroup) identification based on gene expression in unseparated PBMCs

The PBMC samples used in this example were taken from the AAV patients concurrently with the samples used to define subgroups 4.1 and 4.2, i.e. the PBMC sample was taken and the CD4 cells subsequently extracted from that same PBMC sample to generate the AAV CD4 datasets described.

The genes used in this example (PCAF, ANKRD32, ZNF26) were chosen by ranking the PBMC dataset for differential expression (signal-to-noise metric) between the known CD4 subgroups. PCAF, ANKRD32 and ZNF26 were all upregulated in subgroup 4.1 compared to subgroup 4.2. The relevant details for these genes are as follows:

| **HUGO symbol** | **GenBank Accession no.** | **GI no.** |
|---|---|---|
| ZNF26 | NM_019591 | GI 7574 |
| ANKRD32 | NM_032290 | GI 84250 |
| PCAF | NM_003884 | GI 8850 |

By measuring the expression of these genes in the PBMC samples, AAV patients could be classified as subgroups 4.1 and 4.2 (positive predictive value: 100%; negative predictive value: 71%) (Fig. 20). The confidence threshold applied in this case was 25% and one sample remained unclassified.

These results demonstrate it is possible to determine an individuals' subgroup identity by measuring the expression of one or more genes known to be differentially expressed in the relevant subgroup in a sample of e.g. unseparated PBMCs obtained from the individual in question.

### Conclusion

Transcriptional signatures can thus define a subset of AAV patients who, while responding equally well to induction therapy, are far more likely to relapse despite equivalent maintenance therapy. Thus, AAV patient subgroups defined by gene expression predominantly in IL7R and IL2R pathways in CD4 and CD8 T cells predict subsequent prognosis. Similarly, SLE patient subgroups, defined by gene expression particularly in the IL7R pathway and memory T cell population in CD8 T cells, also predict subsequent prognosis.

Similarly, it was found that IBD patients can be divided into two distinct groups on the basis of their CD8 T cell gene expression profiles and these groups correlate with the groups identified in AAV and SLE patients. Again subgroup identity was prognostic of disease progression.

Depending on whether the patient groups were defined on the basis of their CD8 or CD4 gene expression profiles, the subgroups are referred to as 8.1 and 8.2 or 4.1 and 4.2, respectively. In all three patient groups (AAV, SLE and IBD) patients in groups 8.1 and 4.1 (i.e. patients having a 8.1 or 4.1 CD8 or CD4 T cell subtype, respectively) were more likely to experience disease progression than patients in group 8.2.

Patients in groups 8.1 or 4.1 are therefore likely to benefit from intensified maintenance therapy (e.g. intermittent rituximab treatment rather than azathioprine) and from more frequent clinical follow up. In contrast, the two thirds of patients that fall into 8.2 or 4.2 are likely to need less (or no) maintenance therapy, resulting in a reduction in immune suppression-associated toxicity.

The subgroups may also identify patient groups with differential responses to novel therapies, defining their optimal place in therapy. In addition to providing a prognostic biomarker for guiding therapy, these findings focus on the IL-7 receptor pathway as a potential therapeutic one in AAV and SLE.

The discovery of this signature in the normal population suggests that it may identify important prognostic subsets of patients with other immune or infectious conditions, and could help guide therapy in a range of clinical situations, such as vaccination, infection and transplantation. This was confirmed by experiments showing that renal transplant patients also fell into two distinct groups on the basis of their CD8 T cell gene expression profiles. These groups again correlated with the groups identified in AAV, SLE and IBD patients. As expected, patients in group 8.1 (i.e. patients having a 8.1 or CD8 T cell subtype) were more likely to experience acute transplant rejection than patients in group 8.2.

Thus, transplant patients in group 8.1 are also likely to benefit from more intense immunosuppressive therapy and from more frequent clinical follow up. In contrast, transplant patients in group 8.2 are likely to benefit from reduced immunosuppressive therapy, resulting in a reduction in immune suppression-associated toxicity.

### Example 8 - qPCR classification

The primers used in this example were selected from the primers used to validate the platform rather than being chosen specifically for their ability to distinguish between subgroups. Enhanced performance of prediction may therefore be possible with primers, or combinations of primers, selected based on their ability to distinguish between the CD8 and/or CD4 subgroups. Primers suitable for determining the expression level of the genes listed in Tables 1 and 2 may be especially suitable for this purpose, and the sequences of exemplary primers are detailed in these tables.

### qPCR Technique

mRNA levels of Jak1 and Gzmk were determined using Taqman Gene Expression Assays (Applied Biosystems) on an ABI PRISM 7900HT instrument according to the manufacturer's instructions. Transcript abundance was calculated by comparison to a standard curve.

### Classifier

The percent confidence in subgroup prediction (y-axis) was plotted against the individual patients (x-axis) for the vasculitis cohort used in the manuscript (both initial and validation cohorts combined, n=44). The classifier was developed using independent training and test sets with a 50:50 split, preserving the relative proportions of each subgroup in both sets.

A weighted voting algorithm (Golub *et al.,* 1999; Reich *et al.,* 2006) was applied to a qPCR dataset for 2 genes, Jak1 (NM_002227; SEQ ID NO: 67) and GZMK (NM_002104; SEQ ID NO: 68), chosen in this case for example purposes from the dataset available from array validation. Both Jak1 and GZMK were upregulated in subgroup 8.1 compared to subgroup 8.2. The results showed that with a confidence threshold set to 30%, subgroups v8.1 and v8.2 could be distinguished with 100% sensitivity and specificity and with only two uncertain calls (confidence < 30%), i.e. all samples in which a confident call was made were correctly assigned.

### Materials and Methods

### Patients

Forty-four patients attending or referred to the specialist vasculitis unit at Addenbrooke's hospital, Cambridge, UK between July 2004 and May 2007 together with 23 three age, sex and ethnically-matched controls were enrolled into the AAV study (for entry and exclusion criteria see Table 3 below). Thirty two patients presenting with a disease flare between July 2004 and Oct 2006 composed a prospectively-defined initial cohort, while a further 12 patients presenting between November 2006 and May 2007 composed a validation cohort, defined arbitrarily by date of presentation only. Following treatment with an immunosuppressant and tapering dose steroid therapy, patients were followed up monthly for up to 52 months. Of the forty-four vasculitis patients who were enrolled into the study, two were excluded from informatic analysis through quality control (unsatisfactory dye-swap correlation) and three were excluded from follow-up analysis due to failure to meet inclusion criteria (x2 concurrent malignancy, x1 non-compliance with maintenance immunosuppression). Disease monitoring was undertaken with serial BVAS disease scoring (Stone *et al.,* 2001) and full biochemical, haematological and immunological profiling. At each time-point of follow-up disease activity was discretised into one of three categories, Flare (at least 1 major or 3 minor BVAS criteria), low-grade activity (0 major and 1-2 minor BVAS criteria) or no activity (0 major or minor BVAS criteria). All disease flares were cross-checked against patient records to confirm clinical impression of disease activity and the need for intensified therapy as a result. Additional flares were defined in the absence of BVAS scoring if patients attended for emergency investigation (bronchoscopy, ophthalmological or ENT review) which confirmed evidence of active disease. To differentiate between discrete flares clear improvement in disease activity was required in the form of an improvement in flare-related symptoms together with a reduction in BVAS score, a reduction in markers of inflammation (CRP, ESR), and a reduction in immunosuppressive therapy.

**Table 3: Vasculitis Entry and exclusion criteria**

| Entry criteria: | |
|---|---|
| i. | Diagnosis of AAV attending/referred to specialist vasculitis unit, Addenbrooke's hospital conforming to CHCC/ARA criteria (Watts *et al*., 2000). |
| ii. | Active disease flare as defined below with the intention of commencing immunosuppressive therapy |
| iii. | None, or minimal, current immunosuppressive therapy |

| Exclusion criteria: | |
|---|---|
| concurrent diagnosis of malignancy non-compliance with treatment | |

The SLE cohort was composed of 25 patients meeting at least four ACR SLE criteria (Tan *et al.,* 1982) presenting with active disease and in whom immunosuppressive therapy was to be instigated or increased between July 2004 and May 2008. Following treatment with an immunosuppressant patients were followed up monthly for up to 52 months. Disease monitoring was undertaken with serial BILAG disease scoring (Isenberg et al, 2005) and full biochemical, haematological and immunological profiling. An episode was defined as a discrete disease flare if it met the following prospectively-defined criteria: 1. new BILAG score A or B in any system, 2. clinical impression of active disease by the reviewing physician and 3. increase in immunosuppressive therapy as a result. Additional flares were defined in the absence of BILAG scoring if patients were admitted directly to hospital as emergency cases for urgent immunosuppressive therapy. To differentiate between disease flares clear improvement in disease activity was required in the form of an improvement in flare-related symptoms together with a reduction in BILAG score and a reduction in immunosuppressive therapy.

**Table 4: SLE entry and exclusion criteria**

| Entry criteria: |
|---|
| Diagnosis of SLE attending/referred to specialist vasculitis unit, Addenbrooke's hospital conforming to ACR criteria for SLE (Tan *et al*., 1982) |
| Active disease with the intention of commencing immunosuppressive therapy |
| None, or minimal, current immunosuppressive therapy |

| Exclusion criteria: |
|---|
| concurrent diagnosis of malignancy non-compliance with treatment |

Controls for the studies of vasculitis and SLE patients were matched for age, sex and ethnicity.

The IBD cohort was composed of 26 patients with Crohn's disease. The entry and exclusion criteria are set out in Table 5.

**Table 5: IBD entry and exclusion criteria**

| Entry criteria: |
|---|
| Diagnosis of Crohn's disease (CD) or ulcerative colitis (UC) |
| Flare of disease - CD - Harvey-Bradshaw Severity Index > 7 |
| UC - Simple Clinical Colitis Activity Index > 5 |
| Both - endoscopic evidence of moderate-severe disease |
| Patient taking less than 10mg Prednisolone orally |
| Patient not taking any immunomodulatory drugs |
| Patients may be taking oral 5-ASAs or using topical therapy |

| Exclusion criteria: |
|---|
| Patients without CD or UC |
| Patients under 18 years old |
| Patients in disease remission |
| Patients receiving oral Prednisolone (>10mg) or intravenous steroids |
| Patients receiving immunomodulatory drugs |
| Non-compliance with therapy |
| Concurrent diagnosis of malignancy |

The transplantation cohort was composed of 26 patients which required renal transplants. The entry and exclusion criteria are set out in Table 6.

**Table 6: Transplant patient entry and exclusion criteria**

| Entry criteria: |
|---|
| Requirement for renal transplant (patients recruited immediately prior to implantation) |
| Note that both living and deceased donors are included, but only single organ (kidney) transplant recipients. |

| Exclusion criteria: |
|---|
| Age < 18 years old |
| Requirement for pre-operative de-sensitisation |

### Cell separation and RNA extraction

Venepuncture was performed at a similar time of day to eliminate background noise from genes whose expression demonstrates circadian variation (Whitney *et al*., 2003). Peripheral blood mononuclear cells (PBMC), CD4 and CD8 T cells, CD19 B cells, CD14 monocytes and CD16 neutrophils were isolated from whole blood by centrifugation over ficoll and positive selection using magnetic beads as previously described (Lyons *et al*., 2007). The purity of separated cell subsets was determined by two-colour flow cytometry. Total RNA was extracted from each cell population using an RNeasy mini kit (Qiagen) according to the manufacturer's instructions. RNA quality was assessed using an Agilent BioAnalyser 2100 and quantified using a NanoDrop ND-1000 spectrophotometer.

### Microarray hybridisation

For expression profiling using the Mediante custom oligonucleotide platform, total RNA (250 ng) was converted into double-stranded cDNA and labelled with Cy3- or Cy5-dCTP as previously described (Lyons *et al.,* 2007). Appropriate Cy3- and Cy5-labelled samples were pooled and hybridised to custom spotted oligonucleotide microarrays comprised of probes representing 24, 654 genes and control features as previously described (Willcocks *et al*., 2008). All samples were hybridised in duplicate, using a dye-swap strategy, against a common reference RNA derived from pooled PBMC samples. Following hybridisation, arrays were washed and scanned on an Agilent G2565B scanner.

For expression profiling using the Affymetrix human gene ST 1.0 microarray platform, aliquots of total RNA (200ng) were labelled using Affymetrix's WT sense Target labelling kit and hybridised to Human Gene 1.0 ST Arrays (Affymetrix) following the manufacturer's instructions. After washing, arrays were scanned using a GS 3000 scanner (Affymetrix).

### Microarray data analysis

Raw image data was extracted using Koadarray v2.4 software (Koada Technology) and probes with a confidence score >0.3 in at least one channel were flagged as present. Extracted data was imported into R where log transformation and background subtraction were performed followed by within array print-tip Loess normalisation and between-array aquantile and scale normalisation in the Limma package (Smyth 2004), part of the bioconductor project (www.bioconductor.org). Normalised data was then imported into Genepattern or Genespring v7.0 (Agilent) for further analysis. Only data demonstrating a strong negative correlation between dye swap replicates and low level expression of excluded cell specific markers was used in downstream analyses. Differential expression between defined phenotypes was assessed using one-way analysis of variance with the false discovery rate controlled at 5%. Genes showing minimal variation between defined phenotypes were excluded from analysis using a log-fold change (LFC) filter set at either 1.5 or 2 fold as specified. The degree of overlap between different genelists was measured in Genepattern or Genespring using the hypergeometric probability function with a specified universe of all T-cell expressed genes. Enrichment of literature-curated gene signatures within different microarray datasets was determined using Gene Set Enrichment Analysis (Subramanian *et al.,* 2005).

Follow-up analysis of disease activity was performed using the Kaplan-Meier survival method with a log-rank test of significance between groups. Comparisons of outcome and associated clinical variables between subgroups were analysed using the non-parametric Mann-Whitney U test or the Chi-square test as appropriate. Mann-Whitney U tests, Chi-square tests, and Kaplan Meier log-rank tests were performed in Prism (GraphPad Software). The Bonferroni correction was applied to correct for multiple testing where appropriate.

### Clustering

Hierarchical clustering and principal components analysis using an uncentered correlation distance metric and average linkage clustering were performed either in Genepattem, Genespring or Cluster with visualisation in Treeview (Eisen *et al.,* 1998).

Multidimensional scaling was performed in BRB-Array Tools version 3.7.0 Beta_2 release developed by Dr Richard Simon and Amy Peng Lam.

Consensus cluster matrices were generated in Genepattern (Reich *et al*., 2006) using resampling-based clustering (Moreno Machine Learning; Monti S (2003)). Comparisons between flat and hierarchical clustering methods were performed using ClusterComparison (Torrente et al., 2005) in ExpressionProfiler:NG (Kapushesky *et al.* 2004).

The reproducibility of the clustering was assessed using the R measure (McShane, 2002) as implemented in BRB ArrayTools (http:/linus.nci.nih.gov/BRB-ArrayTools.html).

### Quantitative RT-PCR

mRNA levels of IL7Ra and CD69 were determined using Taqman Gene Expression Assays (Applied Biosystems) on an ABI PRISM 7900HT instrument according to the manufacturer's instructions. Transcript abundance was calculated by comparison to a standard curve.

### Flow cytometry

Immunophenotyping was performed using a CyAn ADP flow cytometer (Dako), and data was analysed using FlowJo software (Tree Star). At least 500,000 events were collected per sample, reactions were standardised with multicolour calibration particles (BD Biosciences) with saturating concentrations of the following antibodies: PE-Bcl2 (Clone Bcl-2/100, BD Biosciences), APC-CD45RA (Clone HI100, BD Biosciences), PE-Cy5 CD3 (Clone HIT3a, BD Biosciences), PE-Cy7 CCR7 (Clone 3D12, BD Biosciences), PE-CD127 (Clone hIL-7R-M21, BD Biosciences), PE-CD69 (Clone CH/4, Abcam), PE-IL2RA (Clone 143-13, Abcam), Pacific Blue CD8 (Clone RPA-T8, BD Biosciences).

**Table 1: Genes differentially expressed in subtypes 8.1 and 8.2**

| **No.** | **Gene symbol** | **GenBank Accession No.** | **SEQ ID NO.** | **Description** | **Upreg. in subtype** | **P value** | **FDR** | **Fold Change** | **Primer Sequences** |
|---|---|---|---|---|---|---|---|---|---|
| 1 | TXNDC9 | NM_005783 | 1 | Thioredoxin domain containing 9 | 8.1 | 0.001996 | 0.002577 | 8.274832 | Forward: AATAAGGAGGCGGATGTGAC |
| | | | | | | | | | Reverse: GATGCTCCAGGACTTTGGAA |
| 2 | POLR2H | NM_006232 | 4 | Polymerase (RNA) II (DNA directed) polypeptide H | 8.1 | 0.001996 | 0.002577 | 8.919998 | Forward: CTGCCAAGTCACTCAGGTCA |
| | | | | | | | | | Reverse: TTTTCAGCTCTCCCACTGTGT |
| 3 | MRPL14 | NM_032111 | 7 | Mitochondrial ribosomal protein L14 | 8.1 | 0.001996 | 0.002577 | 8.917444 | Forward: CTGGCCATTGCTCAGAACTT |
| | | | | | | | | | Reverse: TTTTCCCACATCCCAGAAAG |
| 4 | GZMH | NM_033423 | 10 | Granzyme H (cathepsin G-like 2, protein h-CCPX) | 8.1 | 0.001996 | 0.002577 | 6.277355 | Forward: GACACAGACCGGTTTCAAGG' |
| | | | | | | | | | Reverse: CCTCTGTCCCAGAGATGGTC |
| 5 | | S56528 | 13 | T cell receptor V-alpha 23 precursor (TCRA) | 8.1 | 0.001996 | 0.002577 | 7.680165 | Forward: GCTCTGAGTGTCCCAGAAGG |
| | | | | | | | | | Reverse: GAGTCACCAGGCTGAGAAGC |
| 6 | ATP8B1 | AK056031 | 16 | CDNA FLJ31469 fis, clone NT2NE2001428 | 8.1 | 0.001996 | 0.002577 | 7.644786 | Forward: CAGCCCTTTGAGGTTGAGAG |
| | | | | | | | | | Reverse: AAATTCCTCCCGTGTGTGTC |
| 7 | LOC150759 | NM_175853 | 19 | | 8.1 | 0.001996 | 0.002577 | 7.463307 | Forward: CTTGAGCCTGCGAGAAGAGT |
| | | | | | | | | | Reverse: CACCAATCGTTTTGTCCTACC |
| 8 | IL7R | NM_002185 | 22 | Interleukin 7 receptor | 8.1 | 0.001996 | 0.002577 | 6.044504 | Forward: CCCTGGGATCAAATCAAGAA |
| | | | | | | | | | Reverse: TGCCACTGAACTCAGGAAGA |
| 9 | CD69 | NM_001781 | 25 | CD69 antigen (p60, early T-cell activation antigen) | 8.1 | 0.001996 | 0.002577 | 5.973867 | Forward: TCTCAATGCCATCAGACAGC |
| | | | | | | | | | Reverse: GGGTGACCAGGTTCCTTTTT |
| 10 | MCM6 | NM_005915 | 28 | MCM6 minichromosome maintenance deficient 6 (MIS5 homolog, S. pombe) (S. cerevisiae) | 8.1 | 0.001996 | 0.002577 | 6.641499 | Forward: CAATTTCTCAAGCACGTGGA* |
| | | | | | | | | | Reverse: CGCACGTCCATCTTATCAAA |
| 11 | CD8B1 | NM_172100 | 31 | CD8 antigen, beta polypeptide 1 (p37) | 8.1 | 0.001996 | 0.002577 | 10.61614 | Forward: GGTGAAGAGGTGGAACAGGA |
| | | | | | | | | | Reverse: CTTGAGGGTGGACTTCTTGG |
| 12 | ITGA2 | NM_002203 | 34 | Integrin, alpha 2 alpha 2 subunit of VLA-2 receptor) | 8.2 | 0.003387 | 0.000687 | 1.240737 | Forward: AGAGGAAAAGGGCACAGACA |
| | | | | | | | | | Reverse: ATGCACATGGGCAGATAACC |
| 13 | PTPN22 | NM_015967 | 37 | Protein tyrosine phosphatase, non-receptor type 22 (lymphoid) | 8.1 | 0.003387 | 0.000687 | 2.504022 | Forward: GATTGTATGCAGGCCCAATC* |
| | | | | | | | | | Reverse AACGGTTTGCAAAACCAAAA |
| 14 | NOTCH1 | NM_017617 | 40 | Notch 1 | 8.2 | 0.003387 | 0.000687 | 1.388599 | Forward: TTGGGAGGAGCAGATTTTTG |
| | | | | | | | | | Reverse: GAGGCTGCCCTGAGGAGT |

| | | | | | | | | | |
|---|---|---|---|---|---|---|---|---|---|
| * primer overlaps exon boundary | | | | | | | | | |

**Table 2: Genes differentially expressed in subtypes 4.1 and 4.2**

| **No.** | **Gene symbol** | **GenBank Accession No.** | **SEQ ID NO.** | **Description** | **Upreg. in subtype** | **P value** | **FDR** | **Fold Change** | **Primer Sequences** |
|---|---|---|---|---|---|---|---|---|---|
| 1 | SLAMF1 | NM_003037 | 43 | Signaling lymphocytic activation molecule family member 1 | 4.1 | 0.002988 | 0.002578 | 4.882732 | Forward: CGTCACAATGGCAAAATCAC |
| | | | | | | | | | Reverse: TTCTGGAGTGGAGACCTGCT |
| 2 | TNFSF5 | NM_000074 | 46 | Tumor necrosis factor (ligand) superfamily, member 5 (hyper-IgM syndrome) | 4.1 | 0.002988 | 0.002578 | 5.084538 | Forward: AACCCTGGAAAATGGGAAAC |
| | | | | | | | | | Reverse: CCTCCCAAGTGAATGGATTG |
| 3 | DGKA | NM_001345 | 49 | Diacylglycerol kinase, alpha 80kDa | 4.1 | 0.002988 | 0.002578 | 5.146665 | Forward: TCGAATTTGCCACATCTGAA |
| | | | | | | | | | Reverse: GGATATCAGGGTCGGTGATG* |
| 4 | GPR171 | NM_013308 | 52 | G protein-coupled receptor 171 | 4.1 | 0.002988 | 0.002578 | 5.202988 | Forward: CCAGCTACACAACCTTGGAG^{*} |
| | | | | | | | | | Reverse: CCCAGGTTGCAAAACAACTT |
| 5 | IF144 | NM_006417 | 55 | Interferon-induced protein 44 | 4.1 | 0.002988 | 0.002578 | 5.267047 | Forward: AGCCTGTGAGGTCCAAGCTA* |
| | | | | | | | | | Reverse: TTGCTCAAAAGGCAAATCCT* |
| 6 | P2RY5 | NM_005767 | 58 | Purinergic receptor P2Y, G-protein coupled, 5 | 4.1 | 0.002988 | 0.002578 | 5.329141 | Forward: AACACAAACATTTGTTAATTGCTCA |
| | | | | | | | | | Reverse: TGCACCATGAACTTCAGAGAA |
| 7 | LEF1 | NM_016269 | 61 | Lymphoid enhancer-binding factor 1 | 4.1 | 0.002988 | 0.002578 | 5.338575 | Forward: GCTTCTCTGTGAATTGCCTGT |
| | | | | | | | | | Reverse: TGCAAACCAGTCTGCTGAAC |
| 8 | CD69 | NM_001781 | 25 | CD69 antigen (p60, early T-cell activation antigen) | 4.1 | 0.002988 | 0.002578 | 5.924527 | Forward: TCTCAATGCCATCAGACAGC |
| | | | | | | | | | Reverse: GGGTGACCAGGTTCCTTTTT |
| 9 | IL7R | NM_002185 | 22 | Interleukin 7 receptor | 4.1 | 0.002988 | 0.002578 | 6.73535 | Forward: CCCTGGGATCAAATCAAGAA |
| | | | | | | | | | Reverse: TGCCACTGAACTCAGGAAGA |
| 10 | RP42 | NM_020640 | 64 | RP42 homolog | 4.1 | 0.002988 | 0.002578 | 8.379483 | Forward: CAAGCATACACGATGCAAAA |
| | | | | | | | | | Reverse: TCTAGCCTGCCTTACGGAAA |
| 11 | ITGA2 | NM_002203 | 34 | Integrin, alpha 2 (CD49B, alpha 2 subunit of VLA-2 receptor) | 4.2 | 0.003387 | 0.000687 | 1.240737 | Forward: AGAGGAAAAGGGCACAGACA |
| | | | | | | | | | Reverse: ATGCACATGGGCAGATAACC |
| 12 | PTPN22 | NM_015967 | 37 | Protein tyrosine phosphatase, non-receptor type 22 (lymphoid) | 4.1 | 0.003387 | 0.000687 | 2.504022 | Forward: GATTGTATGCAGGCCCAATC* |
| | | | | | | | | | Reverse AACGGTTTGCAAAACCAAAA |
| 13 | NOTCH1 | NM_017617 | 40 | Notch 1 | 4.2 | 0.003387 | 0.000687 | 1.388599 | Forward: TTGGGAGGAGCAGATTTTTG |
| | | | | | | | | | Reverse: GAGGCTGCCCTGAGGAGT |

| | | | | | | | | | |
|---|---|---|---|---|---|---|---|---|---|
| * primer overlaps exon boundary | | | | | | | | | |

### Additional statements of invention

1. A method of assessing whether a subject is at high or low risk of autoimmune disease progression, which method comprises establishing, by determining the expression level of one or more genes in a CD8 cell from said subject, whether said subject has a high risk (CD8.1) or low risk (CD8.2) CD8 cell subtype,
   wherein a CD8.1 subtype is characterised by upregulated expression of genes 1 to 11 listed in Table 1 relative to the level of expression of the same genes in subtype CD8.2.
2. A method according to paragraph 1, wherein the autoimmune disease is selected from the group of: vasculitis, systemic lupus erythematosus (SLE), rheumatoid arthritis, multiple sclerosis, and inflammatory bowel disease.
3. A method of assessing whether a subject is at high or low risk of autoimmune disease progression, which method comprises establishing, by determining the expression level of one or more genes in a CD4 cell from said subject, whether said subject has a high risk (CD4.1) or low risk (CD4.2) CD4 cell subtype,
   wherein a CD4.1 subtype is characterised by differential expression of genes 1 to 10 listed in Table 2 relative to the level of expression of the same genes in subtype CD4.2.
4. A method according to paragraph 3, wherein the autoimmune disease is selected from the group of: vasculitis, rheumatoid arthritis, multiple sclerosis, and inflammatory bowel disease.
5. A method of assessing whether a subject has a CD8.1 or CD8.2 CD8 cell subtype, which method comprises determining the expression level of one or more genes in a CD8 cell from said subject,
   wherein a CD8.1 subtype is characterised by upregulated expression of genes 1 to 11 listed in Table 1 relative to the level of expression of the same genes in subtype CD8.2.
6. A method of assessing whether a subject has a CD4.1 or CD4.2 CD4 cell subtype, which method comprises determining the expression level of one or more genes in a CD4 cell from said subject,
   wherein a CD4.1 subtype is characterised by differential expression of genes 1 to 10 listed in Table 2 relative to the level of expression of the same genes in subtype CD4.2.
7. A method according to any one of the preceding paragraphs, wherein the method comprises determining the expression level of one, two, or three genes selected from the group of: ITGA2, PTPN22 and NOTCH1.
8. A method of assessing whether a subject is at high or low risk of autoimmune disease progression, which method comprises establishing, by determining the level of expression of one or more proteins on the surface of a CD8 cell from said subject, whether said subject has a high risk (CD8.1) or low risk (CD8.2) CD8 cell subtype,
   wherein a CD8.1 subtype is characterised by differential expression of the protein relative to the level of expression of the protein in subtype CD8.2.
9. A method of assessing whether a subject is at high or low risk of autoimmune disease progression, which method comprises establishing, by determining the level of expression of one or more proteins on the surface of a CD4 cell from said subject, whether said subject has a high risk (CD4.1) or low risk (CD4.2) CD4 cell subtype,
   wherein a CD4.1 subtype is characterised by differential expression of the protein relative to the level of expression of the protein in subtype CD4.2.
10. A method of assessing whether a subject is at high or low risk of autoimmune disease progression, which method comprises establishing, by determining level of expression of one or more proteins in a sample obtained from said subject, whether said subject has a high risk (CD8.1) or low risk (CD8.2) CD8 cell subtype,
   wherein a CD8.1 subtype is characterised by differential expression of the protein relative to the level of expression of the protein in subtype CD8.2.
11. A method of assessing whether a subject is at high or low risk of autoimmune disease progression, which method comprises establishing, by determining the level of expression of one or more proteins in a sample obtained from said subject, whether said subject has a high risk (CD4.1) or low risk (CD4.2) CD4 cell subtype,
   wherein a CD4.1 subtype is characterised by differential expression of the protein relative to the level of expression of the protein in subtype CD4.2.
12. A method according to any one of paragraphs 8 to 11, wherein the proteins are one, two, or three proteins selected from the proteins expressed by the genes ITGA2, PTPN22 and NOTCH 1.
13. A method of identifying genes differentially expressed in subjects with a high risk and subjects with a low risk of autoimmune disease progression, comprising:
   (i) determining the level of CD8 or CD4 cell gene expression in subjects with autoimmune disease using microarray analysis,
   (ii) dividing the subjects into two groups based on their CD8 or CD4 cell gene expression levels using a clustering method,
   (iii) identifying the group with the higher level of autoimmune disease progression, and
   (iv) identifying the genes differentially expressed in subjects with a high risk (CD8.1 or CD4.1) cell subtype and subjects with a low risk (CD8.2 or CD4.2) cell subtype.
14. A kit for assessing whether a subject is at high or low risk of autoimmune disease progression, wherein said kit comprises reagents for establishing the expression level of one or more genes in a CD8 cell from said subject, for determining whether said subject has a high risk (CD8.1) or low risk (CD8.2) CD8 cell subtype,
   wherein a CD8.1 subtype is characterised by upregulated expression of genes 1 to 11 listed in Table 1 relative to the level of expression of the same genes in subtype CD8.2.
15. A kit for assessing whether a subject is at high or low risk of autoimmune disease progression, wherein said kit comprises reagents for establishing the expression level of one or more genes in a CD4 cell from said subject, for determining whether said subject has a high risk (CD4.1) or low risk (CD4.2) CD4 cell subtype,
   wherein a CD4.1 subtype is characterised by differential expression of genes 1 to 10 listed in Table 2 relative to the level of expression of the same genes in subtype CD4.2.
16. A kit according to paragraph 14 or 15, wherein the kit comprises reagents for establishing the expression level of one, two, or three genes selected from the group of: ITGA2, PTPN22 and NOTCH1.
17. Use of a kit according to any once of paragraphs 8 to 9 for assessing whether a subject is at high or low risk of autoimmune disease progression by determining whether said subject has a high risk or low risk cell subtype.

### References

Abdulahad, W. H., Stegeman, C. A., Limburg, P. C. & Kallenberg, C. G. Skewed distribution of Th17 lymphocytes in patients with Wegener's granulomatosis in remission. Arthritis Rheum 58, 2196-205 (2008).
Abdulahad, W. H., van der Geld, Y. M., Stegeman, C. A. & Kallenberg, C. G. Persistent expansion of CD4+ effector memory T cells in Wegener's granulomatosis. Kidney Int 70, 938-47 (2006).
Allantaz, F., Chaussabel, D., Banchereau, J. & Pascual, V. Microarray-based identification of novel biomarkers in IL-1-mediated diseases. Curr Opin Immunol 19, 623-32 (2007).
Baechler, E. C. et al. Interferon-inducible gene expression signature in peripheral blood cells of patients with severe lupus. Proc Natl Acad Sci U S A 100, 2610-5 (2003).
Bao T, Davidson NE, Gene expression profiling of breast cancer, Adv. Surg.; 42:249-60 (2008)
Batliwalla, F. M. et al. Peripheral blood gene expression profiling in rheumatoid arthritis. Genes Immun 6, 388-97 (2005).
Bennett, L. et al. Interferon and granulopoiesis signatures in systemic lupus erythematosus blood. J Exp Med 197, 711-23 (2003).
Booth, A. D. et al. Outcome of ANCA-associated renal vasculitis: a 5-year retrospective study. Am J Kidney Dis 41, 776-84 (2003).
Bootsma, H. et al. The predictive value of fluctuations in IgM and IgG class anti-dsDNA antibodies for relapses in systemic lupus erythematosus. A prospective long-term observation. Ann Rheum Dis 56, 661-6 (1997).
Carr et al. CD27 mediates interleukin-2-independent clonal expansion of the CD8+ T cell without effector differentiation. Proc. Natl Acad. Sci 103:19454-19459 (2006).
Charbonnier, L.M. et al. Immature dendritic cells suppress collagen-induced arthritis by in vivo expansion of CD49b+ regulatory T cells. J Immunol 177, 3806-13 (2006).
Chen, Z. Laurence, A. O'Shea, J.J. Signal transduction pathways and transcriptional regulation in the control of Th17 differentiation. Semin Immunol. 19(6):400-8 (2007)
Eisen, M. B., Spellman, P. T., Brown, P. O. & Botstein, D. Cluster analysis and display of genome-wide expression patterns. Proc Natl Acad Sci U S A 95, 14863-8 (1998)
Golub, T.R. et al Molecular classification of cancer: class discovery and class prediction by gene expression monitoring. Science 286, 531-7 (1999)
Haining, W. N. et al. Identification of an evolutionary conserved transcriptional signature of CD8 memory differentiation that is shared by T and B cells. J Immunol 181, 1859-68 (2008).
Hand, T. W., Morre, M. & Kaech, S. M. Expression of IL-7 receptor alpha is necessary but not sufficient for the formation of memory CD8 T cells during viral infection. Proc Natl Acad Sci U S A 104, 11730-5 (2007).
Hochberg, Y and Benjamini, Y. More powerful procedures for multiple significance testing. Stat Med 9, 811-8 (1990).
Isenberg, D. A. et al. BILAG 2004. Development and initial validation of an updated version of the British Isles Lupus Assessment Group's disease activity index for patients with systemic lupus erythematosus. Rheumatology (Oxford) 44, 902-6 (2005).
Ivanov, I.I. Zhou, L. Littman D.R. Transcriptional regulation of Th17 differentiation Semin Immunol; 19(6):409-17 (2007)
Kaech, S. M. et al. Selective expression of the interteukin 7 receptor identifies effector CD8 T cells that give rise to long-lived memory cells. Nat Immunol 4, 1191-8 (2003).
Kapushesky, M. et al. Expression Profiler: next generation-an online platform for analysis of microarray data. Nucleic Acids Res 32, W465-70 (2004).
Kapushesky, M. et al. Expression Profiler: next generation-an online platform for analysis of microarray data. Nucleic Acids Res 32, W465-70 (2004).
Kassiotis, G., Gray, D., Kiafard, Z., Zwimer, J. & Stockinger, B. Functional specialization of memory Th cells revealed by expression of integrin CD49b. J Immunol 177, 968-75 (2006).
Kinter J, Zeis T, Schaeren-Wiemers N., RNA profiling of MS brain tissues, Int. MS J., 15(2):51-8(2008)
Kondrack, R. M. et al. Interleukin 7 regulates the survival and generation of memory CD4 cells. J Exp Med 198, 1797-806 (2003).
Lamprecht, P. Off balance: T-cells in antineutrophil cytoplasmic antibody (ANCA)-associated vasculitides. Clin Exp Immunol 141, 201-10 (2005).
Lane, S. E., Watts, R. A., Shepstone, L. & Scott, D. G. Primary systemic vasculitis: clinical features and mortality. Qjm 98, 97-111 (2005).
Langford, C. A. Antineutrophil cytoplasmic antibodies should not be used to guide treatment in Wegener's granulomatosis. Clin Exp Rheumatol 22, S3-6 (2004).
Le Brigand, K. et al. An open-access long oligonucleotide microarray resource for analysis of the human and mouse transcriptomes. Nucleic Acids Res 34, e87 (2006).
Li, J., Huston, G. & Swain, S. L. IL-7 promotes the transition of CD4 effectors to persistent memory cells. J Exp Med 198, 1807-15 (2003).
Lyons, P. A. et al. Microarray analysis of human leucocyte subsets: the advantages of positive selection and rapid purification. BMC Genomics 8, 64 (2007).
Lyons, P.A. et al. Novel expression signatures identified by transcriptional analysis of separated leukocyte subsets in SLE and vasculitis. Ann Rheum Dis In Press (2009).
Matesic, L.E., Haines, D.C., Copeland, N.G. & Jenkins, N.A. Itch genetically interacts with Notchl in a mouse autoimmune disease model. Hum Mol Genet 15, 3485-97 (2006).
McShane, L. R., MD. Freidlin, B. Yu, R. Li MC. Simon, R. Methods of assessing reproducibility of clustering patterns observed in analyses of microarray data. J Comp Biol 9. 505-11 (2002).
Monti, S. Consensus Clustering: A resampling-based method for class discovery and visualisation of gene expression microarray data. Machine Learning Journal 52, 91-118 (2003).
Okamoto, M. et al. Essential role of Notch signaling in effector memory CD8+ T cell-mediated airway hyperresponsiveness and inflammation. J Exp Med 205, 1087-97 (2008).
Pellegrini, M. et al. Loss of Bim increases T cell production and function in interleukin 7 receptor-deficient mice. J Exp Med 200, 1189-95 (2004).
Petalidis, L. et al. Global amplification of mRNA by template-switching PCR: linearity and application to microarray analysis. Nucleic Acids Res 31, e142 (2003).
Popper, S. J. et al. Gene-expression patterns reveal underlying biological processes in Kawasaki disease. Genome Biol. 8, R261 (2007).
Reich, M. et al. GenePattern 2.0. Nat Genet 38, 500-1 (2006).
Rhee DK, Park SH, Jang YK, Molecular signatures associated with transformation and progression to breast cancer in the isogenic MCF10 model, Genomics 92(6):419-28 (2008).
Rieck, M. et al. Genetic variation in PTPN22 corresponds to altered function of T and B lymphocytes. J Immunol 179, 4704-10 (2007).
Seddon, B., Tomlinson, P. & Zamoyska, R. Interleukin 7 and T cell receptor signals regulate homeostasis of CD4 memory cells. Nat Immunol 4, 680-6 (2003).
Smyth, G. K. Linear models and empirical bayes methods for assessing differential expression in microarray experiments. Stat Appl Genet Mol Biol 3, Article3 (2004).
Smyth, D.J. et al. Shared and distinct genetic variants in type 1 diabetes and celiac disease. N Engl J Med 359, 2767-77 (2008).
Stone, J. H. et al. A disease-specific activity index for Wegeners granulomatosis: modification of the Birmingham Vasculitis Activity Score. International Network for the Study of the Systemic Vasculitides (INSSYS). Arthritis Rheum 44, 912-20 (2001).
Strasser, A., Harris, A. W. & Cory, S. bcl-2 transgene inhibits T cell death and perturbs thymic self- censorship. Cell 67, 889-99 (1991).
Subramanian, A. et al. Gene set enrichment analysis: a knowledge-based approach for interpreting genome-wide expression profiles. Proc Natl Acad Sci U S A 102, 15545-50 (2005).
Tan, E. M. et al. The 1982 revised criteria for the classification of systemic lupus erythematosus. Arthritis Rheum 25, 1271-7 (1982).
Torrente, A., Kapushesky, M. & Brazma, A. A new algorithm for comparing and visualizing relationships between hierarchical and flat gene expression data clusterings. Bioinformatics 21, 3993-9 (2005).
Watts, R. A., Lane, S. E., Bentham, G. & Scott, D. G. Epidemiology of systemic vasculitis: a ten-year study in the United Kingdom. Arthritis Rheum 43,414-9 (2000).
Weidner, S., Geuss, S., Hafezi-Rachti, S., Wonka, A. & Rupprecht, H. D. ANCA-associated vasculitis with renal involvement: an outcome analysis. Nephrol Dial Transplant 19, 1403-11 (2004).
Whitney, A. R. et al. Individuality and variation in gene expression patterns in human blood. Proc Natl Acad Sci U S A 100, 1896-901 (2003).
Willcocks, L. C. et al. Copy number of FCGR3B, which is associated with systemic lupus erythematosus, correlates with protein expression and immune complex uptake. J Exp Med 205, 1573-82 (2008).

## Claims

1. A method of assessing whether a subject is at high or low risk of autoimmune disease progression, which method comprises establishing, by determining the expression level of one or more genes in a CD8 cell from said subject, whether said subject has a high risk (CD8.1) or low risk (CD8.2) CD8 cell subtype, wherein said one or more genes are selected from the group of:
| No.. | GenBank Accession No. | SEQ ID NO. | Upregulated in subtype |
|---|---|---|---|
| 1 | NM_005783 | 1 | 8.1 |
| 2 | NM_006232 | 4 | 8.1 |
| 3 | NM_032111 | 7 | 8.1 |
| 4 | NM_033423 | 10 | 8.1 |
| 5 | S56528 | 13 | 8.1 |
| 6 | AK056031 | 16 | 8.1 |
| 7 | NM_175853 | 19 | 8.1 |
| 8 | NM_002185 | 22 | 8.1 |
| 9 | NM_001781 | 25 | 8.1 |
| 10 | NM_005915 | 28 | 8.1 |
| 11 | NM_172100 | 31 | 8.1 |
and, wherein a CD8.1 subtype is **characterised by** upregulated expression of the above genes relative to the level of expression of the same genes in subtype CD8.2.

2. A method according to claim 1, wherein the autoimmune disease is selected from the group of: vasculitis, systemic lupus erythematosus (SLE), rheumatoid arthritis, multiple sclerosis, and inflammatory bowel disease (IBD).

3. A method of assessing whether a subject is at high or low risk of autoimmune disease progression, which method comprises establishing, by determining the expression level of one or more genes in a CD4 cell from said subject, whether said subject has a high risk (CD4.1) or low risk (CD4.2) CD4 cell subtype, wherein said one or more genes are selected from the group of:
| No. | GenBank Accession No. | SEQ ID NO. | Upregulated in subtype |
|---|---|---|---|
| 1 | NM_003037 | 43 | 4.1 |
| 2 | NM_000074 | 46 | 4.1 |
| 3 | NM_001345 | 49 | 4.1 |
| 4 | NM_013308 | 52 | 4.1 |
| 5 | NM_ 006417 | 55 | 4.1 |
| 6 | NM_005767 | 58 | 4.1 |
| 7 | NM_016269 | 61 | 4.1 |
| 8 | NM_001781 | 25 | 4.1 |
| 9 | NM_002185 | 22 | 4.1 |
| 10 | NM_020640 | 64 | 4.1 |
and, wherein a CD4.1 subtype is **characterised by** differential expression of the above genes relative to the level of expression of the same genes in subtype CD4.2.

4. A method according to claim 3, wherein the method comprises determining the expression level of PCAF (NM_003884; GI 8850).

5. A method according to claim 3 or 4, wherein the autoimmune disease is selected from the group of: vasculitis, rheumatoid arthritis, multiple sclerosis, and inflammatory bowel disease.

6. A method according to any one of claims 1 to 5 comprising providing a sample obtained from the subject.

7. A method according to any one of claims 1 to 6, wherein the sample is a whole blood sample or a peripheral blood mononuclear cell (PBMC) sample.

8. A method according to claim 6 or 7 comprising bringing the sample into contact with a reagent suitable for determining the expression level of said one or more genes.

9. A method according to any one of the preceding claims, wherein said expression level of said one or more genes is determined using polymerase chain reaction (PCR), or using a microarray.

10. A method according to any one of claims 1 to 8, wherein said expression level of said one or more genes is determined by measuring the level of protein expressed from said gene and, wherein the level of protein expression is optionally determined using an enzyme-linked immunosorbent assay (ELISA), western blotting, mass-spectrometry, or flow cytometry.

11. Use of a kit for assessing whether a subject is at high or low risk of autoimmune disease progression by determining whether said subject has a high risk (CD8.1) or low risk (CD8.2) CD8 cell subtype,
wherein said kit comprises primers for establishing the expression level of one or more genes in a CD8 cell from said subject, for determining whether said subject has a high risk (CD8.1) or low risk (CD8.2) CD8 cell subtype, said one or more genes being selected from the genes listed in claim 1, and
wherein a CD8.1 subtype is **characterised by** upregulated expression of the genes listed in claim 1 relative to the level of expression of the same genes in subtype CD8.2.

12. Use of a kit for assessing whether a subject is at high or low risk of autoimmune disease progression by determining whether said subject has a high risk (CD4.1) or low risk (CD4.2) CD4 cell subtype,
wherein said kit comprises primers for establishing the expression level of one or more genes in a CD4 cell from said subject, for determining whether said subject has a high risk (CD4.1) or low risk (CD4.2) CD4 cell subtype, said one or more genes being selected from the genes listed in claim 3, and wherein a CD4.1 subtype is **characterised by** differential expression of the genes listed in claim 3 relative to the level of expression of the same genes in subtype CD4.2.

13. Use of a kit according to claim 12, wherein the kit comprises primers for determining the level of expression of PCAF (NM_003884; GI 8850).

## Patentansprüche

1. Verfahren zur Bewertung, ob bei einem Individuum ein hohes oder geringes Risiko für das Fortschreiten einer Autoimmunerkrankung besteht, wobei das Verfahren das Festlegen, ob das Individuum einen Hochrisiko- (CD8.1) oder einen Niedrigrisiko- (CD8.2) CD8-Zell-Subtypus aufweist, durch Bestimmen des Expressionsausmaßes eines oder mehrerer Gene in einer CD8-Zelle des Individuums umfasst, worin das eine oder mehrere der Gene aus der Gruppe bestehend aus den Folgenden ausgewählt sind:
| Nr. | GenBank-Zugriffsnr. | Seq.-ID Nr. | Hochreguliert im Subtypus |
|---|---|---|---|
| 1 | NM_005783 | 1 | 8.1 |
| 2 | NM_006232 | 4 | 8.1 |
| 3 | NM_032111 | 7 | 8.1 |
| 4 | NM_033423 | 10 | 8.1 |
| 5 | S56528 | 13 | 8.1 |
| 6 | AK056031 | 16 | 8.1 |
| 7 | NM_175853 | 19 | 8.1 |
| 8 | NM_002185 | 22 | 8.1 |
| 9 | NM_001781 | 25 | 8.1 |
| 10 | NM_005915 | 28 | 8.1 |
| 11 | NM_172100 | 31 | 8.1 |
und worin ein CD8.1-Subtypus im Vergleich mit dem Expressionsausmaß derselben Gene im Subtypus CD8.2 durch eine hochregulierte Expression der obigen Gene gekennzeichnet ist.

2. Verfahren nach Anspruch 1, worin die Autoimmunerkrankung aus der Gruppe bestehend aus den Folgenden ausgewählt ist: Vaskulitis, systemischem Lupus erythematodes (SLE), rheumatoider Arthritis, multipler Sklerose und entzündlicher Darmerkrankung (IBD).

3. Verfahren zur Bewertung, ob bei einem Individuum ein hohes oder geringes Risiko für das Fortschreiten einer Autoimmunerkrankung besteht, wobei das Verfahren das Festlegen, ob das Individuum einen Hochrisiko- (CD4.1) oder einen Niedrigrisiko- (CD4.2) CD4-Zell-Subtypus aufweist, durch Bestimmen des Expressionsausmaßes eines oder mehrerer Gene in einer CD4-Zelle des Individuums umfasst, worin das eine oder mehrere der Gene aus der Gruppe bestehend aus den Folgenden ausgewählt sind:
| Nr. | GenBank-Zugriffsnr. | Seq.-ID Nr. | Hochreguliert im Subtypus |
|---|---|---|---|
| 1 | NM_003037 | 43 | 4.1 |
| 2 | NM_000074 | 46 | 4.1 |
| 3 | NM_001345 | 49 | 4.1 |
| 4 | NM_013308 | 52 | 4.1 |
| 5 | NM_006417 | 55 | 4.1 |
| 6 | NM_005767 | 58 | 4.1 |
| | | | |
| 7 | NM_016269 | 61 | 4.1 |
| 8 | NM_001781 | 25 | 4.1 |
| 9 | NM_002185 | 22 | 4.1 |
| 10 | NM_020640 | 64 | 4.1 |
und worin ein CD4.1-Subtypus im Vergleich mit dem Expressionsausmaß derselben Gene im Subtypus CD4.2 durch eine andere Expression der obigen Gene gekennzeichnet ist.

4. Verfahren nach Anspruch 3, worin das Verfahren das Bestimmen des Expressionsausmaßes von PCAF (NM_003884; Gl 8850) umfasst.

5. Verfahren nach Anspruch 3 oder 4, worin die Autoimmunerkrankung aus der Gruppe bestehend aus den Folgenden ausgewählt ist: Vaskulitis, rheumatoider Arthritis, multipler Sklerose und entzündlicher Darmerkrankung.

6. Verfahren nach einem der Ansprüche 1 bis 5, das das Bereitstellen einer Probe umfasst, die aus dem Individuum entnommen wurde.

7. Verfahren nach einem der Ansprüche 1 bis 6, worin die Probe eine Vollblutprobe oder eine Probe aus peripheren mononuklearen Blutzellen (PBMC) ist.

8. Verfahren nach Anspruch 6 oder 7, das das Kontaktieren der Probe mit einem geeigneten Reagens zum Bestimmen des Expressionsausmaßes des einen oder mehrerer der Gene umfasst.

9. Verfahren nach einem der vorangegangenen Ansprüche, worin das Expressionsausmaß des einen oder mehrerer der Gene unter Verwendung einer Polymerasekettenreaktion (PCR) oder unter Verwendung einer Mikroanordnung bestimmt wird.

10. Verfahren nach einem der Ansprüche 1 bis 8, worin das Expressionsausmaß des einen oder mehrerer der Gene durch Messen des Ausmaßes der Proteinexpression aus dem Gen bestimmt wird und worin das Ausmaß der Proteinexpression gegebenenfalls unter Verwendung eines enzymgebundenen Immunosorptionstests (ELISA), Western-Blotting, Massenspektrometrie oder Durchflusszytometrie bestimmt wird.

11. Verwendung eines Sets zur Bewertung, ob bei einem Individuum ein hohes oder geringes Risiko für das Fortschreiten einer Autoimmunerkrankung besteht, durch das Bestimmen, ob das Individuum einen Hochrisiko- (CD8.1) oder einen Niedrigrisiko- (CD8.2) CD8-Zell-Subtypus aufweist,
worin das Set Primer zum Festlegen des Expressionsausmaßes eines oder mehrerer Gene in einer CD8-Zelle aus dem Individuum umfasst, zum Bestimmen, ob das Individuum einen Hochrisiko- (CD8.1) oder Niedrigrisiko- (CD8.2) Zell-Subtypus aufweist, wobei ein oder mehrere Gene aus den in Anspruch 1 aufgelisteten Genen ausgewählt sind, und
worin ein CD8.1-Subtypus durch eine hochregulierte Expression der in Anspruch 1 aufgelisteten Gene, verglichen mit dem Expressionsausmaß derselben Gene in Subtypus 8.2, gekennzeichnet ist.

12. Verwendung eines Sets zur Bewertung, ob bei einem Individuum ein hohes oder geringes Risiko für das Fortschreiten einer Autoimmunerkrankung besteht, durch das Bestimmen, ob das Individuum einen Hochrisiko- (CD4.1) oder einen Niedrigrisiko- (CD4.2) CD4-Zell-Subtypus aufweist,
worin das Set Primer zum Festlegen des Expressionsausmaßes eines oder mehrerer Gene in einer CD4-Zelle aus dem Individuum umfasst, zum Bestimmen, ob das Individuum einen Hochrisiko- (CD4.1) oder Niedrigrisiko- (CD4.2) Zell-Subtypus aufweist, wobei ein oder mehrere Gene aus den in Anspruch 3 aufgelisteten Genen ausgewählt sind, und worin ein CD4.1-Subtypus durch eine andere Expression der in Anspruch 3 aufgelisteten Gene, verglichen mit dem Expressionsausmaß derselben Gene in Subtypus 4.2, gekennzeichnet ist.

13. Verwendung eines Sets nach Anspruch 12, worin das Set Primer zur Bestimmung des Expressionsausmaßes von PCAF (NM_003884; Gl 8850) umfasst.

## Revendications

1. Procédé pour évaluer si un sujet présente un risque important ou faible de progression d'une maladie autoimmune, lequel procédé comporte l'étape consistant à établir, en déterminant le niveau d'expression d'un ou de plusieurs gènes dans une cellule CD8 dudit sujet, si le sujet présente un sous-type de cellule CD8 à risque important (CD8.1) ou à risque faible (CD8.2), dans lequel lesdits un ou plusieurs gènes sont sélectionnés dans le groupe comprenant :
| N° | N° d'accès GenBank | SEQ ID NO. | Régulation vers le haut dans sous-type |
|---|---|---|---|
| 1 | NM_005783 | 1 | 8,1 |
| 2 | NM_006232 | 4 | 8,1 |
| 3 | NM_032111 | 7 | 8,1 |
| 4 | NM_033423 | 10 | 8,1 |
| 5 | S56528 | 13 | 8,1 |
| 6 | AK056031 | 16 | 8,1 |
| 7 | NM_175853 | 19 | 8,1 |
| 8 | NM_002185 | 22 | 8,1 |
| 9 | NM_001781 | 25 | 8,1 |
| 10 | NM_005915 | 28 | 8,1 |
| 11 | NM_172100 | 31 | 8,1 |
et dans lequel un sous-type CD8.1 est **caractérisé par** une expression régulée vers le haut des gènes ci-dessus par rapport au niveau d'expression des mêmes gènes dans le sous-type CD8.2.

2. Procédé selon la revendication 1, dans lequel la maladie auto-immune est sélectionnée dans le groupe comprenant : la vasculite, le lupus érythémateux systémique (SLE), la polyarthrite rhumatoïde, la sclérose en plaques, et une maladie intestinale inflammatoire (IBD).

3. Procédé pour évaluer si un sujet présente un risque important ou faible de progression de maladie auto-immune, lequel procédé comporte l'étape consistant à établir, en déterminant le niveau d'expression d'un ou de plusieurs gènes dans une cellule CD4 dudit sujet, si le sujet présente un sous-type de cellule CD4 à risque important (CD4.1) ou à risque faible (CD4.2), dans lequel lesdits un ou plusieurs gènes sont sélectionnés dans le groupe comprenant :
| N° | N° d'accès GenBank | SEQ ID NO. | Régulation vers le haut dans sous-type |
|---|---|---|---|
| 1 | NM_003037 | 43 | 4,1 |
| 2 | NM_000074 | 46 | 4,1 |
| 3 | NM_001345 | 49 | 4,1 |
| 4 | NM_013308 | 52 | 4,1 |
| 5 | NM_006417 | 55 | 4,1 |
| 6 | NM_005767 | 58 | 4,1 |
| 7 | NM_ | 61 | 4,1 |
| 8 | NM_ | 25 | 4,1 |
| 9 | NM_ | 22 | 4,1 |
| 10 | NM_ | 64 | 4,1 |
et dans lequel un sous-type CD4.1 est **caractérisé par** une expression différentielle des gènes ci-dessus par rapport au niveau d'expression des mêmes gènes dans le sous-type CD4.2.

4. Procédé selon la revendication 3, dans lequel le procédé comprend l'étape consistant à déterminer le niveau d'expression de PCAF (NM_003884 ; GI 8850).

5. Procédé selon la revendication 3 ou 4, dans lequel la maladie auto-immune est sélectionnée dans le groupe comprenant : la vasculite, la polyarthrite rhumatoïde, la sclérose en plaques, et une maladie intestinale inflammatoire.

6. Procédé selon l'une quelconque des revendications 1 à 5, comprenant l'étape consistant à fournir un échantillon obtenu auprès du sujet.

7. Procédé selon l'une quelconque des revendications 1 à 6, dans lequel l'échantillon est un échantillon de sang total ou un échantillon de cellule mononucléaire de sang périphérique (PMBC).

8. Procédé selon la revendication 6 ou 7, comprenant l'étape consistant à amener l'échantillon en contact avec un réactif adapté pour déterminer le niveau d'expression desdits un ou plusieurs gènes.

9. Procédé selon l'une quelconque des revendications précédentes, dans lequel ledit niveau d'expression desdits uns ou plusieurs gènes est déterminé en utilisant une réaction en chaîne par polymérase (PCR) ou en utilisant un micro-dosage.

10. Procédé selon l'une quelconque des revendications 1 à 8, dans lequel ledit niveau d'expression desdits uns ou plusieurs gènes est déterminé en mesurant le niveau de protéines exprimé par ledit gène, et dans lequel le niveau d'expression des protéines est déterminé facultativement en utilisant un dosage immuno-enzymatique (ELISA), la technique de Western blot, une spectrométrie de masse et une cytométrie en flux.

11. Utilisation d'un kit pour évaluer si un sujet présente un risque important ou faible de progression d'une maladie auto-immune, en déterminant si ledit sujet présente un sous-type de cellule CD8 à risque important (CD8.1) ou à risque faible (CD8.2),
où ledit kit comprend des amorces pour établir le niveau d'expression d'un ou de plusieurs gènes dans une cellule CD8 dudit sujet, pour déterminer si le sujet présente un sous-type de cellule CD8 à risque important (CD8.1) ou à risque faible (CD8.2), lesdits un ou plusieurs gènes étant sélectionnés dans les gènes listés dans la revendication 1, et
où un sous-type CD8.1 est **caractérisé par** une expression régulée vers le haut des gènes listés dans la revendication 1 par rapport au niveau d'expression des mêmes gènes dans le sous-type CD8.2.

12. Utilisation d'un kit pour évaluer si un sujet présente un risque important ou faible de progression d'une maladie auto-immune, en déterminant si ledit sujet présente un sous-type de cellule CD4 à risque important (CD4.1) ou à risque faible (CD4.2),
où ledit kit comprend des amorces pour établir le niveau d'expression d'un ou de plusieurs gènes dans une cellule CD4 dudit sujet, pour déterminer si le sujet présente un sous-type de cellule CD4 à risque important (CD4.1) ou à risque faible (CD4.2), lesdits un ou plusieurs gènes étant sélectionnés dans les gènes listés dans la revendication 3, et où un sous-type CD4.1 est **caractérisé par** une expression différentielle des gènes listés dans la revendication 3 par rapport au niveau d'expression des mêmes gènes dans le sous-type CD4.2.

13. Utilisation d'un kit selon la revendication 12, où le kit comporte des amorces pour déterminer le niveau d'expression de PCAF (NM_003884 ; GI 8850).
